(19) Europäisches Patentamt
European Patent Office
Office européen des brevets

(11) EP 3 462 172 B1

(12) EUROPEAN PATENT SPECIFICATION

(45) Date of publication and mention of the grant of the patent:
**06.09.2023 Bulletin 2023/36**

(21) Application number: **18194844.9**

(22) Date of filing: **17.12.2013**

(51) International Patent Classification (IPC):
***G01N 15/10*** *(2006.01)* ***G01N 15/14*** *(2006.01)*
***G01N 33/53*** *(2006.01)* ***G01N 21/75*** *(2006.01)*
***C12M 1/34*** *(2006.01)* ***B01L 3/00*** *(2006.01)*
***G01N 21/64*** *(2006.01)* ***G01N 21/03*** *(2006.01)*
***G01N 35/00*** *(2006.01)* ***G01N 33/558*** *(2006.01)*
***G01N 33/569*** *(2006.01)*

(52) Cooperative Patent Classification (CPC):
**G01N 33/56972; B01L 3/502761; G01N 15/1459; G01N 15/1484; G01N 21/03; G01N 21/6428; G01N 21/75;** B01L 2200/10; G01N 2015/1006; G01N 2021/0328; G01N 2021/6439; G01N 2021/6482; G01N 2035/00158; G01N 2333/70535

(54) **SYSTEMS AND METHODS FOR DETECTING A BIOLOGICAL CONDITION**

SYSTEME UND VERFAHREN ZUM ERKENNEN EINES BIOLOGISCHEN ZUSTANDS

SYSTÈMES ET PROCÉDÉS DE DÉTECTION D'UNE CONDITION BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2012 US 201261737854 P**
**17.12.2012 US 201261737856 P**
**17.12.2012 US 201213716246**

(43) Date of publication of application:
**03.04.2019 Bulletin 2019/14**

(62) Document number(s) of the earlier application(s) in accordance with Art. 76 EPC:
**13865771.3 / 2 932 268**

(73) Proprietor: **Accellix Ltd**
**9777019 Jerusalem (IL)**

(72) Inventors:
- **KASDAN, Harvey Lee**
**Jerusalem 9777019 (IL)**
- **MEISSONNIER, Julien**
**Jerusalem 9777019 (IL)**
- **ZUTA, Yoav**
**Jerusalem 9777019 (IL)**
- **ROSEN, Micha**
**Jerusalem 9777019 (IL)**
- **HIMMEL, Yael**
**Jerusalem 9777019 (IL)**
- **BRODER, Yehoshua**
**Jerusalem 9777019 (IL)**
- **DAVIS, Bruce**
**Bangor, ME 04401 (US)**
- **GOLDMAN, Bruce**
**Jerusalem 9777019 (IL)**
- **GIRON, Boaz**
**Jerusalem 9777019 (IL)**
- **BOTESAZAN, Zion**
**Jerusalem 9777019 (IL)**
- **BLASBERG, Eliezer**
**Jerusalem 9777019 (IL)**
- **SEMMEL, Ilan**
**Jerusalem 9777019 (IL)**
- **ASCHKENASY, Jacques**
**Jerusalem 9777019 (IL)**

(74) Representative: **Pearl Cohen Zedek Latzer Baratz UK LLP**
**The Gridiron Building**
**One Pancras Square**
**London N1C 4AG (GB)**

(56) References cited:
**WO-A2-2006/055816**
**WO-A2-2007/076549**
**WO-A2-2011/094577**
**WO-A2-2011/128893**
**WO-A2-2012/120506**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

## FIELD OF THE INVENTION

The present invention relates generally to methods for detecting a biological condition, and more specifically to methods for detecting a biological condition in small fluid samples.

## BACKGROUND OF THE INVENTION

There are numerous medical conditions which are bard to diagnose. Often diagnosis by a physician is based on the physician's observation of combinations of symptoms in a patient. This sometimes, leads to misdiagnosis. Furthermore, the patient's response to a treatment, whether drug or other modality is often followed up by physician's observation.

Many laboratory tests are performed in the diagnostic arena on a bodily specimen or fluid to determine a biological condition in a patient. However, these tests are performed off-line in diagnostic laboratories. Often, the laboratory services are only provided during a single 8-hour shift during the day and tend to be labor intensive. Some prior art publications in the field include, *inter alia* US 8,116,984, US2006215155 and US2012187117. WO 2007/076549 discloses an assay implementation in a microfluidic format in a cartridge relating to a point-of-care instrument platform for monitoring and diagnosing infectious diseases.

Despite the inventions mentioned hereinabove, there still remains an unmet need to provide improved apparatus and methods for detecting and diagnosing biological conditions in a patient.

## **SUMMARY** OF THE INVENTION

The present invention provides a method for determining a biological condition in a subject, according to claim 1. Further embodiments are disclosed in the dependent claims.

The method according to the invention comprises:

a) providing a self-contained, valveless stationary cartridge [110], the cartridge comprising:

a sample chamber (1404);
a first blister comprising a first treatment composition (1420);
a second blister comprising a second composition (1422);
a third blister comprising a third composition (1424);
a first mixing chamber (1412);
a second mixing chamber (1411);
first mixing bellows (1415);
second reading bellows (1417);
a tortuous conduit (1413) connecting the first and second mixing chambers;
a reading cuvette (1430) connected to a reading region (1450) of a reading channel (1452);

wherein:

the sample chamber, first blister and second blister are each connected to the first mixing chamber;
the third blister (1424) is connected via a channel to the second mixing chamber (1411);
the first bellows is connected to the second mixing chamber;
the second bellows (1417) is connected to the first mixing chamber (1412) via the reading cuvette (1430);
the reading cuvette is connected to the first mixing chamber;

wherein:

activation of said first mixing bellows selectively moves fluid through fluidic channels within said cartridge, allowing a reaction of said sample with said composition(s);
activation of said second bellows selectively moves fluid through fluidic channels within said cartridge toward a reading region, allowing optical detection of said reaction in said fluid;

b) incubating a sample from said subject, in said cartridge [110], wherein at least one of said compositions comprises a reporter functionality, said reporter functionality being adapted to report a reaction of said treatment composition with said sample to report an assay of said reaction;

c) transferring said sample to said reading region (1450) through at least one of said fluidic channels by activating said second bellows;

d) optically detecting said reaction in a moving fluid, the detecting comprising:

1. providing an excitation beam [6032] to said reading region (1450);
2. measuring a forward scatter measurement [1606] from particles in said reading channel (1452);
3. passing a returned beam from said sample via a high-pass filter [449] and a concave grating [453];
4. providing an optical detector comprising detection elements for independent detection of different wavelengths;
5. detecting using said detector, a plurality of at least eight spectrally distinct signals [601] associated with said at least one reporter functionality produced by said concave grating in at least one of photomultiplier array (PMT) and a light-receiving reader unit and scatter channels data;

e) processing data outputted in said detecting step to determine said assay; and

f) receiving an indication responsive to said assay,

wherein said assay is a flow cytometric assay, thereby providing an indication of the biological condition in said subject, wherein the biological condition is selected from blood diseases, leukemia, thrombocytopenia, immune system disorders, local infections, urinary tract disorders, autoimmune diseases and sepsis;
and wherein said cartridge is sealed after receiving said sample.

In an embodiment, the detecting step comprises detecting a signal associated with at least one reporter functionality, said at least one reporter functionality adapted to report a reaction of said at least one composition with said sample, wherein said at least one composition comprises:

i. a cell surface marker;
ii. a cell stain;
iii. a reagent bound to a solid support;
iv. a chemical indicator; and
v. a biological cell indicator;

wherein said reagent bound to said solid support is selected from the group consisting of an immobilized enzyme, an immobilized substrate, a plasma protein bead, an antibody bead and an antigen bead.

In an embodiment, the biological cell indicator is selected from a cell cycle stage indicator, a cell proliferation indicator, a cytokine indicator, a metabolic indicator and an apoptosis indicator.

In an embodiment, the at least one composition comprises at least two compositions.

In an embodiment, the at least two compositions comprise at least one of:

a. a cell surface marker and a cell element stain;
b. a cell surface marker and a plasma protein bead assay;
c. a cell surface marker and a solution change marker;
d. a cell element stain and a plasma protein bead assay; and
e. a cell element stain and a solution change marker.

In an embodiment, the at least one composition comprises:

a) a detection composition comprising at least one of;

i. at least one target antibody;
ii. at least one positive control identifying antibody; and
iii. at least one negative control identifying detection moiety or characteristic; and

b) at least one reference composition comprising at least one of;

i. a target signal reference composition; and
ii. a reference identifier composition.

In an embodiment, the at least one composition comprises:

a) an antibody composition comprising at least one of;

i. at least one target antibody;
ii. at least one positive control identifying antibody; and
iii. at least one negative control identifying antibody or characteristic; and

b) at least one reference composition comprising at least one of;

iv. a target signal reference composition; and
v. a reference identifier composition.

In an embodiment, the sample comprises at least one of;

i. a bodily specimen comprising a target moiety;
ii. a positive control moiety; and
iii. a negative control moiety.

In an embodiment, the at least one composition further comprises at least one conditioning moiety comprising;

a. at least one lysis reagent; and
b. at least one diluent.

In an embodiment, the providing step further comprises passing said excitation beam via a dichroic mirror through an objective onto a reading channel in said cartridge.

In an embodiment, the method further comprises collecting particle fluorescent emission through said objective, further passing said particle fluorescent emission through said dichroic mirror and reflecting said fluorescent emission from a beamsplitter into a detection path.

In some embodiments of the present invention, improved methods are provided for detecting and diagnosing a biological condition in a patient.

In other embodiments of the present invention, the method is described for providing rapid detection of biological moieties in a sample from a patient.

In further embodiments of the present invention, the method provides detection of biological moieties in a small fluid sample from a patient.

Additionally, according to an embodiment of the present invention, the cartridge further includes an alignment means adapted to align a reading channel on the cartridge for a detection of the least one reporter functionality.

Furthermore, according to an embodiment of the present invention, the cartridge further comprises a plurality of fluidic open channels, all the channels in liquid communication with each other.

Moreover, according to an embodiment of the present invention, the cartridge is adapted to be sealed after receiving a fluid specimen and to pass a predetermined quantity of the sample through at least part of the plurality of fluidic open channels.

Further, according to an embodiment of the present invention, the cartridge further includes at least one inflatable deformable elastic chamber adapted to apply at least one of a negative pressure and a positive pressure in the fluidic channels.

Additionally, according to an embodiment of the present invention, the at least one deformable elastic chamber is adapted to further contact the at least one reagent stored in a sealed on-board storage chamber with a predetermined quantity of the sample in a reaction chamber to induce the reaction.

Further, according to an embodiment of the present invention, a first urging means is disposed proximal to the on-board storage chamber such that upon movement is adapted to break a frangible seal on the storage chamber.

Yet further, according to an embodiment of the present invention, the alignment means adapted to align with a reading channel on the cartridge for a detection of a reaction in the predetermined quantity of the sample.

Additionally, according to an embodiment of the present invention, some of the plurality of fluidic open channels is of a cross-section of 0.1 to $2\ mm^2$.

Notably, according to an embodiment of the present invention, the predetermined quantity is of a volume of 10 to 500 microliters.

Furthermore, according to an embodiment of the present invention, the cartridge is adapted to contact a plurality of on-board reagents with the at least one of the sample and a reaction product.

In some cases, according to an embodiment of the present invention, the cartridge is adapted to induce cascaded sequential reactions of the plurality of on-board reagents, with the at least one of the sample and the reaction product

Additionally, according to an embodiment of the present invention, the cartridge includes at least one reaction chamber of a volume of 200 to 10000 microliters.

Additionally, according to an embodiment of the present invention, the cartridge has a shelf-life of 6 to 24 months.

Importantly, according to an embodiment of the present invention, the cartridge is valveless.

Notably, according to an embodiment of the present invention, the assay is a flow cytometric assay.

Additionally, according to an embodiment of the present invention, the chemical state is a biochemical state.

Furthermore, according to an embodiment of the present invention, the biochemical state is indicative of a biological condition.

Additionally, according to an embodiment of the present invention, the sample is a biological sample.

In some cases, according to an embodiment of the present invention, the biological sample is a bodily sample.

Moreover, according to an embodiment of the present invention, the bodily sample is blood, serum, plasma, urine, saliva, cerebrospinal fluid (CSF), serous fluid, peritoneal fluid and synovial fluid blood, urine, plasma, serum or saliva.

Additionally, according to an embodiment of the present invention, the cartridge is valveless.

Further, according to an embodiment of the present invention, the cartridge is a disposable microfluidics cartridge.

Yet further, according to an embodiment of the present invention, the sample is introduced to the cartridge via capillary action.

Additionally, according to an embodiment of the present invention, the at least one reagent disposed in the cartridge includes at least one of;

a. at least one target antibody;
b. at least one positive control identifying antibody; and
c. at least one negative control identifying detection moiety.

Moreover, according to an embodiment of the present invention, the at least one reagent disposed in the cartridge includes at least one reference composition including at least one of;

a. a target signal reference composition; and
b. a reference identifier composition.

Additionally, according to an embodiment of the present invention, the at least one reagent disposed in the cartridge includes at least one of;

a. a positive control moiety; and
b. a negative control moiety.

Furthermore, according to an embodiment of the present invention, the at least one reagent disposed in the cartridge includes at least one sepsis biomarker.

According to another embodiment of the present invention, the method for determining a biological condition in a subject further includes;

a. incubating a sample from the subject in the cartridge [110] for a predetermined period of time; and
b. receiving an indication responsive to the at least one reporter functionality thereby providing an indication of the biological condition in the subject in accordance with the chemical state.

Additionally, according to an embodiment of the present invention, the biological condition is selected from blood diseases such as leukemia, thrombocytopenia, immune system disorders, local infections, urinary tract disorders, autoimmune diseases and sepsis.

Furthermore, according to an embodiment of the present invention, the indication is quantitative.

Moreover, according to an embodiment of the present invention, the method is completed within twenty minutes.

According to another embodiment of the present invention, the method for determining a biological condition in a mammalian subject includes;

a. incubating a specimen from the subject with at least one composition in the cartridge [110], for a predetermined period of time to form at least one reaction product, when the subject has the biological condition; and

b. receiving an indication of the at least one reaction product responsive to at least one reporter element thereby providing the indication of the biological condition in the subject.

According to another embodiment of the present invention, the automated method is for determining the presence or absence of sepsis in a subject and includes;

a. contacting a blood sample from the subject with a fluorescently-labeled binding moiety in the cartridge [110], the moiety specific to a sepsis marker, wherein the volume of the blood sample is 50 μL or smaller; and

b. detecting the presence, absence or level of the binding moiety in the sample, thereby determining the presence or absence of sepsis in the subject within twenty minutes.

Furthermore, according to an embodiment of the present invention, wherein the sepsis marker is CD64.

Additionally, according to an embodiment of the present invention, the sepsis marker is CD 163.

Moreover, according to an. embodiment of the present invention, the method further includes contacting the blood sample with a second fluorescently-labeled binding moiety specific for a second sepsis marker.

Further, according to an embodiment of the present invention, the sepsis marker is CD64 and the second sepsis marker is CD163.

According to another embodiment of the present invention, the method for performing an assay for determining a chemical state in a self-contained stationary cartridge includes;

a. introducing a sample into the cartridge [110];

b. reacting at least one reagent with the sample; and

c. detecting a signal associated with at least one reporter functionality, at least one reporter functionality adapted to report a reaction of the at least one reagent with the sample, thereby determining the chemical state.

Furthermore, according to an embodiment of the present invention, the method further includes forming at least one product and detecting a signal associated with the product.

Additionally, according to an embodiment of the present invention, the assay is a flow cytometric assay.

Further, according to an embodiment of the present invention, the chemical state is a biochemical state.

Further, according to an embodiment of the present invention, the biochemical state is indicative of a biological condition.

Furthermore, according to an embodiment of the present invention, the sample is a biological sample.

Additionally, according to an embodiment of the present invention, the biological sample is a bodily sample. Furthermore, according to an embodiment of the present invention, the bodily sample is blood, serum, plasma, urine, saliva, cerebrospinal fluid (CSF), serous fluid, peritoneal fluid or synovial fluid.

Moreover, according to an embodiment of the present invention, the at least one reagent includes at least one of;

a. a cell surface marker;

b. a cell stain;

c. a reagent bound to a solid support;

d. a chemical indicator; and

e. a biological cell indicator.

Furthermore, according to an embodiment of the present invention, the cell surface marker is CD64, CD4, CD8, a stem cell indicator, a Minimal Residual Disease indicator or a lymphocyte subtype indicator.

Additionally, according to an embodiment of the present invention, the cell stain is a white blood cell differential indicator, or an apoptosis indicator.

Further, according to an embodiment of the present invention, the reagent bound to the solid support is an immobilized enzyme, an immobilized substrate, a plasma protein bead, an antibody bead, an antigen bead or an ELISA assay.

Additionally, according to an embodiment of the present invention, the chemical indicator is a color indicator, a turbidity indicator, a pH indicator, an adsorption indicator, an emission indicator or a chemical reaction indicator.

Furthermore, according to an embodiment of the present invention, the biological cell indicator is a cell cycle stage indicator, a cell proliferation indicator, a cytokine indicator, a metabolic indicator or an apoptosis indicator.

Further, according to an embodiment of the present invention, the at least one reagent includes at least two reagents.

Moreover, according to an embodiment of the present invention, the at least two reagents include at least one of;

a. a cell surface marker and a cell element stain;
b. a cell surface marker and a plasma protein bead assay;
c. a cell surface marker and a solution change marker;
d. a cell element stain and a plasma protein bead assay; and
e. a cell element stain and a solution change marker.

Furthermore, according to an embodiment of the present invention, the biological condition is selected from blood diseases such as leukemia, thrombocytopenia immune system disorders, local infections, urinary tract disorders, autoimmune diseases and sepsis.

According to another embodiment of the present invention, the method forms a chemical reaction in a stationary cartridge method and includes;

a. storing at least one composition in the cartridge described herein; and
b. activating at least one inflatable chamber in the cartridge to provide at least one pressure force to the at least one reagent thereby inducing the chemical reaction.

Moreover, according to an embodiment of the present invention, the disposable cartridge is a disposable microfluidics cartridge.

According to an embodiment of the present invention, the composition used for evaluating a biological condition comprises;

a. a sample composition comprising at least one of;

  i. a bodily specimen comprising a target moiety;
  ii. a positive control moiety; and
  iii. a negative control moiety;

b. a detection composition comprising a least one of;

  i. at least one target antibody;
  ii. at least one positive control identifying antibody; and
  iii. at least one negative control identifying detection moiety or characteristic; and

c. at least one reference composition comprising at least one of;

  i. a target signal reference composition; and
  ii. a reference identifier composition.

According to another embodiment of the present invention, the composition used for evaluating a biological condition comprises;

a. a sample composition comprising at least one of;

  iii. a bodily specimen comprising a target moiety;
  iv. a positive control moiety; and
  v. a negative control moiety;

b. an antibody composition comprising at least one of;

  vi. at least one target antibody (CD64 antibody);
  vii. at least one positive control identifying antibody (CD163); and
  viii. at least one negative control identifying antibody or characteristic; and

c. at least one reference composition comprising at least one of;

  ix. a target signal reference composition; and

x. a reference identifier composition.

Additionally, according to an embodiment of the present invention, the composition further comprises at least one conditioning moiety comprising;

d. at least one lysis reagent; and
e. at least one diluent.

Furthermore, according to an embodiment of the present invention, the biological condition is a blood disease such as leukemia, thrombocytopenia immune system disorders, local infections, urinary tract disorders, autoimmune diseases or sepsis.

Moreover, according to an embodiment of the present invention, the bodily specimen is blood, serum, plasma, urine, salvia, cerebrospinal fluid (CSF), serious fluid, peritoneal fluid or synovial fluid.

According to another embodiment of the present invention, the target moiety includes a CD64 surface antigen on neutrophils.

Additionally, according to a further embodiment of the present invention, the positive control moiety includes monocytes and the negative control includes lymphocytes.

Additionally, according to embodiment of the present invention, the target moiety is CD64 on neutrophils, the positive control moiety includes CD64 expression on monocytes, and the negative control moiety includes lymphocytes without CD64 expression.

Further, according to an embodiment of the present invention, the target indicator is bound to a signaling moiety on the at least one target antibody.

Yet further, according to an embodiment of the present invention, the at least one reference composition includes beads.

Additionally, according to an embodiment of the present invention, the beads include polystyrene microbeads.

Moreover, according to an embodiment of the present invention, the target antibody reference composition includes a first fluorescent signal and the reference identifier composition includes a second fluorescent signal.

Furthermore, according to an embodiment of the present invention, the first fluorescent signal includes FITC and the second fluorescent signal includes Starfire Red fluor.

According to an embodiment of the present invention, the method further comprises quantifying a biomarker in a sample;

a. contacting the sample with a fluorescently-labeled binding moiety that specifically binds to the biomarker;
b. detecting a first fluorescent signal from at least a portion of the labeled sample;
c. detecting a second fluorescent signal from a population of fluorescently-labeled particles, wherein the population includes a known fluoresecent intensity over a fixed time; and
d. normalizing the first fluorescent signal to the second fluorescent signal, thereby quantifying the biomarker, wherein the normalizing includes using a device comprising software capable of comparing the first and second fluorescent signal.

Furthermore, according to an embodiment of the present invention, the biomarker is a sepsis biomarker.

Moreover, according to an embodiment of the present invention, the biomarker is CD64 or CD163.

Additionally, according to an embodiment of the present invention, the sample is a blood sample.

According to another embodiment of the present invention, the fluorescent label of the binding moiety and the fluorescent label of the particles is the same fluorescent label.

Further, according to an embodiment of the present invention, the binding moiety is an antibody.

According to an embodiment of the present invention, the software is capable of recognizing a specific lot of fluorescently-labeled particles.

Moreover, according to an embodiment of the present invention, the individual fluorescent signals include at least one first fluorescent signal and at least one second fluorescent signal.

Additionally, according to an embodiment of the present invention, the fluorescently-labeled binding moiety targets a first cell population and a second cell population in the sample.

According to another embodiment of the present invention, the detection of binding of the binding moiety to the second cell population provides an internal positive control for the sample.

Furthermore, according to an embodiment of the present invention, the binding moiety is anti-CD64 antibody and the first cell population includes polymorphonuclear leukocytes.

Yet further, according to an embodiment of the present invention, the second cell population includes monocytes.

According to an embodiment of the present invention, the method further comprises the step of determining the presence of at least one cell population in the sample that is not bound by the binding moiety, thus proving an internal negative control for the sample.

According to another embodiment of the present invention, the composition used for evaluating a biological condition comprises;

a. a sample comprising at least one of;

i. a bodily specimen comprising a target moiety;
ii. a positive control moiety; and
iii. a negative control moiety;

b. an antibody composition comprising at least one of;

iv. at least one target antibody;
v. at least one positive control identifying antibody; and
vi. at least one negative control identifying antibody or characteristic; and

c. at least one reference composition comprising at least one of;

vii. a target antibody reference composition; and
viii. a reference identifier composition.

According to an embodiment of the present invention, the composition further comprises at least one conditioning moiety comprising;

a) at least one lysis reagent; and
b) at least one diluent.

According to another embodiment of the present invention, the method is for determining the presence or absence of sepsis in a subject and includes;

a) contacting a blood sample from the subject with a fluorescently-labeled binding moiety specific to a sepsis marker, wherein the volume of the blood sample is 50 μL or smaller; and
b) detecting the presence, absence or level of the binding moiety in the sample, thereby determining the presence or absence of sepsis in the subject.

According to another embodiment of the present invention, the method comprises quantifying a biomarker in a sample;

a) contacting the sample with a fluorescently-labeled binding moiety that specifically binds to the biomarker;
b) detecting a first fluorescent signal from at least a portion of the labeled sample;
c) detecting a second fluorescent signal from a population of fluorescently-labeled particles, wherein the population includes a known fluorescent intensity over a fixed time; and
d) normalizing the first fluorescent signal to the second fluorescent signal, thereby quantifying the biomarker, wherein the normalizing includes using a device comprising software capable of comparing the first and second fluorescent signal.

According to some embodiments, the sample may be liquid, according to other embodiments, the sample may be a colloid or suspension. According to further embodiments, the sample may be a solid, such as in a powder or crystal form.

Typical turnaround times for diagnostic prior art assays are 30-120 minutes. Often, the time lost in waiting for laboratory results can lead to a further deterioration in a patient, and sometimes death. In some cases, the physician has to act without having the laboratory results. This can lead to providing the patient with the wrong treatment. The method of the present invention includes rapid assays to save lives.

According to an embodiment of the present invention, the automated method is for determining the presence or absence of sepsis in a subject and includes;

a) contacting a blood sample from the subject with a fluorescently-labeled binding moiety specific to a sepsis marker, wherein the volume of the blood sample is 50 μL or smaller; and

b) detecting the presence, absence or level of the binding moiety in the sample, thereby determining the presence or absence of sepsis in the subject within twenty minutes.

Additionally, according to an embodiment of the present invention, the sepsis marker is CD64.

Furthermore, according to an embodiment of the present invention, a second sepsis marker is CD163.

Moreover, according to an embodiment of the present invention, the method further includes contacting the blood sample with a second fluorescently-labeled binding moiety specific for a second sepsis marker.

Further, according to an embodiment of the present invention, the sepsis marker is CD64 and the second sepsis marker is CD163.

Additionally, according to an embodiment of the present invention, the binding moiety is an antibody.

Moreover, according to an embodiment of the present invention, the detecting step is performed in a device capable of receiving the sample and capable of detecting the binding moiety.

Additionally, according to an embodiment of the present invention, the method further includes the step of calibrating the device by detecting a population of the fluorescently-labeled particles.

According to another embodiment of the present invention, the particles include the same fluorescent label as the fluorescently-labeled binding moiety.

Additionally, according to an embodiment of the present invention, the method further includes a second population of particles that include the same fluorescent label as the second fluorescently-labeled binding moiety.

Moreover, according to an embodiment of the present invention, the method further includes performing an internal calibration after the detecting the fluorescently-labeled binding moiety.

Notably, according to an embodiment of the present invention, the calibration is completed in less than 5 minutes.

According to some embodiments, the particles are microbeads.

Additionally, according to an embodiment of the present invention, the method is performed in less than 15 minutes.

Furthermore, according to an embodiment of the present invention, the method further includes the step of determining the presence of at least one cell population in the sample that is not bound by the binding moiety, thus providing an internal negative control for the sample.

The present invention will be more fully understood from the following detailed description of the preferred embodiments thereof, taken together with the drawings.

## BRIEF DESCRIPTION OF THE DRAWINGS

The invention will now be described in connection with certain preferred embodiments with reference to the following illustrative figures so that it may be more fully understood.

With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

Fig. 1A is a simplified three dimensional front view of a system for carrying out the method of the present invention for detecting a biological condition;

Fig. 1B is a simplified three dimensional inner front view of a reader assembly for detecting a biological condition, in accordance with an embodiment of the present invention;

Fig. 1C is a simplified three dimensional inner rear view of a reader assembly for detecting a biological condition, in accordance with an embodiment of the present invention;

Fig. 2A is a simplified blown up diagram of an optical reader assembly for detecting a biological condition, in accordance with an embodiment of the present invention;

Fig. 2B is another simplified blown up diagram of a photomultiplier tube of the optical reader assembly for detecting a biological condition, in accordance with an embodiment of the present invention;

Fig. 3A shows a reader optics assembly, a cartridge handling unit, and a forward scatter detection unit, in accordance with an embodiment of the present invention;

Fig. 3B shows a right side view of a reader optics assembly, in accordance with an embodiment of the present invention;

Fig. 3C shows a left side view of a reader optics assembly, in accordance with an embodiment of the present invention;

Fig. 3D is a forward scatter detection assembly, in accordance with an embodiment of the present invention;
Fig. 3E is a side view of the forward scatter detection assembly, in accordance with an embodiment of the present invention;
Fig. 4A shows a cutaway view of a reader assembly, in accordance with an embodiment of the present invention;
Fig. 4B shows an exploded right side view of a reader assembly, in accordance with an embodiment of the present invention;
Fig 4C shows a left side blown up view of the reader assembly, in accordance with an embodiment of the present invention;
Fig. 4D shows a rear view of a cartridge handling unit (CHU), in accordance with an embodiment of the present invention;
Fig. 4E shows a front view of a cartridge handling unit (CHU), in accordance with an embodiment of the present invention;
Fig. 5 shows an exploded view of a reader optics assembly, in accordance with an embodiment of the present invention;
Fig. 6 is a simplified illustration of a disposable cartridge of the system for carrying out the method of the present invention of Fig. 1 A;
Fig. 7A is a simplified schematic illustration of an optical arrangement of a reader optics assembly, in accordance with an embodiment of the present invention;
Fig. 7B is another simplified schematic illustration of optical arrangement of a reader optics assembly, in accordance with an embodiment of the present invention;
Fig. 8A is a schematic representation of one example of multi-wavelength excitation in the optical unit of Fig. 7A or 7B, in accordance with an embodiment of the present invention;
Fig. 8B shows a graphical output of transmission as a function of wavelength for a dichroic filter of Fig. 7B, employing the multi-wavelength excitation of Fig. 8A, in accordance with an embodiment of the present invention;
Fig. 8C is a schematic representation of part of the optical unit employing multi-wavelength excitation of Fig. 8A and the dichroic filter of Fig. 5B, in accordance with an embodiment of the present invention.
Fig. 9A is a schematic view of a sampling cartridge of the system for carrying out the method of the present invention of Fig. 1A, in accordance with an embodiment of the present invention;
Fig. 9B shows a schematic view of disposable cartridge in flow-cytometer device, in accordance with an embodiment of the present invention;
Fig. 10 is a simplified flowchart of a method for rapid determination of a medical condition, in accordance with an embodiment of the present invention;
Fig. 11 is a three-dimensional graph showing the optical output over time of reference beads (RM) relative to a sample from a human patient (PMN), in accordance with an embodiment of the present invention;
Figs. 12A-12C show graphs of optical outputs over time of the reference beads and the sample from a human patient, in accordance with an embodiment of the present invention;
Fig. 13 A is an outer side view of a cartridge assembly, in accordance with an embodiment of the present invention;
Fig. 13B is an inner side view of a cartridge assembly, in accordance with an embodiment of the present invention;
Fig. 14A-14O show a sequence of process events in a cartridge assembly, in accordance with an embodiment of the present invention;
Fig. 15 is a schematic illustration of a micro flow spectrometer reading, in accordance with an embodiment of the present invention;
Fig. 16 is a flow chart of a method for optical processing, in accordance with an embodiment of the present invention;
Figs. 17A-17B are schematic illustrations of steps of use of a graphical user interface, in accordance with an embodiment of the present invention;
Fig. 18 is a cartridge block diagram showing a role of signal processing software, in accordance with an embodiment of the present invention;
Fig. 19A is a flow chart of an algorithm for biological detection, in accordance with an embodiment of the present invention;
Fig. 19B is a flow chart of an algorithm for biological detection, in accordance with an embodiment of the present invention;
Figs. 20A-20B shows bandwidth leveled and smoothed arrays, in accordance with an embodiment of the present invention;
Figs. 21A-21C are schematics for solving a fluor decomposition of an observed signal, in accordance with an embodiment of the present invention;
Figs. 22A-22B is a graphical comparison of system performance with FITC beads with MESF detection versus FACS, in accordance with an embodiment of the present invention;
Figs. 23A-23B show graphical displays of linearity of system performance with Alexa 488 MESF, in accordance with an embodiment of the present invention;

Fig. 24 is a three-dimensional graph showing the optical output over time of a CD4-CD8 assay, in accordance with an embodiment of the present invention;

Fig. 25 is a graphical display showing a cluster analysis of a CD4-CD8 assay, in accordance with an embodiment of the present invention;

Figs. 26-27 are graphical displays showing cluster separations of the cluster analysis of Fig. 25, in accordance with an embodiment of the present invention; and

Fig. 28 is a comparison table of different array options, in accordance with an embodiment of the present invention.

Fig. 29A is a flowchart of a specific implementation of an algorithm for selecting groups of data from a scatterplot, in accordance with an embodiment of the present invention;

Fig. 29B is a flowchart of a general implementation of an algorithm for selecting groups of data from a scatterplot, in accordance with an embodiment of the present invention;

Fig. 30 is a scatterplot matrix of the four fluors signatures showing four distinct event groups, in accordance with an embodiment of the present invention;

Fig. 31A is a histogram of data of Starfire Red (SFR) signature values, in accordance with an embodiment of the present invention;

Fig. 31B is a plot of a polynomial and first and second derivative thereof of the histogram shown in Fig. 31A, in accordance with an embodiment of the present invention;

Fig. 32A is a histogram of data of PE488 signature values, in accordance with an embodiment of the present invention;

Fig. 32B shows a polynomial fitted to the histogram in Fig. 32A as well as corresponding first and second derivatives, in accordance with an embodiment of the present invention;

Fig. 33A is a histogram of data of PEAF488 signature values, in accordance with an embodiment of the present invention;

Fig. 33B shows a polynomial fitted to the histogram in Fig. 33A as well as corresponding first and second derivatives, in accordance with an embodiment of the present invention.

Fig. 34A is a histogram of data of Diode 1 channel signature values, in accordance with an embodiment of the present invention; and

Fig. 34B shows the polynomial fitted to the histogram in Fig. 34A as well as the corresponding first and second derivatives, in accordance with an embodiment of the present invention.

In all the figures similar reference numerals identify similar parts.

## DETAILED DESCRIPTION OF PREFERRED EMBODIMENTS

In the detailed description, numerous specific details are set forth in order to provide a thorough understanding of the invention. However, it will be understood by those skilled in the art that these are specific embodiments and that the present invention may be practiced also in different ways that embody the characterizing features of the invention as described and claimed herein.

International patent application publication no. WO2011/128893 to Kasdan et al., describes a device, system and method for rapid determination of a medical condition.

The present invention provides a method for determining a biological condition in a subject, the method comprising:

a) providing a self-contained, valveless stationary cartridge [110], the cartridge comprising:

- a sample chamber (1404);
- a first blister comprising a first treatment composition (1420);
- a second blister comprising a second composition (1422);
- a third blister comprising a third composition (1424);
- a first mixing chamber (1412);
- a second mixing chamber (1411);
- first mixing bellows (1415);
- second reading bellows (1417);
- a tortuous conduit (1413) connecting the first and second mixing chambers;
- a reading cuvette (1430) connected to a reading region (1450);

wherein:

the sample chamber, first blister and second blister are each connected to the first mixing chamber;
the third blister (1424) is connected via a channel to the second mixing chamber (1411);
the first bellows is connected to the second mixing chamber;
the second bellows (1417) is connected to the first mixing chamber (1412) via the reading cuvette (1430);
the reading cuvette is connected to the first mixing chamber;
the reading cuvette is connected to the reading region;

wherein:

activation of said first mixing bellows selectively moves fluid through fluidic channels within said cartridge, allowing a reaction of said sample with said composition(s);
activation of said second bellows selectively moves fluid through fluidic channels within said cartridge toward a reading region, allowing optical detection of said reaction in said fluid;

b) incubating a sample from said subject, in said cartridge [110], wherein at least one of said compositions comprises a reporter functionality, said reporter functionality being adapted to report a reaction of said treatment composition with said sample;
c) transferring said sample to said reading region (1450) through at least one of said fluidic channels by activating said second bellows;
d) optically detecting said reaction in a moving fluid, the detecting comprising:

1. providing an excitation beam [6032] to said reading region (1450);
2. measuring a forward scatter measurement [1606] from particles in at least one of said sample and said composition(s) in an optical reader;
3. passing a returned beam from said sample via a high-pass filter [449] and a concave grating [453];
4. providing an optical detector comprising detection elements for independent detection of different wavelengths;
5. detecting using said detector, a plurality of at least eight spectrally distinct signals [601] associated with said at least one reporter functionality produced by said concave grating in at least one of photomultiplier array (PMT) and a light-receiving reader unit and scatter channels data;

e) processing data outputted in said detecting step to determine said assay; and
f) receiving an indication responsive to said assay,

wherein said assay is a flow cytometric assay, thereby providing an indication of the biological condition in said subject, wherein the biological condition is selected from blood diseases, leukemia, thrombocytopenia, immune system disorders, local infections, urinary tract disorders, autoimmune diseases and sepsis;
wherein said cartridge has a shelf-life of six to twenty-four months, and
wherein said cartridge is sealed after receiving said sample.

Reference is now made to Fig. 1A. which is a simplified three dimensional front view of a system 101 for carrying out the methods of the invention comprising a reader assembly 100 and a cartridge 110 for detecting a biological condition, in accordance with an embodiment of the present invention.

Shown in Fig. 1A are the reader assembly 100 and the cartridge 110. The cartridge is inserted in the reader assembly as shown. Once the cartridge is inserted in the reader assembly all assay pre-analytical processing and analysis are performed automatically. Results of the analysis are displayed on a user interface touchscreen 115, which is also used to control operation of the reader.

Fig. 1B shows a simplified three dimensional inner front view 103 of reader assembly 100 for detecting a biological condition, in accordance with an embodiment of the present invention.

The internal components of the reader assembly are shown in Fig. 18. There is seen left side view 120, showing an ITX computer, 122. a Galil motor controller, 124, an electronics power supply 126, cartridge, 110, inserted into a cartridge handling unit (CHU) 128 and a forward scatter detector 130. Also seen, is a right side view 140 showing reader optics 142, a data acquisition board 144 and a general electronics printed circuit board 146.

Reference is now made to Fig. 2A, which is a simplified blown up diagram of a reader optics assembly 200 for detecting a biological condition, in accordance with an embodiment of the present invention. Fig. 2B is another simplified blown up diagram of a photomultiplier tube 250 of the optical reader assembly for detecting a biological condition, in accordance with an embodiment of the present invention.

Fig. 2A shows the main modular components of a reader optics assembly 200. A complete side view 220 of the optical assembly is seen, in addition to a top view 222. A laser unit 203 includes a laser and beam expander 223 in its heat sink assembly 221. The assembly further comprises an excitation and emission collection optics 204. The reader optics assembly also comprises a photomultiplier assembly 202, a laser mirror cover 205, a PMT mirror cover 206, a modified M6 set screw 207, a box clamp 208, and various screws 209-211. The reader optics assembly is assembled as shown in Fig. 2A.

Fig. 2B shows details of the PMT assembly. A side view and an end view of the PMT assembly are shown as side view 270 and end view 272 respectively. The major elements of the PMT assembly include a PMT box 251, a PMT grating assembly 252, a PMT bridge assembly 255, a PMT cover 258, a PMT unit 259, a PMT lens assembly 260, a PMT pinhole nut 261, a pinhole 262, a pinhole hood 263 and an adjustment bar 265.

Fig. 3A shows a reader optics assembly 310, a cartridge handling unit 312 and a forward scatter detection unit 314, in accordance with an embodiment of the present invention.

Fig. 3B shows a right side view of a complete reader optics assembly 142, in accordance with an embodiment of the present invention.

Fig. 3C shows a left side view of the reader optics assembly, in accordance with an embodiment of the present invention.

Fig. 3D is a forward scatter detection assembly 130, in accordance with an embodiment of the present invention. This assembly contains LEDs, 352, to illuminate a reading channel (such as reading channel 1452 (Fig. 14M) during an autofocus process, a stop 358, to block low angle scatter and a lens 356 to collect the desired forward scatter for the detection photodiode, (forward scatter detector 130, Fig. 1B).

Fig. 3E is a side view of forward scatter detection assembly 130, in accordance with an embodiment of the present invention. Shown in this view are an illumination lens 350, collection lenses 356, 357, and 358, as well as a detection photodiode 360.

Fig. 4A shows a cutaway view of reader assembly 130, in accordance with an embodiment of the present invention. This is cutaway view of the reader assembly showing its components in its front and on a left side. These components include an ITX board 122, a cartridge handling unit 128, and the forward scatter detection assembly, 130.

Fig. 4B shows an exploded right side view of a reader assembly 129, in accordance with an embodiment of the present invention The three major components in this view are a reader optics assembly 142, a cartridge handling unit 128, and a forward scatter detection module 130.

Fig 4C shows a left side blown up view of the reader assembly, in accordance with an embodiment of the present invention. Shown in this view are ITX computer board 122, cartridge handling unit 128, forward scatter detection assembly 130, and the other side of the reader optics assembly, 142.

Fig 4D shows a rear view of cartridge handling unit (CHU) 128, in accordance with an embodiment of the present invention. In this view, a handle 199 of the inserted cartridge, 110, can be seen. Sensors 412 are configured therein to detect the position of motors 410, and actuators 414, which are adapted to crush the blisters, as well as an actuator 416 (940, Fig. 9, 1415, 1417 Figs. 14A-L), can be seen on the shafts 417 of the motor. An opening 418 is provided for the microscope objective 438 to view the reading channel on the cartridge.

Fig. 4E shows a front view of a cartridge handling unit (CHU), in accordance with an embodiment of the present invention. This figure shows the front view of the cartridge handling unit (CHU) 128. In this view, the handle in the upper portion of cartridge 110 can be seen. A port 420 to view the microfluidic path is provided. This port is viewed by a camera 430, in order to ensure that the correct operation occurs within the cartridge. Another opening 441 is provided for the forward scatter to exit the cartridge handling unit and be observed by the forward scatter detection assembly 130.

Fig. 5 shows an exploded view of a reader assembly 130, in accordance with an embodiment of the present invention.

Fig. 6 is a simplified illustration of a disposable cartridge 6050 for rapid determination of a medical condition, in accordance with an embodiment of the present invention;

Disposable cartridge 6050 is adapted to receive a bodily fluid, such as blood, urine, serum or plasma. The disposable cartridge is constructed and configured to have several different sections 6052, 6054, 6056 and 6058. Section 6052 is a body fluid aspiration section, which is adapted to receive the body fluid directly or indirectly from the patient (or animal) and this section acts as a reservoir of the body fluid.

Disposable cartridge 6050 comprises fluid conveying means between the sections, such as air pressure, liquid pressure, mechanical means and combinations thereof. Body fluid aspiration section 6052 is adapted to convey a predetermined quantity of the body fluid (a body fluid sample 6051) to a pre- analytical sample processing section 6054.

In pre-analytical sample processing section 6054, at least one preparatory step is performed on the body fluid such as:

a) incubation with at least one antibody;
b) incubation with at least one antigen;
c) staining of at least one cell type in the body fluid;

d) enzymatic lysing of at least one cell type of the body fluid;
e) osmotic lysing of at least one cell type of the body fluid;
f) heat or cool at least part of the bodily fluid;
g) addition of reference material to the bodily fluid; and
h) chemical reaction with at least one element of the body fluid.

The pre-treated sample of bodily fluid is then conveyed from pre-analytical sample processing section 6054 to a sample excitation/interaction zone or section 6056. This pre-treated sample may be conveyed continuously or in a batch mode to sample excitation/interaction section 6056.

Fig. 7A is a simplified schematic illustration of an optical arrangement of a reader optics assembly 400, in accordance with an embodiment of the present invention;

A laser 440 or other appropriate light source provides a light beam 442, which may be directed towards a plurality of optical elements, including a dichroic filter 443, a beam splitter 444, a focusing lens 445, a pinhole 446 and a silicon reader unit 447, for recording a signal from a beam 442 directed through the objective 438 towards a sample 450 and returned to the optical unit. Additional optical elements may include an optional attenuator 448, a high-pass filter 449, a focusing lens 451, a slit 452, a concave grating 453, and a PMT array 454. This arrangement of elements, representing an embodiment of the present invention, allows for generation of excitation light, focusing it on a sample, collecting reflected and emitted light signal resulting from the interaction of the excitation light and fluorophores in the sample and recording said returned light so as to determine fluorescence of sample in response to light illumination from laser 440.

With respect to Fig. 7A, the laser illumination 442 is reflected by the dichroic filter 443 through the objective 438 and focused on the channel containing the flowing particles 458. This illumination excites the fluorophores attached to the protein markers that are bound to the cells.

The resulting fluorescent illumination is collected by the objective 438 and because of the longer wavelength of this emission passes through the dichroic filter 443 and is reflected by the beam splitter 444 through the high pass filter 449. The high pass filter blocks any reflected laser illumination. The focusing lens 451 focuses the multi-wavelength emission illumination on the slit 452. The concave grating 453 images the slit at multiple wavelengths on the elements of the PMT array 454. This completes the process of creating a multispectral detection of the fluorescent emission.

While most of the illumination collected by the objective is reflected by the beam splitter 444 a small fraction is allowed to pass through and is focused by focusing lens 445 through a pinhole 446 on the silicon reader unit 447, which may be a single photodiode or a focal plane array such as CCD sensor. During the focusing operation best focus is achieved when the signal on this reader unit 447 is maximized. When this signal is maximized, the intensity of the signal on the PMT array 454 is also maximized.

Reference is now made to Fig. 7B, which is another simplified schematic illustration of optical arrangement 460 of a reader optics assembly 400 (Fig. 7A), in accordance with an embodiment of the present invention;

With respect to Fig. 7B, as in Fig 7A, the laser illumination is reflected by the dichroic filter 472 through the objective 476 and focused on the channel containing the flowing particles. This illumination excites the fluorophores attached to the protein markers that are bound to the cells. The resulting fluorescent illumination is collected by the objective 476 and because of the longer wavelength of this emission passes through the dichroic filter 472 and is reflected by the beam splitter 468 through the high pass filter 470. The high pass filter 470 blocks any reflected laser illumination. The focusing lens 466 focuses the multi-wavelength emission illumination on the slit 478. The concave grating 482 images the slit at multiple wavelengths on the elements of the PMT array 476. This completes the process of creating a multispectral detection of the fluorescent emission. While most of the illumination collected by the objective 476 is reflected by the beam splitter 468 a small fraction is allowed to pass through and is focused through a pinhole 464 on the silicon reader unit 462. During the focusing operation best focus is achieved when the signal on this reader unit is maximized. When this signal is maximized, the intensity of the signal on the PMT array 476 is also maximized.

Fig. 8A is a schematic representation of one example of multi-wavelength excitation in the optical unit of Fig. 7A or 7B, in accordance with an embodiment of the present invention;

Reference is now made to Fig. 8A, which is a schematic representation 500 of one example of multi-wavelength excitation in the optical unit of Fig. 7A or 7B, in accordance with an embodiment of the present invention. Figs. 8A-8C show an extension of the optical configuration in Figs. 7A and 7B, to allow multiple excitation wavelengths.

Fig. 8A shows the configuration for combining multiple lasers of different wavelengths to yield a single coaxial beam 514 containing all of the wavelengths. Two different wavelengths, such as green 502 and red 506, may be combined using a dichroic mirror 504. One of the beams, red 506 is reflected by the dichroic mirror, while the second beam, green 502 passes through the dichroic mirror to yield a single beam 508, yellow, containing both wavelengths. This combined wavelength beam is now used as one of the inputs to a second dichroic mirror 516 with the third wavelength 512 being reflected by the second dichroic mirror to yield a single coaxial beam 510 containing all three wavelengths.

Reference is now made to Fig. 8B, which shows a graphical output 520 of transmission as a function of wavelength for dichroic filter 500 of Fig. 7B, employing the multi-wavelength excitation of Fig. 8A, in accordance with an embodiment of the present invention.

A multiband dichroic mirror (not shown) similar, or identical to, mirror 552 of Fig. 8C is used to illuminate the sample through an objective 554 (Fig. 8C), while allowing the resulting emission to pass through dichroic mirror 552 at all wavelengths, except those of multibeam excitation 514 (Fig. 8A).

In this way the same epi-configuration used with a single wavelength can, in fact, be used with appropriate changes to dichroic mirror 552 and the addition of multiple lasers 502, 506, 512 to provide multi-wavelength excitation, while maintaining virtually all of the detection wavelengths of a single excitation system.

Turning to Fig. 8C, a schematic representation of part 550 of the optical unit is seen, employing multi-wavelength excitation of Fig. 8A and the dichroic filter of Fig. 5B, in accordance with an embodiment of the present invention. Part 550 may, in some cases, replace subsystem 475 (Fig. 7B).

Table 1 shows representative values for representative components for use in the present invention.

| | | |
|---|---|---|
| Laser Wavelength | 405nm | 488nm |
| Laser Power | 50mW | 20m W or 50mW |
| Sensing Spectral Range | 200nm | 200nm |
| Spectral Resolution | 25nm | 25nm |
| Number of Detectors | 8 | 8 |
| Collecting Optics | Microscope Objective N.A.X1.4, W.D. ≈ 6mm | Microscope Objective N.A.>0.4, W.D. ≈ 6mm |
| Detector Type | S.S. PMT 8 ch | S.S. PMT 8 ch |

While much of the previous discussion has focused on the optical elements of some embodiments of the present invention, one of the key components of the diagnostic system for carrying out the present invention, herewith presented, is a disposable sample cartridge.

Reference is now made to Fig. 9A, which is a schematic view of a sampling cartridge 110 of Fig. 1A, in accordance with an embodiment of the present invention. The cartridge 650 includes a pre-analytical component 652 into which a sample (not shown) may be introduced.

The sample will generally be blood, either whole or a component (serum, etc.) thereof. Other liquid samples may additionally or alternatively be employed. In the pre-analytical component 652, the sample is allowed to interact with chemicals prepackaged into component 652. The interaction may be either passive or include active mixing. The chemicals included in the analytical component 652 may be either wet or dry, and generally include antibodies associated with fluorescent probes. Antibodies are pre-selected for their ability to bind with predetermined biological markers or the like. In a typical experiment, a predetermined volume (generally less than 50 microliters) of blood is introduced into the pre-analytical component 652 of a disposable cartridge 650. The sample is actively mixed with chemical reagents present in the pre-analytical component 652 for a predetermined period of time, generally less than ten minutes. The sample is then moved through a capillary region 653 by means to be discussed, where it is exposed to a light beam 642 delivered from an objective 638. Direction of sample flow is as shown by the arrow in the capillary region 653.
The capillary region 653 is designed to allow flow of particles in a single-file past the light beam 642. Such an arrangement allows both for counting the number of particles as well as individual interrogation of particles to determine the presence of biological markers (via their associated fluorescent tags) on each particle. Such a physical arrangement allows for detection of one or more biological markers (independent of particle-specific properties such as size, shape, and number) on each particle.

Finally, there is a collection component 654 which receives sample after exposure to light beam 642. This is a waste region and allows for a completely self-contained disposable for sample preparation, analysis and waste collection. It is noted that the disposable cartridge may be of any relevant shape and is shown as it is in Fig. 6 for ease of understanding of its components and functionality.

As mentioned above, the sample, after pre-analytical treatment to allow for binding of fluorescent tag to cells/particles, must flow under a light beam 642, produced by an optical unit (not shown). The flow is generally "single file" so as to allow for accurate determination of cell-specific markers on each analyzed cell. Methods to induce flow include electrical stimulation, chemical induction, and vacuum pull. In an electrical stimulation system, charge is applied across the capillary region 653 so as to induce charged particles to move from the pre-analytical component 652 towards the collection component 654. The charge could be supplied by the cytometer in which the disposable cartridge 650 is placed or from an external source.

Alternatively, the capillary region may include chemical features (hydrophilic/hydrophobic; positive/negative charge) to encourage sample to move from left to right as shown in Fig. 9A. Alternatively, a vacuum from the collection component 654 could be applied to pull sample from the pre-analytical component 652 through the capillary region 653. Other methods of the present invention may be employed to get liquid sample to move underneath the light beam 642 for analysis.

As described herein, the optics and sample handling have been handled separately. Such an arrangement is not mandatory, as some of the optical features needed for proper sample analysis may be included in a disposable cartridge.

Reference is now made to Fig. 9B, which shows a schematic view of disposable cartridge 800 in flow-cytometer device, such as system 100 in accordance with an embodiment of the present invention. Attention is currently turned to Fig. 9B which shows an expanded view of a capillary region 853.

In the capillary region 853, particles flow in the direction as suggested by the arrow 880. Particles 890 flow past an objective 838 that shines light 842 through the capillary 853. Flow restriction elements 894 may be present in the capillary region 853 so as to encourage particles 890 to move past the light 842 in a nearly single-file manner. Passage of multiple particles together may be resolved through processing software.

A molecular marker 895 on a particle 890 may be illuminated by light 842 and its fluorescence will be captured by a proximate photomultiplier tube 899. The photomultiplier tube 899 may distinguish the wavelength of the fluorescence and thus which biological marker 895 is present on particle 890. Thus, the systems used for carrying out the methods of the present invention may determine which biological markers are present on particles 890, which are detected in the systems used for carrying out the methods of the present invention. A photomultiplier tube 899 may have a plurality of tubes or an array of elements for fine wavelength discrimination and alternatively may be replaced with film, CCD or other appropriate light-receiving reader unit. It should be understood that Fig. 9B shows one embodiment of the configuration of system 101 (Fig. 1) in a transmissive configuration, wherein detector (photomultiplier tube 899) is disposed on an opposing side of the cartridge 800 to objective 838.

The systems used for carrying out the method of the present invention comprise controller software which is adapted to run a diagnostic process. It is understood that the controller software may be an integral part of the flow-cytometer or alternatively be installed on an associated computing device 122 (Fig. IB), which may include a laptop computer, iPod, iPad, cell phone or mainframe computer.

Reference is now made to Fig. 10, which is a simplified flowchart 1000 of a method for rapid determination of a medical condition, in accordance with an embodiment of the present invention. It is to be understood that the method described herein depicts an embodiment of the present invention for determining the health state of a patient. Further embodiments are also construed to be part of the present invention.

In a body fluid provision step 1002, a body fluid, such as blood, urine, serum or plasma is provided from a human or animal patient. Typically, the sample is fresh, but may also be a stored, refrigerated or frozen-thawed sample. The fluid is typically liquid and at a temperature of 4-37 °C.

In a body fluid introduction step 1004, part or all of the body fluid sample 6051 (Fig. 6) is introduced into disposable cartridge (110, Fig. 1A).

In a reacting step 1006, the fluid sample is reacted with at least one reactant in the cartridge forming a treated sample. According to some embodiments, this step is performed in pre-analytical sample processing section 6054 (Fig. 6) as described in detail hereinabove.

In an impinging step 1008, radiation is impinged on the treated sample, such as in a sample excitation/interaction section 6056, thereby generating a plurality of spectrally distinct signals in the direction of optics unit 142 (Fig. 1C, see description hereinabove).

In a spectral emissions detection step 1010, a plurality of spectrally distinct signals is detected by multiple emission detector 454 (Fig. 7A). The detector outputs data.

Thereafter, in a data processing step 1012, the outputted data is processed by signal processor 6036 (Fig. 6) and/or by computer 122 (Fig. 1C) to provide an output indicative of a medical condition.

Fig. 11 is a three-dimensional graph showing the optical output over time of reference beads (RM) relative to a sample from a human patient (PMN), in accordance with an embodiment of the present invention.

Fig. 11 shows a three-dimensional graph showing the optical output over time of reference beads (RM) relative to a sample from a human patient (PMN), in accordance with an embodiment of the present invention. The emission amplitude in the six bands, 500-525 nm, 525-550 nm, 550-575 nm, 575-600 nm, 600-625 nm and 625 to 650 nm is displayed in the graph for each sample time. Different fluorophores have different emission spectra. It can be appreciated that both spectral content or shape and amplitude at individual wavelengths are significantly different for neutrophils stained with Acridine Orange (AO) and reference beads (RM) containing a bright broad spectrum fluorophore. The peak of the AO emission is in the 525-550 nm band, while that of RM is in the 500-525 nm band and is of a significantly greater amplitude than AO in any band.

Figs. 12A-12C show graphs of optical outputs over time of the reference beads and the sample from a human patient, in accordance with an embodiment of the present invention.

Turning to Figs. 12A-12C, there can be seen graphs of optical outputs over time of the reference beads and the sample from a human patient, in accordance with an embodiment of the present invention. In these two-dimensional figures, the traces from each of the bands are overlaid on the same graph. Fig. 12A shows the boxed pulses from neutrophils in Fig. 12B. It is clear from these graphs that the amplitude in the 525-550 nm channel exceeds the amplitude in the 500-525 nm channel, which is the characteristic of AO. Fig. 12C shows a comparison of the AO stained neutrophil emission spectrum to that of the RM emission spectrum. The relative amplitude of the spectrum in the 500-525 nm band to that of the amplitude in the 525-550 nm band clearly distinguishes the two fluorophores. In addition, the maximum amplitude of the RM emission is significantly greater than that of AO.

In an embodiment, described and shown herein are uses, such as at least one of the four following scenarios:

a) When multiple pieces of information, such as biological markers and white cell state are required in order to make an accurate diagnostic determination;
b) When multiple sequential measurements must be made in order to determine the position of a patient on an illness curve;
c) When white cell and similar data are needed quickly and in a POC environment; and
d) When fluorescent signals overlap in wavelength and there is need to determine relative contribution of each signal for a given wavelength range.

The instant invention includes software and algorithms for proper data analysis and conversion of raw fluorescence data into actual concentrations of relative biological markers.

Fig. 13A is an outer side view of a cartridge assembly 1300, in accordance with an embodiment of the present invention and Fig. 13B shows an inner side view 1350 of a cartridge assembly 1300, in accordance with an embodiment of the present invention.

Fig. 14A-14O show a sequence of process events in a cartridge assembly, in accordance with an embodiment of the present invention;
Fig. 14A-14O are a sequential set of schematic drawings of the operation of a system 101 (Fig. 1A) for detecting a biological condition, in accordance with an embodiment of the present invention.

In Fig. 14A, a blood sample 1401 enters a specimen receiving element 1418 and fills a chamber 1404.

In Fig. 14B, a blister 1420 comprising a treatment composition 120 (Fig. 1) is pressed and an antibody cocktail is mixed with 10 microliters of the blood sample.

In Fig. 14C, a mixing bellows 1415 is pressed and this effects mixing of the antibody cocktail and the 10 microliters of the blood sample in a first mixing chamber to form a first mixture 1403.

In Fig. 14D, the bellows is released and mixture 1403 is siphoned along a tortuous channel 1413 and into a second mixing chamber 1411. Upon release of the bellows, the first mixture returns from the second mixing chamber, back along the tortuous channel to the first mixing chamber. Every time the bellows is pressed the mixture moves towards the second chamber and every time it is released, it returns, wholly or in part to the first chamber. This mixing may be performed multiple times.

In Figs. 14E-14G, a second composition blister 1422 is pressed, releasing a second composition 122 (Fig. 1), such as a lysis composition thereby forming a second mixture 1405. The second mixture is mixed by pressing of bellows 1415, the second mixture returns from the second mixing chamber, back along tortuous channel to the first mixing chamber. Every time the bellows is pressed the mixture moves towards the second chamber 1411 and every time it is released, it returns, wholly or in part to the first chamber 1412. This mixing may be performed multiple times.

In Figs. 14H-14J, a third blister 1424 is released comprising a third composition 124 (Fig. 1), such as a control reference, into the second mixing chamber, thereby forming a third composition 1407. The third mixture is mixed by pressing of bellows 1415, the third mixture returns from the second mixing chamber, back along tortuous channel 1413 to the first mixing chamber. Every time the bellows is pressed the mixture moves towards the second chamber 1411 and every time it is released, it returns, wholly or in part to the first chamber 1412. This mixing may be performed multiple times.

In Figs. 14J-14M, a reading bellows 1417 is pressed, which forces some of the third composition towards a reading cuvette 1430.

In Figs. 14N-14O, particles 1460 from the third composition flow from the cuvette 1430 along a reading channel 1452 to a reading region 1450. The cells pass through the reading region and are excited by one or more lasers 1462, 1463. At least one excitation laser beam 1464 impinges on cell 1460 and an emission beam 1466 is detected by a detector 1470. In one example, this is cell emission fluorescence and detector 1470 is a spectrometer.

Fig. 15 is a schematic illustration of a micro flow spectrometer reading, in accordance with an embodiment of the present invention;

An individual cell 1505 flows through a detection region 1510 in a microfluidic channel (seen as 1452, Fig. 14M). Additionally, tagged cells 1520 labeled with antibodies conjugated with multiple wavelength fluorescent tags flow through the detection region. A diode laser 1530 impinges a ray/beam 1510 onto the cells and tagged cells. The cells and tagged cells emit different emission spectra (not shown). An optical grating 1540 disperses emission spectra via a grating 1540 into its constituent wavelengths 1550.

A photomultiplier tube (PMT) array 1560 or avalanche diode array detects fluorescence at 8 different spatial locations corresponding to 8 spectral regions.

Fig. 16 is a flow chart of a method of the present invention for optical processing 1600.

In a forming laser step 1602, a laser excitation beam shape is formed.

The excitation beam is reflected from a dichroic mirror 504 (Fig. 8A, or 472 Fig. 7B) and through objective 476 (Fig. 7B) onto a reading channel 1452 (Fig. 14M), in a reflecting step 1604.

In a forward scatter measuring step 1606, the forward scatter from particles 1460 (Fig. 14N) in the reading channel is measured to detect events.

Thereafter, in a passing step 1608, particle fluorescent emission is allowed to pass through a dichroic mirror and be reflected from a beamsplitter 468 into a detection path.

In an imaging step 1610, parts of the beam emission, which are not reflected are passed through the beamsplitter onto an image sensor, such as silicon detector 462 (Fig. 7B).

In parallel to step 1610, the reflected part of the beam is filtered in a beam filtering step 1612 in the detection path to allow only wavelengths above an excitation wavelength to pass through a filter.

In a focusing step 1614, the filtered beam from step 1612 is focused onto a pinhole or slit to select a reading zone region to be analyzed.

Thereafter, in a dispersing step 1616, the dispersed pinhole or slit is dispersed and imaged onto a multi-element electrooptical detector (6034, Fig. 6).

Figs. 17A-17B are schematic illustrations of steps of use of a graphical user interface, in accordance with an embodiment of the present invention;

Upon powering up the unit a first screen 1702 appears with a message notifying the user that the system is performing a self-check along with a countdown indicator 1703. Once the self-check is complete, an assay selection screen 1704 appears. The user touches the button corresponding to the assay to be performed. The next screen 1706 is used to enter the patient identification. This may be done by touching the numerals of the touchpad 1709 or by scanning a barcode. Once the entry is complete, the user touches the forward button 1707 and a screen requesting the user to enter the cartridge 1708 appears. Once the user inserts the cartridge the system checks to ensure that the cartridge identified by its barcode label corresponds to the selected assay and begins the processing. While processing, a screen 1710 is displayed showing the processing progress and the time remaining. Once the pre-analytical and analytical processing is completed the results are displayed on a screen 1712 with an indication of where the results lie in the range of possible results 1713. After the user touches a "proceed to next screen indicator" 1711 a screen instructing the user to remove the cartridge 1714 appears. The user has the option of repeating this test with another sample by pressing the repeat icon 1715 or displaying the most recent results on a screen 1716.

Reference is now made to Fig. 18, which is a simplified illustration of a method of the present invention for a disposable cartridge 1850 of system 101 of Fig. 1A for rapid determination of a medical condition, in accordance with an embodiment of the present invention.

When practicing the method of disposable cartridge 1850 a bodily fluid, such as blood, urine, serum or plasma is transferred from the donor to the cartridge 1851. The disposable cartridge method includes multiple steps to effect the analysis and diagnosis 1852, 1854, 1856 and 1858. In step 1852 a body fluid aspiration step, receives the body fluid directly or indirectly from the patient (or animal) and transfers the body fluid to a reservoir.

The disposable cartridge method 1850 utilizes fluid conveying means, such as air pressure, liquid pressure, mechanical means and combinations thereof to move fluids. Body fluid aspiration step 1852 is adapted to convey a predetermined quantity of the body fluid (a body fluid sample 1851) for a pre-analytical sample processing step 1854.

In pre-analytical sample processing 1854, at least one preparatory step is performed on the body fluid such as:

i) incubation with at least one antibody;
j) incubation with at least one antigen;
k) staining of at least one cell type in the body fluid;
l) enzymatic lysing of at least one cell type of the body fluid;
m) osmotic lysing of at least one cell type of the body fluid;
n) heating or cooling at least part of the bodily fluid;
o) addition of reference material to the bodily fluid; and
p) chemical reaction with at least one element of the body fluid.

The pre-treated sample of bodily fluid is then transferred (1855) after pre-analytical sample processing step 1854 to a sample excitation/interaction step 1856.

This pre-treated sample transfer for excitation/interaction 1856 may be performed continuously or in a batch mode.

Part of sample excitation/interaction 1856 is to position the sample to sit in the light path of an excitation illumination. The excitation illumination passes radiation, such as coherent or incoherent radiation in or outside the visible range into the pre-treated sample. Resultant emission or emissions from the pre-treated sample is detected 1834, and processed 1836 to produce a report 1812 summarizing the analysis and diagnosis.

Multi-spectral emission detection 1834 receives the emission from the pre-treated sample in multiple spectral bands. In some cases these bands are nonoverlapping bands. Multi-spectral emission detection 1834 is adapted to pass data representing the spectral bands to multi-spectral fluorescence signal processing 1836.

Multi-spectral fluorescence signal processing 1836 may comprise two or more sub-elements (not shown) including:

a) a photon counting analysis;
b) other detecting analysis elements (not shown) for measuring other optical outputs of multi-spectral emission detection 1834.

In an embodiment, the method of the present invention further comprises a spent sample disposal method 1858, for receiving a sample from the sample excitation/interaction processing.

In an embodiment, the method of the present invention further comprises computer program 1810, the computer program is adapted to receive data related to the plurality of spectrally distinct signals and a processor, adapted to process said data and to output at least one output related to said medical condition. One type of output provided is a visual output which is outputted onto a screen 1812 of the computer.

Fig. 19A is a simplified flow chart of a method 600 of the present invention for differentiating between different particles.

The input to the processing is a time series from each of the channels in the eight channel photomultiplier array 601. In addition, data from multiple scatter channels 609 is introduced. Each fluorescent time series and scatter time series may be processed individually employing respective spectral cross-correlation algorithm 606 and scatter algorithm 607 to smooth it and minimize noise. Two possible processing methods are boxcar averaging algorithm 602 and matched filtering algorithm 604. In addition, groups of individual channels may be correlated to yield a multiple spectral crosscorrelations 606. One or more of these derived time series may be used to determine event locations.

Once an event is located in the eight channel time series the composition of that event in terms of known fluorophore signatures is determined using a minimum mean square error fit 610. The event is now described in terms of its composition of known fluors. Each event thus described is stored in an event store, i.e. memory, together with the data from the eight time series for that event and its description 612. Based on the fluor composition for each event in the data store, it is possible to determine the type of particle. For example, a neutrophil 616 is characterized by the single fluor attached to the CD64 antibody shown in Fig. 5 as Wl. Thus events that are preponderantly characterized by the single fluor attached to the CD64 antibody are identified as neutrophils.

Similarly, monocytes 618 are characterized by fluors Wl and W2 so that an event with both of these fluor signatures is identified as a monocyte. Similarly, a bead 620 is characterized by an event that has fluors W1 and W3. Lymphocytes 622 do not express significant fluorescence but are identified by their scatter as events. Events that do not match any of the known combinations of the fluorophores are identified as rejects 626.

Given the population of identified events, the median intensity of the neutrophil population and the median intensity of the bead population are determined. The ratio of the neutrophil median to the bead median is the desired Leuko64 index. The positive control value is determined as the median intensity of the CD64 fluorophore bound to monocytes divided by the median intensity of the same fluorophore on the bead population. The negative control value is determined by the median intensity of the CD64 fluorophore bound to lymphocytes. These are the key steps in performing the Leuko64 assay.

Fig. 19B is a flow chart of an algorithm for biological detection 1900, in accordance with an embodiment of the present invention;

Figure 19B shows schematically that the CD4/CD8 assay is performed by determining a particle type for each event in the event store 1912. One way to accomplish this particle selection is to use K means clustering to determine data clusters in the event store based on the signatures Alexa 488, PE 488N and PEAF 488N as shown in Figure 25. Based on these three signatures events with large values of PE 488N are classified as CD4 positive lymphocytes 1938 since the phycoerythrin (PE) fluor is attached to the CD4 antibody. Events with large values of Alexa 488 are classified as CD8 positive lymphocytes 1940 since the Alexa 488 fluor is attached to this CD8 antibody. Events with large values of PEAF 488N are classified as lymphocytes since this fluor is attached to the CD3 antibody which is expressed by lymphocytes to the exclusion of other WBC. The group 1942 has large values of PEAF 488N but small values of PE 488N and Alexa 488 which corresponds to lymphocytes not expressing either CD4 or CD8. Finally, non-lymphocytes WBC 1936 may be determined by a pan WBC antibody for those events not expressing CD3, and rejects as those events not expressing the pan WBC antibody.

Figs. 20A-20B shows bandwidth leveled and smoothed arrays, in accordance with an embodiment of the present invention.

Figs. 20A and 20B show the typical response of the system for carrying out the invention to fluorescent beads with an F488 signature. The response in 25 nm bands from 500 nm to 700 nm are respectively 2010, 2020, 2030, 2040, 2050, 2060, 2070, and 2080. The traces represent the outputs from the eight channel fluorescent detector with noise, characterized as the median trace value, subtracted. The large signals above the background level are the raw signal smoothed by a box car averager of length 10. The peak value for the F488 signature occurs in the range 525 to 549 nm, 2020. The next highest value occurs in the range 500 to 524 nm, 2010, with decreasing amplitudes in each 25 nm band, 2030,2040,2050,2060, 2070, and 2080.

Figs. 21A-21B are schematics for solving a fluor decomposition of an observed signal, in accordance with an embodiment of the present invention.

Fig. 21A is the Matlab function used to solve the matrix equation Ax=b for the signature value vector or vectors b corresponding to the observed eight channel emission values x. The \ function in Matlab is used to solve for x using the Matlab expression x = A\b.

Fig. 21B is a table of fluor signatures use as the matrix A described in Fig. 21A.

Figs. 22A-22B is a graphical comparison of system performance with FITC beads with MESF detection versus FACS, in accordance with an embodiment of the present invention.

Fig. 22A shows a comparison of the linearity of the method of the instant invention with that of a Becton Dickinson FACS flow cytometer. The tabulated median values for the FITC MESF beads are shown in column 2211 in table 2210. The median fluorescent intensity levels (in arbitrary units) for the FACS flow cytometer are shown in column 2212, while those for carrying out the instant invention are shown in column 2213. Column 2214 shows the number of events on which the median value is based for the instant invention. The linearity plot for the full range of values for carrying out the instant invention is shown in 2220 while that for the FACS flow cytometer is shown in 2230. Also shown in each of these figures is the best fit line through the points along with the square of the correlation value, R2. Comparison of these plots shows comparable performance of the instant invention and the FACS flow cytometer.

Fig. 22B shows a comparison of the linearity of the instant invention 2240 with that of the FACS flow cytometer 2250 over the range restricted to the first four smallest data points. Again, comparable performance is demonstrated.

Figs. 23A-23B show graphical displays of linearity of system performance with Alexa 488 MESF, in accordance with an embodiment of the present invention.

Figs 23A and 23B shows the linearity performance of the instant invention for the Alexa Fluor 488 MESF bead series when the system is run both add fast speed 2310 and flow speed 2330. In this case the measure of performance is the F488 signature normalized to the length of the event. This normalized signature is designated F488N. The tabular listings 2320 and 2340 summarize the statistics for the regression line fit.

Fig. 24 is a three-dimensional graph showing the optical output over time of a CD4-CD8 assay, in accordance with an embodiment of the present invention.

Fig. 24 is a surface plot showing the relative amplitude of event emission in each of the detectors for a thirty second interval. The scale running from left to right 2440 is in 10 μs intervals. The scale running from right to left 2450 and numbered one through eight are the detector elements. Eight corresponds to waveband 1, seven corresponds to waveband 2, and finally one corresponds to waveband 8. An example of CD4 events tagged with phycoerythrin shown in 2510 in Fig. 25 is the trace 2430. An example of CD8 events tagged with Alexa Fluor 488 shown in 2520 in Fig. 25 is the trace 2410. Finally an example of non-CD4 non-CD8 lymphocytes tagged only with Alexa Fluor 610 shown in the group 2530 in figure 25 is the trace 2420.

Fig. 25 is a graphical display showing a cluster analysis of a CD4-CD8 assay, in accordance with an embodiment of the present invention;

Fig. 25 is the scatterplot matrix showing the result of applying K means clustering to the signatures Alexa 488N, PE488N and PEAF488N. The meaning of each of the clusters 2510, 2520 and 2530 is described in the description of figure 19 B.

Figs. 26-27 are graphical displays showing cluster separations of the cluster analysis of Fig. 19A, in accordance with an embodiment of the present invention.

Fig. 26 is a scatterplot matrix showing four-color separation of the neutrophil, monocyte, lymphocyte and reference bead populations required to effect a CD64 assay. The leftmost column 2710 shows the complete 4-color separation. The top frame 2720 shows separation of NE & LY from MO and BEADS based on Waveband2 (Alexa488) and separation of MO from BEADS based on Waveband4 (PE).

The middle frame 2730 shows separation of LY from NE based on Waveband6 (A610).

The bottom frame 2740 shows separation of beads from cells based on Waveband8 (Starfire Red).

Since the separation is based on individual narrow bands (not signatures) 45 degree clusters 2750,2760, 2770 show emission presence in two bands, which in each case is as expected as can be seen from the emission signatures in the table below.

Fig. 28 is a comparison table of different array options, in accordance with an embodiment of the present invention.

Fig. 28 is a tabular listing of photomultiplier arrays produced by Hamamatsu Corporation. The H95308 channel array is the one used in the current implementation of the extant invention. One skilled in the art will appreciate that finer resolution or greater extent of the spectral sampling can be achieved by using either the 16 or 32 channel array products.

Fig. 29A is a flowchart of a specific implementation of an algorithm 1300 for selecting groups of data from a scatterplot, in accordance with an embodiment of the present invention;

The algorithm in Fig. 29A is a specific implementation of the general algorithm in Fig. 29B to select each of the groups 3010, 3020, 3030 and 3040 (Fig. 30) and determine specific parameter values in each of the groups.

In a first ordering signature step 1304 the Star Fire Red (SFR) signature is used to order (from smallest SFR signature to largest) the entire dataset of waveband and signature values 1302.

In a second step 1320, an analysis of a histogram of an SFR signature values as shown in Fig. 14A to select the group 1210. This is a small group 1404 at the upper end of group 1402 in the histogram 1400 in Fig. 31 A. The next step is to remove this group from the overall dataset as shown in Fig. 29A Step 1322. The removed group is the bead dataset 1324.

A dataset of Waveband and Signature values with bead dataset removed 1340 is then manipulated as follows. In an ordering step 1342, the data is organized according to its PE (phycoerythrin) signature from smallest to largest PE (phycoerythrin) signature.

In an analyzing PE histogram set step, 1344, the data is manipulated to find a group corresponding to monocytes.

In an extracting monocytes dataset step 1346, a monocyte dataset of waveband and signature values 1348 is extracted. A dataset of waveband and signature values with beads and monocytes removed 1360 is then further processed as follows. Set 1360 is organized according to its PEAF (full name PEAF®488) (see above for beads and PE) signature in an order according to PEAF signature ordering step 1362.

In an analyzing PEAF histogram to find a group corresponding to lymphocytes step 1364, set 1360 is analyzed to determine if any of the data have behavior corresponding to lymphocytes.

In an extraction step 1366, a lymphocyte dataset of waveband and signature values 1368 is extracted from set 1360 and the remaining dataset is a dataset of waveband and signature values with bead, monocytes and lymphocytes removed 1380.

In an order by Diode 1 signature step 1382, dataset 1380 is analyzed according to a Diode 1 signature (see above). Dataset 1380 is then analyzed in an analyzing step 1384 to find a group of data having properties of neutrophils.

In an extracting step 1386, a group of data having properties of non-neutrophils 1388 is removed. A remaining group 1391 (assumed to be neutrophils) is used in a computing step 1392 to compute desired metric from the group parameters.

Reference is now made to Fig. 29B, which is a flowchart of a general implementation of an algorithm 1350 for selecting groups of data from a scatterplot, in accordance with an embodiment of the present invention.

In a first ordering signature step 1305 a first signature is used to order the dataset of waveband and signature values 1303.

In a second step 1321, an analysis of a histogram of a 1$^{st}$ signature values to find the group corresponding to 1$^{st}$ signature 1325, as exemplified in Fig. 31A to select the group 3010. This is a small group 1404 at the upper end of group 1402 in the histogram 1400 in Fig. 31 A. It should be noted that this is but one way to select the group and other methods employing additional data set values in combination may be used. The next step is to remove this group from the overall dataset as shown in Fig. 31B Step 1323. A removed group is a 1$^{st}$ signature dataset 1325.

A dataset of Waveband and Signature values with 1$^{st}$ dataset removed 1341 is then manipulated as follows. In an ordering step 1343, the data is organized according to its 2$^{nd}$ signature.

In an analyzing 2$^{nd}$ signature histogram set step, 1345, the data is manipulated to find a group corresponding to the 2$^{nd}$ signature.

In an extracting 2$^{nd}$ signature dataset step 1347, a 2nd signature dataset of waveband and signature values 1349 is extracted. A dataset of waveband and signature values with 1$^{st}$ and 2$^{nd}$ signatures groups removed 1361 is then further processed as follows. Set 1361 is organized according to its i$^{th}$ signature in an order according to i$^{th}$ signature ordering step 1363.

In an analyzing i$^{th}$ histogram to find a group corresponding to i$^{th}$ signature step 1365, set 1361 is analyzed to determine if any of the data have behavior corresponding to the i$^{th}$ signature.

In an i$^{th}$ signature extraction step 1367, an i$^{th}$ signature dataset of waveband and signature values 1369 is extracted from set 1381 and the remaining dataset is a dataset of waveband and signature values with 1$^{st}$ 2$^{nd}$ and i$^{th}$ signature groups removed 1381.

In an order by N$^{th}$ signature step 1383, dataset 1381 is analyzed according to an N$^{th}$ signature. Dataset 1381 is then analyzed in an analyzing step 1385 to find a group of data having properties of not having $N^{th}$ signature properties.

In an extracting step 1387, a group of data having properties of non-$N^{th}$ signatures 1397 is removed. A remaining group 1395 (assumed to be $N^{th}$ group) is used in a computing step 1393 to compute desired metric from the group parameters.

Fig. 31A is a histogram 1400 of data of Starfire Red (SFR) signature values, in accordance with an embodiment of the present invention.

Fig. 31B shows a plot 1450 of a polynomial 1452 and first derivative thereof 1456 and second derivative thereof 1458 of histogram 1400 shown in Fig. 31 A, in accordance with an embodiment of the present invention.

Referring to Fig. 31B, the method of determining an upper group 1404 in Fig. 31A is as follows. A polynomial 1452 of sufficient degree is fitted to the histogram data 1454 (as shown in Fig. 13A, set 1324) is shown in Fig. 14B. The first derivative 1456 and the second derivative 1458 of this polynomial are computed. A plurality of zeros 1460 of the first derivative are indicated by the square boxes along the zero line. A point where the polynomial is both maximum and has a zero derivative 1462 is indicated by the box with an X in it. This point in the histogram corresponds to the peak of the large group 1402 (Fig. 31 A). A next zero 1464 of the derivative of the polynomial corresponds to the end of the large group in the histogram. All points in the histogram above this value are in the small group. Since the dataset has been ordered from smallest to largest based on the value of SFR488, and the histogram horizontal axis is also ordered from smallest to largest value of SFR488 the point at which the large group ends is the value of SFR488 above which records in the SFR488 ordered dataset are to be removed and identified as the bead dataset 1324 of waveband and signature values as indicated in Fig. 13A.

Fig. 32A is a histogram 1500 of data of PE488 signature values, in accordance with an embodiment of the present invention.

Fig. 32B shows a polynomial fitted to the histogram in Fig. 32A as well as a corresponding first derivative 1556 and a second derivative 1558, in accordance with an embodiment of the present invention.

The records remaining in the dataset are now reordered using the PE488 signature from smallest to largest. Histogram 1500 of the PE488 signature values 1502 is shown in Fig. 32A. Again in this case, there is a small group 1504 to the right of the large group 1502 which corresponds to the desired monocyte population. Fig. 32B shows the polynomial 1552 fitted to data 1554 of histogram 1500 in Fig. 32A as well as the corresponding first and second derivatives. The upper group 1504 is determined in the same way as the upper group of the SFR488 histogram as was described previously. It should be noted that while in both of these cases only a one dimensional histogram was analyzed and used as the basis for selecting the desired population, multiple fields from each record in the dataset may be used to effect a group selection.

As noted in Fig. 31 A, the monocyte group 1504 is removed from the dataset which now contains primarily lymphocytes, neutrophils and other particles such as unlysed erythrocytes and other debris.

Fig. 33A is a histogram 1600 of data 1602 of PEAF488 signature values, in accordance with an embodiment of the present invention.

Fig. 33B shows a polynomial 1652 fitted to histogram data 1654 from Fig. 33A as well as a corresponding first derivative 1656 and a second derivative 1658, in accordance with an embodiment of the present invention.

The records remaining in the dataset are now reordered using a PEAF488 signature corresponding to lymphocytes. A histogram 1600 of the PEAF488 signature is shown in Fig. 33A and the corresponding polynomial fit with its first and second derivatives are shown in Fig. 33B. The process outlined above is applied in this case as well to identify and remove a small group 1604 appearing at an upper end of the histogram, from a large group 1602. The lymphocyte group is now removed as shown in Fig. 29A leaving a dataset 1380 which now contains primarily neutrophils and other particles such as unlysed erythrocytes and other debris.

While neutrophils 1391 are tagged with a fluorophore with an F488 signature, other particles appear to express this signature because of the unbound fluorophore in solution. The other particles, however, are smaller than neutrophils, which now comprise the group with the largest forward scatter as measured by a Diode 1 (forward scatter detector) channel. A histogram of the Diode 1 channel is shown in Fig. 34A.

Fig. 34A is a histogram 1700 of data of Diode 1 channel signature values, in accordance with an embodiment of the present invention.

Fig. 34B shows a polynomial 1752 fitted to data 1754 from the histogram in Fig. 34A, as well as a corresponding first derivative 1756 and a second derivative 1758, in accordance with an embodiment of the present invention.

As described above, an upper group 1704 (Fig. 34A) corresponding to larger particles, which are the neutrophils is selected. This completes the decomposition of the original dataset 1302 into the four distinct event groups (1324, 1348, 1368, 1391) shown in Fig. 29A.

Within each group various parameters may be computed from the fields in the dataset. An example is shown in the following table.

| Observat Ions | NAM | MEDUG | MEDF4 88 | MEDWa veband2 | MEDWa veband2N | INDEX4 88 | INDEX Waveba nd2 | INDEXWaveb and2N |
|---|---|---|---|---|---|---|---|---|
| SFR488 | 166 | 978.72 | 3395.26 | 3062.00 | 503.80 | 1.00 | 1.00 | 1.00 |
| PE488 | 73 | 3851.88 | 5968.83 | 5843.50 | 723.66 | 1.76 | 1.91 | 1.44 |
| PEAF48 8 | 332 | 1164.38 | -4.36 | 37.00 | 4.63 | 0.00 | 0.01 | 0.01 |
| F488 | 620 | 379.98 | 379.98 | 361.00 | 37.92 | 0.11 | 0.12 | 0.08 |
| Diode1 | 159 | 7027.00 | -113.54 | -73.00 | -6.81 | -0.03 | -0.02 | -0.01 |

The observations column contains the name of the group. The NAM column is the number of events in the group. The MEDUG column is the median value of the signature for that group. For example in the SFR488 row the median SFR488 signature value is 978.72. The MEDF488 column contains the median value of the F488 signature for the specified group. The MEDWaveband2 column contains the median value of the Waveband2 values in the group. The MEDWaveband2N column contains the median value of the Waveband2N values in the group. The INDEX488 column contains the ratio of the MEDF488 value for the group to that of the SFR488 group. Similarly, INDEXWaveband2 and INDEXWaveband2N are the ratios of the Waveband2 and Waveband2N medians for the group to that of the SFR488 group.

Although, specific groups corresponding to leukocyte subsets and a specific algorithm to compute a specific index based on these groups has been illustrated, one skilled in the art can use this basic approach whenever it is necessary to select groups from a dataset and compute numeric values based on parameters associated with these groups as shown in the general diagram of Fig. 29B.

Other suitable operations or sets of operations may be used in accordance with some embodiments. Some operations or sets of operations may be repeated, for example, substantially continuously, for a pre-defined number of iterations, or until one or more conditions are met. In some embodiments, some operations may be performed in parallel, in sequence, or in other suitable orders of execution.

Discussions herein utilizing terms such as, for example, "processing," "computing," "calculating," "determining," "establishing", "analyzing", "checking", or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other information storage medium that may store instructions to perform operations and/or processes.

Some embodiments may take the form of an entirely hardware embodiment, an entirely software embodiment, or an embodiment including both hardware and software elements. Some embodiments may be implemented in software, which includes firmware, resident software, microcode, or the like.

Some embodiments may utilize client/server architecture, publisher/subscriber architecture, fully centralized architecture, partially centralized architecture, fully distributed architecture, partially distributed architecture, scalable Peer to Peer (P2P) architecture, or other suitable architectures or combinations thereof.

Some embodiments may take the form of a computer program product accessible from a computer-usable or computer-readable medium providing program code for use by or in connection with a computer or any instruction execution system. For example, a computer-usable or computer-readable medium may be or may include any apparatus that can contain, store, communicate, propagate, or transport the program for use by or in connection with the instruction execution system, apparatus, or device.

In some embodiments, the medium may be or may include an electronic, magnetic, optical, electromagnetic, InfraRed (IR), or semiconductor system (or apparatus or device) or a propagation medium. Some demonstrative examples of a computer-readable medium may include a semiconductor or solid state memory, magnetic tape, a removable computer diskette, a Random Access Memory (RAM), a Read-Only Memory (ROM), a rigid magnetic disk, an optical disk, or the like. Some demonstrative examples of optical disks include Compact Disk-Read-Only Memory (CD-ROM), Compact Disk-Read/Write (CD-R/W), DVD, or the like.

In some embodiments, a data processing system suitable for storing and/or executing program code may include at least one processor coupled directly or indirectly to memory elements, for example, through a system bus. The memory elements may include, for example, local memory employed during actual execution of the program code, bulk storage, and cache memories which may provide temporary storage of at least some program code in order to reduce the number of times code must be retrieved from bulk storage during execution.

In some embodiments, input/output or I/O devices (including keyboards, displays, pointing devices, etc.) may be coupled to the system either directly or through intervening I/O controllers. In some embodiments, network adapters may be coupled to the system to enable the data processing system to become coupled to other data processing systems or remote printers or storage devices, for example, through intervening private or public networks. In some embodiments, modems, cable modems and Ethernet cards are demonstrative examples of types of network adapters. Other suitable components may be used.

Some embodiments may be implemented by software, by hardware, or by any combination of software and/or hardware as may be suitable for specific applications or in accordance with specific design requirements. Some embodiments may include units and/or sub-units, which may be separate of each other or combined together, in whole or in part, and may be implemented using specific, multi-purpose or general processors or controllers. Some embodiments may include buffers, registers, stacks, storage units and/or memory units, for temporary or long-term storage of data or in order to facilitate the operation of particular implementations.

Some embodiments may be implemented, for example, using a machine-readable medium or article which may store an instruction or a set of instructions that, if executed by a machine, cause the machine to perform the method of the present invention and/or operations for carrying out the invention. Such machine may include, for example, any suitable processing platform, computing platform, computing device, processing device, electronic device, electronic system, computing system, processing system, computer, processor, or the like, and may be implemented using any suitable combination of hardware and/or software for carrying out the invention. The machine-readable medium or article may include, for example, any suitable type of memory unit, memory device, memory article, memory medium, storage device, storage article, storage medium and/or storage unit; for example, memory, removable or non-removable media, erasable or non-erasable media, writeable or re-writeable media, digital or analog media, hard disk drive, floppy disk, Compact Disk Read Only Memory (CD-ROM), Compact Disk Recordable (CD-R), Compact Disk Re-Writeable (CD-RW), optical disk, magnetic media, various types of Digital Versatile Disks (DVDs), a tape, a cassette, or the like. The instructions may include any suitable type of code, for example, source code, compiled code, interpreted code, executable code, static code, dynamic code, or the like, and may be implemented using any suitable high-level, low-level, object-oriented, visual, compiled and/or interpreted programming language, e.g., C, C++, Java, BASIC, Pascal, Fortran, Cobol, assembly language, machine code, or the like.

Functions, operations, components and/or features described herein with reference to one or more embodiments, may be combined with, or may be utilized in combination with, one or more other functions, operations, components and/or features described herein with reference to one or more other embodiments, or vice versa.

Any combination of one or more computer usable or computer readable medium(s) may be utilized. The computer-usable or computer-readable medium may be an electronic, magnetic, optical, electromagnetic, infrared, or semiconductor system, apparatus, device, or propagation medium for carrying out the invention. More specific examples (a non-exhaustive list) of the computer-readable medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CDROM), an optical storage device, a transmission media such as those supporting the Internet or an intranet, or a magnetic storage device. Note that the computer-usable or computer- readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory. In the context of this document, a computer-usable or computer-readable medium may be any medium that can contain, store, communicate, propagate, or transport the program for use by or in connection with the instruction execution system, apparatus, or device. The computer-usable medium may include a propagated data signal with the computer-usable program code embodied therewith, either in baseband or as part of a carrier wave. The computer usable program code may be transmitted using any appropriate medium, including wireless, wireline, optical fiber cable, RF, etc.

Computer program code for carrying out operations of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The present invention is described herein with reference to flow chart illustrations and/or block diagrams of methods of the invention, and apparatus (systems) and computer program products for carrying out the invention. It will be understood that each block of the flow chart illustrations and/or block diagrams, and combinations of blocks in the flow chart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer or other programmable data processing apparatus to function in a particular manner, such that the instructions stored in the computer-readable medium produce an article of manufacture including instruction means which implement the function/act specified in the flow charts and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for carrying out the invention specified in the flow charts and/or block diagram block or blocks.

The flow charts and block diagrams in the Figures illustrate the architecture, functionality, and operation of possible implementations of systems for carrying out the invention, methods of the invention and computer program products for carrying out the invention. In this regard, each block in the flow charts or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow chart illustrations, and combinations of blocks in the block diagrams and/or flow chart illustrations, can be implemented by special purpose hardware-based systems that perform the specified functions or acts, or combinations of special purpose hardware and computer instructions.

Although the embodiments described above mainly address assessing test coverage of software code that subsequently executes on a suitable processor, the methods and systems for carrying out the methods of the invention described herein can also be used for assessing test coverage of firmware code. The firmware code may be written in any suitable language, such as in C. In the context of the present patent application and in the claims, such code is also regarded as a sort of software code.

The cartridges may be constructed and configured such that the treatment composition comprises proteins attached to a surface, such as to beads. A plurality of beads or other structural elements with proteins attached to their surfaces can be made by any one or more of the following methodologies:-

- simple attachment such as by adsorption via electrostatic or hydrophobic interactions with the surface, entrapment in immobilized polymers, etc.
- non-covalent or physical attachment;
- covalent bonding of the protein to the bead surface
- biological recognition (e. g., biotin/streptavidin).
- requires two steps: a first layer is formed by silane chemistry such that the surface presents a reactive group (e. g., epoxy, amino, thiol, etc.), and a second layer (e. g., the protein to be immobilized or a linker molecule) is covalently attached via the immobilized reactive groups.
- covalent attachment to functionalized polymer coatings on the interior of the device or linkage to the free end of a self-assembled monolayer (SAM) on a gold surface.

The reaction type may include any one or more of antigen-antibody binding, sandwich (such as antibody-antigen-antibody), physical entrapment, receptor-ligand, enzyme-substrate, protein-protein, aptamers, covalent bonding or biorecognition.

Table 2 shows some representative applications of apparatus 100 for carrying out the invention and methods of the present invention.

Table 2 Applications of the apparatus for carrying out the invention and methods of this invention.

| Application | Type of Test | Relevant Figures in this invention | Typical Prior Art Laboratory Turnaround time (TAT)-see references | This invention Turnaround time (TAT) | References |
|---|---|---|---|---|---|
| Application #1 - CD64 Infection & Sepsis | Surface Marker | Figs. 1-2 and 3-5D | 4 hours | 10 minutes | US 8,116,984, Davis, BH et al., (2006) |
| 1 - Fetal Hemoglobin Test | Plasma Protein | Figs. 1-2 and 6-8D | 4 hours | 10 minutes | Dziegiel et al. (2006) |
| 2 - Low Platelet Count | Surface Marker | Figs. 1-2 and 3-5D | 4 hours | 10 minutes | Segal, H. C., et al. (2005): |
| 3 - Resolving BLAST Flag for hematology Lab | Surface Marker | Figs. 1-2 and 3-5D | 4 hours | 10 minutes | Guerti, K., et al. |

| | | | | | |
|---|---|---|---|---|---|
| 4 - CD34 Stem Cell Enumeration Assay | Surface Marker | Figs. 1-2 and 3-5D | 4 hours | 10 minutes | Sutherland et al. (1996) |
| 5 - Platelets Activation Assay CD62 | Surface Marker | Figs. 1-2 and 3-5D | 4 hours | 10 minutes | Graff et al. (2002) Divers, S. G., et al. (2003) |
| 6 - D-dimer (Bead based protein) | Plasma Protein | Figs. 1-2 and 6-8D | 4 hours | 10 minutes | Stein et al. (2004) Rylatt, D. B., et al. (1983): |
| 7 - Chorioamnioitis CD64 | Surface Marker | Figs. 1-2 and 3-5D | 4 hours | 10 minutes | Hillier et al. (1988) |
| 8 - CD20 Cell Quantitation (Therapy Monitoring | Surface Marker | Figs. 1-2 and 3-5D | 4 hours | 10 minutes | Rawstron et al. (2001) Cheson et al. (1996) |
| 9 - CD52 Cell quantitation (Therapy Monitoring) | Surface Marker | Figs. 1-2 and 3-5D | 4 hours | 10 minutes | Rawstron et al. (2001) |
| 10 - Circulating Tumor Cells | Surface Marker | Figs. 1-2 and 3-5D | 4 hours | 10 minutes | Cristofanilliet al. (2004 |
| 11 - Reticulated Platelet Assay | Surface Marker | Figs. 1-2 and 3-5D | 4 hours | 10 minutes | Matic et al. (1998) Ault et al (1993) Wang et al. (2002) |

| | | | | | |
|---|---|---|---|---|---|
| 12 - Bacteria Detection in platelet packs | | | 4 hours | 10 minutes | Blajchman et al (2005) McDonald et al. (2005) |
| 13 - Platelet Associated Antibodies | Surface Marker | Figs. 1-2 and 3-5D | 4 hours | 10 minutes | Michelson (1996) |
| 14 - Residual Leukocyte Count in blood products | Surface Marker | Figs. 1-2 and 3-5D | 4 hours | 10 minutes | Bodensteiner, (2003) |
| 15 - CD4 HIV AIDS | Surface Marker | Figs. 1-2 and 3-5D | 4 hours | 10 minutes | Rodriguez (2005). Dieye et al. (2005) |
| 16 - Leukemia Panels - Very complex | Surface Marker | Figs. 1-2 and 3-5D | 4 hours | 10 minutes | Drexler et al (1986) |
| 17 - Bladder Cancer Screening in Urine - Urine sample | Surface Marker | Figs. 1-2 and 3-5D | 4 hours | 10 minutes | Ramakumar et al (1999) Lotan et al. (2009) |
| 18 - HLA DR Sepsis and Immunosuppression | Surface Marker | Figs. 1-2 and 3-5D | 4 hours | 10 minutes | Hershman et al. (2005) Perry et al (2003) |
| 19 - RECAF Protein for Canine and other Cancers | Plasma Protein | Figs. 1-2 and 6-8D | 4 hours | 10 minutes | Moro et al. (2005). |

| | | | | | |
|---|---|---|---|---|---|
| 20 - CytoImmun - Cervical Screening | | | 4 hours | 10 minutes | Hilfrich et al. (2008) |
| 21 - Procalcitonin (Bead Based Protein) + Feasibility | Plasma Protein | Figs. 1-2 and 6-8D | 4 hours | 10 minutes | Assicot et al. (1993) Christ-Crain et al. (2004) |

It should be understood that each of the steps of the method of the present invention may take a predetermined period of time to perform, and in between these steps there may be incubation and/or waiting steps, which are not shown for the sake of simplicity.

According to some embodiments, the volume of the specimen or sample is less than 200 μL, less than 100 μL, less than 50 μL, less than 25 μL or less than 11 μL.

Typically, the total sample volumes are in the range of 10 to 1000 μL, 100 to 900 μL, 200 to 800 μL, 300 to 700 μL, 400 to 600 μL, or 420 to 500 μL.

According to some embodiments, the volume of the treatment composition chambers 106, 108, 110 (also called blisters) is from about 1 μL to 1000 μL. According to other embodiments, the volume of the specimen is from about 10 μL to 200 μL.According to other embodiments, the volume of the specimen is about 1, 10, 20, 30, 40, 50, 60, 70, 80, 90, 100, 120, 140, 160, 180, 200, 250, 300, 350, 400, 450, or 500 μL.

According to some embodiments, the volume of the treatment compositions 120, 122, 124 is at most about 500 μL. According to other embodiments, the volume of the specimen is at most about 200 μL. According to other embodiments, the volume of the specimen at most about 500, 450, 400, 350, 300, 250, 200, 180, 160, 140, 120, 100, 90, 80, 70, 60, 50,40, 30,20, 10, or 1 μL.

According to some embodiments, the volume of a reactant is at least about 1 μL. According to other embodiments, the volume of the specimen is from about 10 μL. According to other embodiments, the volume of the specimen is at least about 1, 10, 20, 30, 40, 50, 60, 70, 80, 90, 100, 120, 140, 160, 180, 200, 250, 300, 350, 400, 450, or 500 μL.

The sequence of transfer of the various treatment compositions may be important to the reaction sequence and is typically predefined.

The reading region 1450 (Fig. 14M-N) is configured and constructed for one or more evaluation steps. These may include any of the following, or combinations thereof:

a) transfer of radiation there-through,
b) impinging radiation thereupon;
c) detecting reflected, refracted, and/or transmitted radiation,
d) detecting emitted radiation;
e) capturing one or more images thereof;
f) performing image analysis on the captured images;
g) measuring electrical characteristics of the treated specimen;
h) impinging sonic energy thereon;
i) detecting sonic energy therefrom; and
j) analyzing the outputs of any one or more of the above steps.

According to some embodiments, the cartridge is introduced into a system as described in International patent application publication no. WO2011/128893 to Kasdan et al..

According to some embodiments; the apparatus may have on-board means for showing a result, such as a colorimetric strip (not shown). Additionally or alternatively, the results are displayed in a display unit, separate and remote from system 101.

The blood sample is typically whole blood recently removed from a patient. The whole blood comprises mainly red blood cells (also called RBCs or erythrocytes), platelets and white blood cells (also called leukocytes), including lymphocytes and neutrophils. Increased number of neutrophils, especially activated neutrophils are normally found in the blood stream during the beginning (acute) phase of inflammation, particularly as a result of bacterial infection, environmental exposure and some cancers.

CD64 (Cluster of Differentiation 64) is a type of integral membrane glycoprotein known as an Fc receptor that binds monomeric IgG-type antibodies with high affinity. Neutrophil CD64 expression quantification provides improved diagnostic detection of infection/sepsis compared with the standard diagnostic tests used in current medical practice.

CD163 (Cluster of Differentiation 163) is a human protein encoded by the CD 163 gene. It has also been shown to mark cells of monocyte/macrophage lineage.

Typically, the total sample volumes are in the range of 10 to 1000 μL, 100 to 900 μL, 200 to 800 μL, 300 to 700 μL, 400 to 600 μL, or 420 to 500 μL.

According to some embodiments, the volume of the treatment composition chambers 106, 108, 110 (also called blisters) is from about 1 μL to 1000 μL. According to other embodiments, the volume of the specimen is from about 10 μL to 200 μL. According to other embodiments, the volume of the specimen is about 1, 10, 20, 30, 40, 50, 60, 70, 80, 90, 100, 120, 140, 160, 180, 200, 250, 300, 350, 400, 450, or 500 μL.

According to some embodiments, the volume of the treatment compositions 120, 122, 124 is at most about 500 μL. According to other embodiments, the volume of the specimen is at most about 200 μL. According to other embodiments, the volume of the specimen at most about 500, 450, 400, 350, 300, 250, 200, 180, 160, 140, 120, 100, 90, 80, 70, 60, 50,40, 30,20, 10, or 1 μL.

According to some embodiments, the volume of a reactant is at least about 1 μL. According to other embodiments, the volume of the specimen is from about 10 μL. According to other embodiments, the volume of the specimen is at least about 1, 10, 20, 30, 40, 50, 60, 70, 80, 90, 100, 120, 140, 160, 180, 200, 250, 300, 350, 400, 450, or 500 μL.

The time required to complete an assay using system 101 of the present invention varies depending on a number of factors. In some embodiments, the time required to complete an assay is from about 0.5 to 100 minutes. In other embodiments, the time required to complete an assay is from about 1 to 20 minutes. In still other embodiments, the time required to complete an assay is from about 1 to 10 minutes. In some examples, the time required to complete an assay is from about 1, 2, 3, 4, 5, 6, 7, 8, 9, 10, 15, 20, 25, 30, 35,40, 50, 60, 80, or 100 minutes.

EXAMPLE

Application No. 1 - CD64 Infection & Sepsis

A cartridge 110 (Fig. 1A) is prepared for receiving a blood sample. The cartridge comprises a number of treatment composition chambers 106, 108, 110, adapted to respectively house a corresponding number of treatment compositions 120, 122, 124. These compositions are described in further detail in US 8,116,984 and in Davis, BH et al., (2006)). In brief, Reagent A comprises a mixture of murine monoclonal antibodies (contains buffered saline), Reagent B - 10X Concentrated Trillium Lyse solution (contains ammonium chloride), Reagent C - suspension of 5.2 pm polystyrene beads labeled with Starfire Red and fluorescein isothiocyanate (FITC), (contains < 0.1% sodium azide and 0.01% Tween 20).

Table 3 Time sequences of steps in the methodology of the present invention for detecting sepsis using CD64 and CD163 antibodies.

| LeukoDx device- present invention | | | | |
|---|---|---|---|---|
| Step | Description | Volume (uL) | Duration (min) | comments |
| 1 | Mixing blood and antibodies | Blood- 10 Abs- 50 | 4 | |
| 2 | Adding RBC lysis buffer | 250 | 3 | Might require heating the buffer to 37C |
| 3 | Incubating, Vortexing | | 3 | |
| 4 | Adding | 2 | Less than 1 | |
| | | | | |
| | normalization beads | | | |
| 5 | Reading | | Less than 1 | |
| | Total | 312 | 10 | |

In the case of sepsis, by "normalization" is meant taking the ratio of the median of the target population fluorescence emission to the median of the reference bead population fluorescence emission.

According to some embodiments, the readout may comprise an optoelectronics core, which enables identification and detection of fluorescent signals.

The CCD in the core, used for focusing, can also be used to read chemiluminescent signals. The readout to user may also indicate where the result falls relative to reference ranges.

**REFERENCES**

Assicot, Marcel, et al. "High serum procalcitonin concentrations in patients with sepsis and infection." The Lancet 341.8844 (1993): 515-518.

Aulesa, C., et al. "Validation of the Coulter LH 750 in a hospital reference laboratory." Laboratory Hematology 9.1 (2003): 15-28.

Hawkins, Robert C. "Laboratory turnaround time." The Clinical Biochemist Reviews 28.4 (2007): 179.

Ault, Kenneth A. "Flow cytometric measurement of platelet function and reticulated platelets." Annals of the New York Academy of Sciences 677.1 (1993): 293- 308.

Blajchman, Morris A., et al. "Bacterial detection of platelets: current problems and possible resolutions." Transfusion medicine reviews 19.4 (2005): 259-272.

Bodensteiner, David C. "A flow cytometric technique to accurately measure post-filtration white blood cell counts." Transfusion 29.7 (1989): 651-653.

Cheson, Bruce D., et al. "National Cancer Institute-sponsored Working Group guidelines for chronic lymphocytic leukemia: revised guidelines for diagnosis and treatment." Blood 87.12 (1996): 4990-4997.

Christ-Crain, Mirjam, et al. "Effect of procalcitonin-guided treatment on antibiotic use and outcome in lower respiratory tract infections: cluster-randomised, single-blinded intervention trial." Lancet 363.9409 (2004): 600-607.

Cristofanilli, Massimo, et al. "Circulating tumor cells, disease progression, and survival in metastatic breast cancer." New England Journal of Medicine 351.8 (2004): 781-791.

Davis, Bruce H., et al. "Neutrophil CD64 is an improved indicator of infection or sepsis in emergency department patients." Archives of pathology & laboratory medicine 130.5 (2006): 654-661.

Dieye, Tandakha Ndiaye, et al. "Absolute CD4 T-cell counting in resource-poor settings: direct volumetric measurements versus bead-based clinical flow cytometry instruments." JAIDS Journal of Acquired Immune Deficiency Syndromes 39.1 (2005): 32-37.

Divers, S. G., et al. "Quantitation of CD62, soluble CD62, and lysosomeassociated membrane proteins 1 and 2 for evaluation of the quality of stored platelet concentrates." Transfusion 35.4 (2003): 292-297.

Drexler, Hans G., et al. "Diagnostic value of immunological leukemia phenotyping." Acta haematologica 76.1 (1986): 1-8.

Dziegiel, Morten Hanefeld, Leif Kofoed Nielsen, and Adela Berkowicz. "Detecting fetomatemal hemorrhage by flow cytometry." Current opinion in hematology 13.6 (2006): 490.

Fischer, Johannes C., et al. "Reducing costs in flow cytometric counting of residual white blood cells in blood products: utilization of a single platform bead free flowrate calibration method." Transfusion 51.7 (2011): 1431-1438.

Graff, Jochen, et al. "Close relationship between the platelet activation marker CD62 and the granular release of platelet-derived growth factor." Journal of Pharmacology and Experimental Therapeutics 300.3 (2002): 952-957.

Guerti, K., et al. "Performance evaluation of the PENTRA 60C+ automated hematology analyzer and comparison with the AD VIA 2120." International journal of laboratory hematology 31.2 (2009): 132-141.

Hershman, M. J., et al. "Monocyte HLA-DR antigen expression characterizes clinical outcome in the trauma patient." British Journal of Surgery 77.2 (2005): 204-207.

Hilfrich, Ralf, and Jalil Hariri. "Prognostic relevance of human papillomavirus L1 capsid protein detection within mild and moderate dysplastic lesions of the cervix uteri in combination with pl6 biomarker." Analytical and Quantitative Cytology and Histology 30.2 (2008): 78-82.

Hillier, Sharon L., et al. "A case-control study of chorioamnionic infection and histologic chorioamnionitis in prematurity." New England Journal of Medicine 319.15 (1988): 972-978.

Hoffmann, Johannes JML. "Neutrophil CD64 as a sepsis biomarker." Biochemia Medica 21.3 (2011): 282-290.

Kibe, Savitri, Kate Adams, and Gavin Barlow. "Diagnostic and prognostic biomarkers of sepsis in critical care." Journal of Antimicrobial Chemotherapy 66.suppl 2 (2011): ii33-ii40.

LaRosa, Steven P., and Steven M. Opal. "Biomarkers: the future." Critical care clinics 27.2 (2011): 407.

Liu, N. I. N. G., A. H. Wu, and Shan S. Wong. "Improved quantitative Apt test for detecting fetal hemoglobin in bloody stools of newborns." Clinical chemistry 39.11 (1993): 2326-2329.

Lotan, Yair, et al. "Bladder cancer screening in a high risk asymptomatic population using a point of care urine based protein tumor marker." The Journal of urology 182.1 (2009): 52-58.
Masse, M., et al. "Validation of a simple method to count very low white cell concentrations in filtered red cells or platelets." Transfusion 32.6 (2003): 565-571.
Matic, Goran B., et al. "Whole blood analysis of reticulated platelets: improvements of detection and assay stability." Cytometry 34.5 (1998): 229-234.
McDonald, C. P., et al. "Use of a solid-phase fluorescent cytometric technique for the detection of bacteria in platelet concentrates." Transfusion Medicine 15.3 (2005): 175-183.
Michelson, Alan D. "Flow cytometry: a clinical test of platelet function." Open Access Articles (1996): 290.
Miller, E.M.; Freire, S.L.S.; Wheeler, A.R. "Proteomics in Microfluidic Devices" In Encyclopedia of Micro- and Nanofluidics; Li, D. Q., Ed.; Springer: Heidelberg, Germany, 2008; Vol. 3, pp 1749-1758.."
Moro, Ricardo, et al. "A new broad-spectrum cancer marker." Vitro Diagnostic Technology (2005).
Perry, Sara E., et al. "Is low monocyte HLA-DR expression helpful to predict outcome in severe sepsis?." Intensive care medicine 29.8 (2003): 1245-1252.
Ramakumar, Sanjay, et al. "Comparison of screening methods in the detection of bladder cancer." The Journal of urology 161.2 (1999): 388-394.
Rawstron, Andy C., et al. "Quantitation of minimal disease levels in chronic lymphocytic leukemia using a sensitive flow cytometric assay improves the prediction of outcome and can be used to optimize therapy." Blood 98.1 (2001): 29-35.
Rodriguez, William R., et al. "A microchip CD4 counting method for HIV monitoring in resource-poor settings." PLoS medicine 2.7 (2005): el82.
Rylatt, D. B., et al. "An immunoassay for human D dimer using monoclonal antibodies." Thrombosis research 31.6 (1983): 767-778.
Sacks, David B., et al. "Guidelines and recommendations for laboratory analysis in the diagnosis and management of diabetes mellitus." Clinical Chemistry 48.3 (2002): 436-472.
Segal, H. C., et al. "Accuracy of platelet counting haematology analysers in severe thrombocytopenia and potential impact on platelet transfusion." British Journal of Haematology 128.4 (2005): 520-525.
Stein, Paul D., et al. "D-dimer for the exclusion of acute venous thrombosis and pulmonary embolism: a systematic review." Annals of internal medicine 140.8 (2004): 589.
Sutherland, D. Robert, et al. "The ISHAGE guidelines for CD34+ cell determination by flow cytometry." Journal ofhematotherapy 5.3 (1996): 213-226.
Wang, Chao, et al. "Reticulated platelets predict platelet count recovery following chemotherapy." Transfusion 42.3 (2002): 368-374.

## Claims

**1.** A method for determining a biological condition in a subject, the method comprising:

a) providing a self-contained, valveless stationary cartridge (110) the cartridge comprising:

a sample chamber (1404);
a first blister comprising a first treatment composition (1420);
a second blister comprising a second composition (1422);
a third blister comprising a third composition (1424);
a first mixing chamber (1412);
a second mixing chamber (1411);
first mixing bellows (1415);
second reading bellows (1417);
a tortuous conduit (1413) connecting the first and second mixing chambers;
a reading cuvette (1430) connected to a reading region (1450) via a reading channel (1452);

wherein:

the sample chamber, first blister and second blister are each connected to the first mixing chamber;
the third blister (1424) is connected via a channel to the second mixing chamber (1411);
the first bellows is connected to the second mixing chamber;
the second bellows (1417) is connected to the first mixing chamber (1412) *via* the reading cuvette (1430);
the reading cuvette is connected to the first mixing chamber;

wherein:

activation of said first mixing bellows selectively moves fluid through fluidic channels within said cartridge, allowing a reaction of said sample with said composition(s);
activation of said second bellows selectively moves fluid through fluidic channels within said cartridge toward the reading region, allowing optical detection of said reaction in said fluid;

b) incubating a sample from said subject, in said cartridge (110) , wherein at least one of said compositions comprises a reporter functionality, said reporter functionality being adapted to report a reaction of said treatment composition with said sample to report an assay of said reaction;
c) transferring said sample to said reading region (1450) through at least one of said fluidic channels by activating said second bellows;
d) optically detecting said reaction in a moving fluid, the detecting comprising:

1. providing an excitation beam (6032) to said reading region (1450);
2. measuring a forward scatter measurement (1606) from particles in said reading channel (1452);
3. passing a returned beam from said sample via a high-pass filter (449) and a concave grating (453);
4. providing an optical detector comprising detection elements for independent detection of different wavelengths;
5. detecting using said detector, a plurality, for example eight, spectrally distinct signals (601) associated with said at least one reporter functionality produced by said concave grating in at least one of photomultiplier array, PMT, and a light- receiving reader unit and
scatter channels data;

e) processing data outputted in said detecting step to determine said assay; and
f) receiving an indication responsive to said assay,
wherein said assay is a flow cytometric assay, thereby providing an indication of the biological condition in said subject, wherein the biological condition is selected from blood diseases, leukemia, thrombocytopenia, immune system disorders, local infections, urinary tract disorders, autoimmune diseases and sepsis;
wherein said cartridge is sealed after receiving said sample.

**2.** A method according to claim 1, wherein said detecting step comprises detecting a signal associated with at least one reporter functionality, said at least one reporter functionality adapted to report a reaction of said at least one composition with said sample, wherein said at least one composition comprises:

i. a cell surface marker;
ii. a cell stain;
iii. a reagent bound to a solid support;
iv. a chemical indicator; and
v. a biological cell indicator;

wherein said reagent bound to said solid support is selected from the group consisting of an immobilized enzyme, an immobilized substrate, a plasma protein bead, an antibody bead and an antigen bead.

**3.** A method according to claim 2, wherein said biological cell indicator is selected from the group consisting of a cell cycle stage indicator, a cell proliferation indicator, a cytokine indicator, a metabolic indicator and an apoptosis indicator.

**4.** A method according to claim 2, wherein said at least one composition comprises at least two compositions.

**5.** A method according to claim 4, wherein said at least two compositions comprise at least one of:

a. a cell surface marker and a cell element stain;
b. a cell surface marker and a plasma protein bead assay;
c. a cell surface marker and a solution change marker;
d. a cell element stain and a plasma protein bead assay; and
e. a cell element stain and a solution change marker.

**6.** A method according to claim 1, wherein said at least one composition comprises:

a) a detection composition comprising at least one of;

i. at least one target antibody;
ii. at least one positive control identifying antibody; and
iii. at least one negative control identifying detection moiety

or characteristic; and
b) at least one reference composition comprising at least one of;

i. a target signal reference composition; and
ii. a reference identifier composition.

**7.** A method according to claim 1, wherein said at least one composition comprises:

a) an antibody composition comprising at least one of;

i. at least one target antibody;
ii. at least one positive control identifying antibody; and
iii. at least one negative control identifying antibody or characteristic; and

b) at least one reference composition comprising at least one of;

iv. a target signal reference composition; and
v. a reference identifier composition.

**8.** A method according to claim 1, wherein said sample comprises at least one of;

i. a bodily specimen comprising a target moiety;
ii. a positive control moiety; and
iii. a negative control moiety.

**9.** A method according to claim 1, wherein the at least one composition further comprises at least one conditioning moiety comprising;

a. at least one lysis reagent; and
b. at least one diluent.

**10.** A method according to claim 1, wherein said providing step further comprises passing said excitation beam via a dichroic mirror through an objective onto a reading channel in said cartridge.

**11.** A method according to claim 10, further comprising, collecting particle fluorescent emission through said objective, further passing said particle fluorescent emission through said dichroic mirror and reflecting said fluorescent emission from a beamsplitter into a detection path.

**Patentansprüche**

**1.** Verfahren zum Bestimmen eines biologischen Zustands bei einem Subjekt, wobei das Verfahren wie folgt umfasst:

a) Bereitstellen einer in sich geschlossenen, ventillosen ortsfesten Patrone (110), wobei die Patrone wie folgt umfasst:

eine Probenkammer (1404);
eine erste Blase, die eine erste Behandlungszusammensetzung (1420) umfasst;
eine zweite Blase, die eine zweite Behandlungszusammensetzung (1422) umfasst;
eine dritte Blase, die eine dritte Behandlungszusammensetzung (1424) umfasst;

eine erste Mischkammer (1412);
eine zweite Mischkammer (1411);
einen ersten Mischbalg (1415);
einen zweiten Ablesebalg (1417);
eine gewundene Leitung (1413), welche die erste und die zweite Mischkammer miteinander verbindet;
eine Ableseküvette (1430), die über einen Ablesekanal (1452) mit einer Ableseregion (1450) verbunden ist, wobei gilt wie folgt:

die Probenkammer, die erste Blase und die zweite Blase sind jeweils mit der ersten Mischkammer verbunden;
die dritte Blase (1424) ist über einen Kanal mit der zweiten Mischkammer (1411) verbunden;
der erste Balg ist mit der zweiten Mischkammer verbunden;
der zweite Balg (1417) ist über die Ableseküvette (1430) mit der ersten Mischkammer (1412) verbunden;
die Ableseküvette ist mit der ersten Mischkammer verbunden;
wobei gilt:

durch Aktivierung des ersten Mischbalgs wird Fluid selektiv durch Fluidkanäle innerhalb der Patrone bewegt, wodurch eine Reaktion der Probe mit der (den) Zusammensetzung(en) ermöglicht wird;
durch Aktivierung des zweiten Balgs wird Fluid selektiv durch Fluidkanäle innerhalb der Patrone auf die Ableseregion hinbewegt, wodurch eine optische Erfassung der Reaktion in dem Fluid ermöglicht wird;

b) Inkubieren einer dem Subjekt entnommenen Probe in der Patrone (110), wobei mindestens eine der Zusammensetzungen eine Reporterfunktionalität umfasst, wobei die Reporterfunktionalität dazu adaptiert ist, eine Reaktion der Behandlungszusammensetzung auf die Probe zu melden, um ein Assay der Reaktion zu melden;
c) Transferieren der Probe durch mindestens einen der Fluidkanäle zur Ableseregion (1450), indem der zweite Balg aktiviert wird;
d) optisches Erfassen der Reaktion in einem sich in Bewegung befindlichen Fluids, wobei das Erfassen wie folgt umfasst:

1. Bereitstellen eines auf die Ableseregion (1450) gerichteten Erregungsstrahls (6032);
2. Messen einer Vorwärtslichtstreuung (1606) der Partikel in dem Ablesekanal (1452);
3. Passieren eines von der Probe zurückgeworfenen Strahls durch einen Hochpassfilter (449) und ein konkaves Gitter (453);
4. Bereitstellen eines optischen Detektors, der Erfassungselemente zur unabhängigen Erfassung verschiedener Wellenlängen umfasst;
5. Unter Verwendung des Detektors Erfassen einer Vielzahl von beispielsweise acht spektral verschiedenen Signalen (601), die mit der mindestens einen Reporterfunktionalität assoziiert sind, die durch das konkave Gitter in mindestens einer Photomultiplier-Anordnung (PMT) produziert werden, und eine lichtempfangende Ableseeinheit sowie Streukanaldaten;

e) Verarbeiten von Daten, die in dem Erfassungsschritt ausgegeben werden, um das Assay zu bestimmen; und
f) Empfangen einer Anzeige, die auf das Assay anspricht,

wobei das Assay ein durchflusszytometrisches Assay ist, wodurch es eine Anzeige des biologischen Zustands bei dem Subjekt bereitstellt, wobei der biologische Zustand unter Bluterkrankungen wie Leukämie, Thrombozytopenie, Störungen des Immunsystems, lokalen Infektionen, Störungen der Harnwege, Autoimmunerkrankungen und Sepsis ausgewählt ist;
wobei die Patrone nach dem Empfangen des Probe versiegelt wird.

**2.** Verfahren nach Anspruch 1, wobei der Detektionsschritt ein Erfassen eines Signal umfasst, das mit mindestens einer Reporterfunktionalität assoziiert ist, wobei die mindestens eine Reporterfunktionalität dazu adaptiert ist, eine Reaktion mindestens einer Zusammensetzung mit der Probe zu melden, wobei die mindestens eine Zusammensetzung wie folgt umfasst:

i. einen Zell-Oberflächenmarker;
ii. ein Zell-Färbemittel;
iii. einen Reagenzstoff, der an einen festen Träger gebunden ist;

iv. einen chemischen Anzeiger; und
v. einen biologischen Zellanzeiger;

wobei das Reagenzmittel, das an den festen Träger gebunden ist, aus der Gruppe bestehend aus einem immobilisierten Enzym, einem immobilisierten Substrat, einem Plasmaprotein-Bead, einem Antikörper-Bead und einem Antigen-Bead ausgewählt ist.

**3.** Verfahren nach Anspruch 2, wobei der biologische Zellanzeiger aus der Gruppe bestehend aus einem Zellzyklusstadiumsanzeiger, einem Proliferationsanzeiger, einem Zytokinanzeiger, einem Stoffwechselanzeiger und einem Apoptoseanzeiger ausgewählt ist.

**4.** Verfahren nach Anspruch 2, wobei die mindestens eine Zusammensetzung mindestens zwei Zusammensetzungen umfasst.

**5.** Verfahren nach Anspruch 4, wobei die mindestens zwei Zusammensetzungen mindestens eines hiervon umfassen:

a. einen Zell-Oberflächenmarker und ein Zellelement-Färbemittel;
b. einen Zell-Oberflächenmarker und ein Plasmaprotein-Bead-Assay;
c. einen Zell-Oberflächenmarker und einen Lösungs-Änderungsmarker;
d. ein Zellelement-Färbemittel und ein Plasmaprotein-Bead-Assay; und
e. ein Zellelement-Färbemittel und einen Lösungs-Änderungsmarker.

**6.** Verfahren nach Anspruch 1, wobei mindestens eine Zusammensetzung wie folgt umfasst:

a) eine Detektionszusammensetzung, die mindestens eines hiervon umfasst:

i. mindestens einen Zielantikörper;
ii. mindestens eine Positivkontrolle, die Antikörper identifiziert; und
iii. mindestens eine Negativkontrolle, die Detektionsrest oder Charakteristik identifiziert;

b) mindestens eine Referenzzusammensetzung, die mindestens eines hiervon umfasst:

i. eine Zielsignal-Referenzzusammensetzung; und
ii. eine Referenz-Identifizierungszusammensetzung.

**7.** Verfahren nach Anspruch 1, wobei die mindestens eine Zusammensetzung wie folgt umfasst:

a) eine Antikörperzusammensetzung, die mindestens eines hiervon umfasst;

i. mindestens einen Zielantikörper;
ii. mindestens eine Positivkontrolle, die einen Antikörper identifiziert; und
iii. mindestens eine Negativkontrolle, die Antikörper oder Charakteristik identifiziert; und

b) mindestens eine Referenzzusammensetzung, die mindestens eines hiervon umfasst;

iv. eine Zielsignal-Referenzzusammensetzung; und
v. eine Referenz-Identifizierungszusammensetzung.

**8.** Verfahren nach Anspruch 1, wobei die mindestens eine Zusammensetzung ferner mindestens eines hiervon wie folgt umfasst:

i. eine einem Körper entnommene Probe, die einen Zielrest umfasst;
ii. einen Positivkontrollrest; und
iii. einen Negativkontrollrest.

**9.** Verfahren nach Anspruch 1, wobei die mindestens eine Zusammensetzung ferner mindestens einen Zustandsrest umfasst, der wie folgt umfasst:

a. mindestens ein Lyse-Reaktionsmittel; und
b. mindestens ein Verdünnungsmittel.

**10.** Verfahren nach Anspruch 1, wobei der Bereitstellungsschritt ferner ein Passieren eines Erregungsstrahls über einen dichroischen Spiegel durch ein Objektiv auf einen Ablesekanal in der Patrone umfasst.

**11.** Verfahren nach Anspruch 10, ferner umfassend Sammeln von Fluoreszenzemissionen von Partikeln durch das Objektiv, ferner Passieren der Fluoreszensemissionen der Partikel durch den dichroischen Spiegel und Reflektieren der Fluoreszensemissionen von einem Strahlenteiler auf einen Detektionsweg.

## Revendications

**1.** Procédé de détermination d'un état biologique chez un sujet, le procédé consistant à :

a) fournir une cartouche stationnaire autonome sans valve (110), la cartouche comprenant :

une chambre d'échantillons (1404) ;
un premier blister comprenant une première composition de traitement (1420) ;
un deuxième blister comprenant une deuxième composition (1422) ;
un troisième blister comprenant une troisième composition (1424) ;
une première chambre de mélange (1412) ;
une deuxième chambre de mélange (1411) ;
un premier soufflet de mélange (1415) ;
un deuxième soufflet de lecture (1417) ;
un conduit tortueux (1413) reliant les première et deuxième chambres de mélange ;
une cuvette de lecture (1430) reliée à une région de lecture (1450) par un canal de lecture (1452) ;

dans lequel :

la chambre d'échantillons, le premier blister et le deuxième blister sont chacun reliés à la première chambre de mélange ;
le troisième blister (1424) est relié par un canal à la deuxième chambre de mélange (1411) ;
le premier soufflet est relié à la deuxième chambre de mélange ;
le deuxième soufflet (1417) est relié à la première chambre de mélange (1412) par la cuvette de lecture (1430) ;
la cuvette de lecture est reliée à la première chambre de mélange ;

dans lequel :

l'activation dudit premier soufflet de mélange déplace sélectivement le fluide à travers des canaux fluidiques à l'intérieur de ladite cartouche, permettant une réaction dudit échantillon avec ladite ou lesdites compositions ;
l'activation dudit deuxième soufflet déplace sélectivement le fluide à travers des canaux fluidiques à l'intérieur de ladite cartouche vers la région de lecture, permettant la détection optique de ladite réaction dans ledit fluide ;

b) incuber un échantillon provenant dudit sujet, dans ladite cartouche (110), dans laquelle au moins une desdites compositions comprend une fonctionnalité de rapporteur, ladite fonctionnalité de rapporteur étant adaptée pour rapporter une réaction de ladite composition de traitement avec ledit échantillon pour rapporter un dosage de ladite réaction ;
c) transférer ledit échantillon vers ladite région de lecture (1450) à travers au moins un desdits canaux fluidiques en activant ledit deuxième soufflet ;
d) détecter optiquement ladite réaction dans un fluide en mouvement, la détection consistant à :

i. fournir un faisceau d'excitation (6032) à ladite région de lecture (1450) ;
ii. effectuer une mesure de diffusion vers l'avant (1606) à partir de particules dans ledit canal de lecture (1452) ;

iii. faire passer un faisceau renvoyé dudit échantillon à travers un filtre passe-haut (449) et un réseau de diffraction concave (453) ;
iv. fournir un détecteur optique comprenant des éléments de détection pour une détection indépendante de différentes longueurs d'onde ;
v. détecter à l'aide dudit détecteur, une pluralité, par exemple huit signaux spectralement distincts (601) associés à ladite au moins une fonctionnalité de rapporteur produite par ledit réseau de diffraction concave dans au moins l'un parmi un réseau de photomultiplicateurs, PMT, et une unité de lecture réceptrice de lumière et des données de canaux de diffusion ;

e) traiter les données produites lors de ladite étape de détection pour déterminer ledit dosage ; et
f) recevoir une indication répondant audit dosage,

ledit dosage étant un dosage par cytométrie en flux, fournissant ainsi une indication de l'état biologique chez ledit sujet, l'état biologique étant choisi parmi les maladies du sang, la leucémie, la thrombocytopénie, les troubles du système immunitaire, les infections locales, les troubles des voies urinaires, les maladies auto-immunes et la septicémie ;
dans lequel ladite cartouche est scellée après réception dudit échantillon.

**2.** Procédé selon la revendication 1, dans lequel ladite étape de détection comprend la détection d'un signal associé à au moins une fonctionnalité de rapporteur, ladite au moins une fonctionnalité de rapporteur étant adaptée pour rapporter une réaction de ladite au moins une composition avec ledit échantillon, ladite au moins une composition comprenant :

i. un marqueur de surface cellulaire ;
ii. un colorant cellulaire ;
iii. un réactif lié à un support solide ;
iv. un indicateur chimique ; et
v. un indicateur cellulaire biologique ;

dans lequel ledit réactif lié audit support solide est choisi dans le groupe constitué par une enzyme immobilisée, un substrat immobilisé, une bille de protéine plasmatique, une bille d'anticorps et une bille d'antigène.

**3.** Procédé selon la revendication 2, dans lequel ledit indicateur cellulaire biologique est choisi dans le groupe constitué par un indicateur d'étape du cycle cellulaire, un indicateur de prolifération cellulaire, un indicateur de cytokine, un indicateur métabolique et un indicateur d'apoptose.

**4.** Procédé selon la revendication 2, dans lequel ladite au moins une composition comprend au moins deux compositions.

**5.** Procédé selon la revendication 4, dans lequel lesdites au moins deux compositions comprennent au moins l'un parmi :

a) un marqueur de surface cellulaire et un colorant d'élément cellulaire ;
b) un marqueur de surface cellulaire et un dosage de billes de protéines plasmatiques ;
c) un marqueur de surface cellulaire et un marqueur de changement de solution ;
d) un colorant d'élément cellulaire et un dosage de billes de protéines plasmatiques ; et
e) un colorant d'élément cellulaire et un marqueur de changement de solution.

**6.** Procédé selon la revendication 1, dans lequel ladite au moins une composition comprend :

a) une composition de détection comprenant au moins l'un parmi :

i. au moins un anticorps cible ;
ii. au moins un anticorps d'identification de témoin positif ; et
iii. au moins un témoin négatif identifiant un fragment ou une caractéristique de détection ; et

b) au moins une composition de référence comprenant au moins l'un parmi :

i. une composition de référence de signal cible ; et

ii. une composition d'identifiant de référence.

**7.** Procédé selon la revendication 1, dans lequel ladite au moins une composition comprend :

a) une composition d'anticorps comprenant au moins l'un parmi :

i. au moins un anticorps cible ;
ii. au moins un anticorps d'identification de témoin positif ; et
iii. au moins un témoin négatif identifiant un anticorps ou une caractéristique ; et

b) au moins une composition de référence comprenant au moins l'un parmi :

iv. une composition de référence de signal cible ; et
v. une composition d'identifiant de référence.

**8.** Procédé selon la revendication 1, dans lequel ledit échantillon comprend au moins l'un parmi :

a) un spécimen corporel comprenant un fragment cible ;
b) un fragment de témoin positif ; et
c) un fragment de témoin négatif.

**9.** Procédé selon la revendication 1, dans lequel l'au moins une composition comprend en outre au moins un fragment conditionneur comprenant :

a) au moins un réactif de lyse ; et
b) au moins un diluant.

**10.** Procédé selon la revendication 1, dans lequel ladite étape de fourniture consiste en outre à faire passer ledit faisceau d'excitation par le biais d'un miroir dichroïque à travers un objectif sur un canal de lecture dans ladite cartouche.

**11.** Procédé selon la revendication 10, consistant en outre à recueillir l'émission fluorescente de particules à travers ledit objectif, à faire passer en outre ladite émission fluorescente de particules à travers ledit miroir dichroïque et à réfléchir ladite émission fluorescente depuis un séparateur de faisceau dans un chemin de détection.

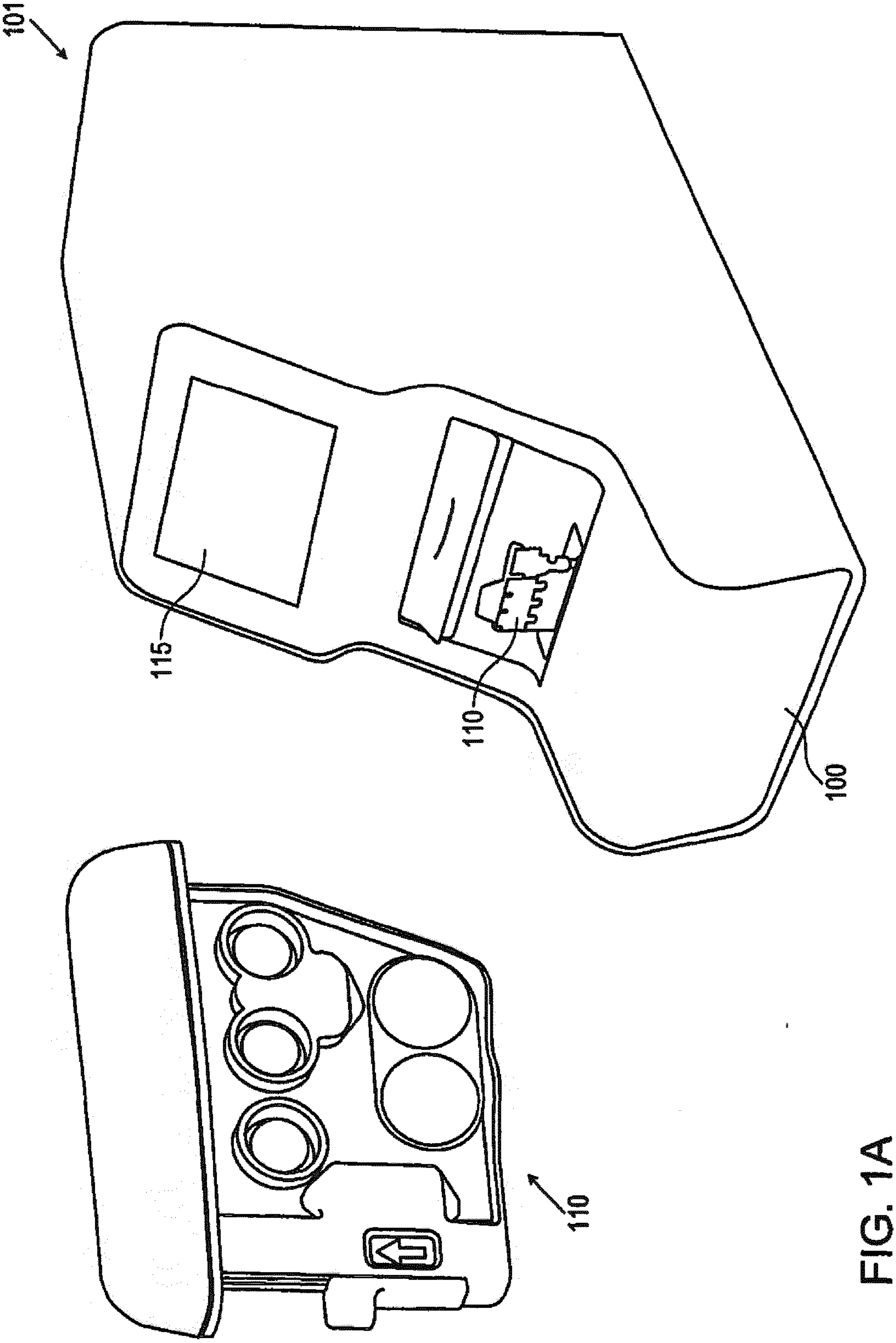


FIG. 1A

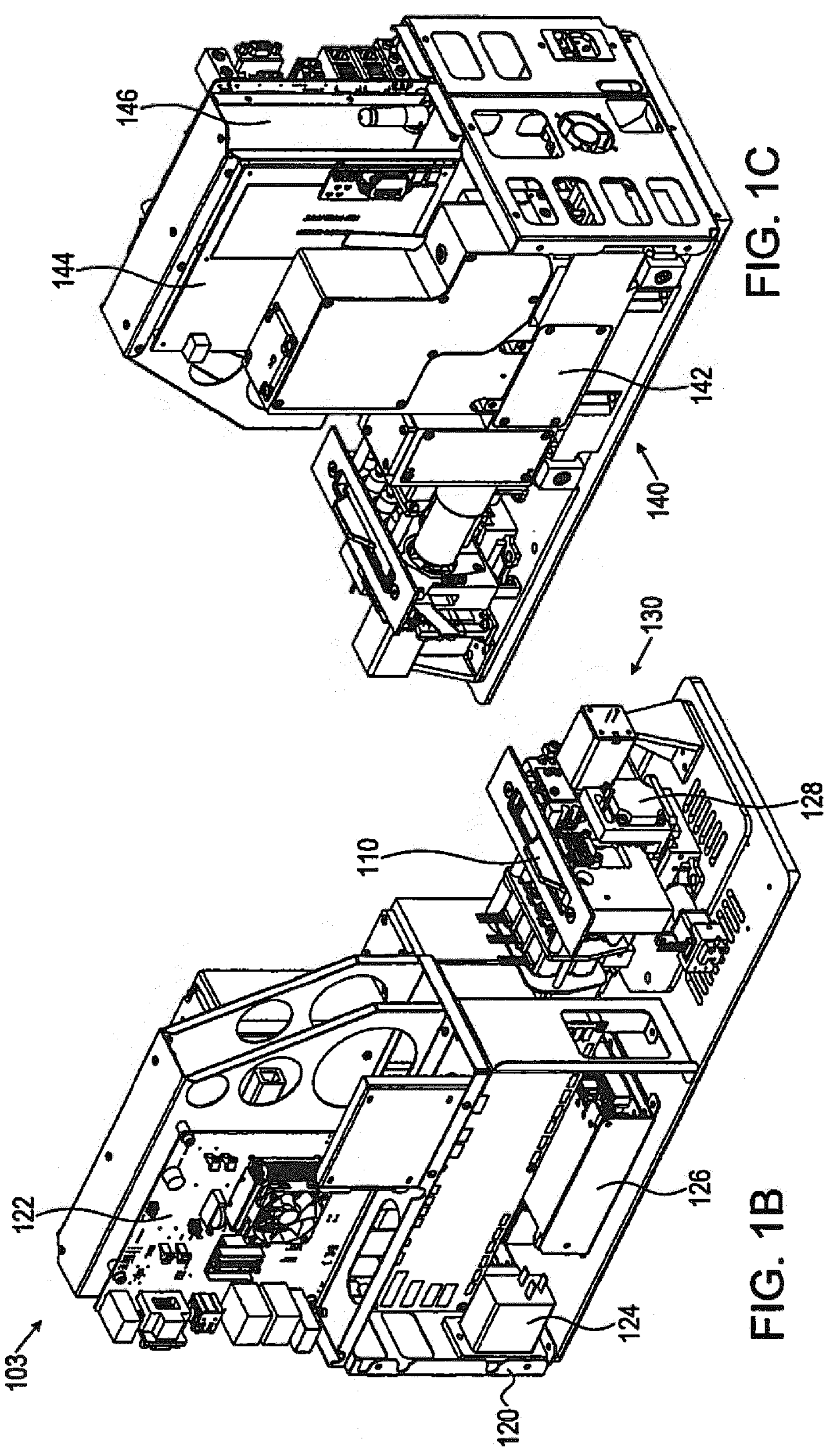


FIG. 1B

FIG. 1C

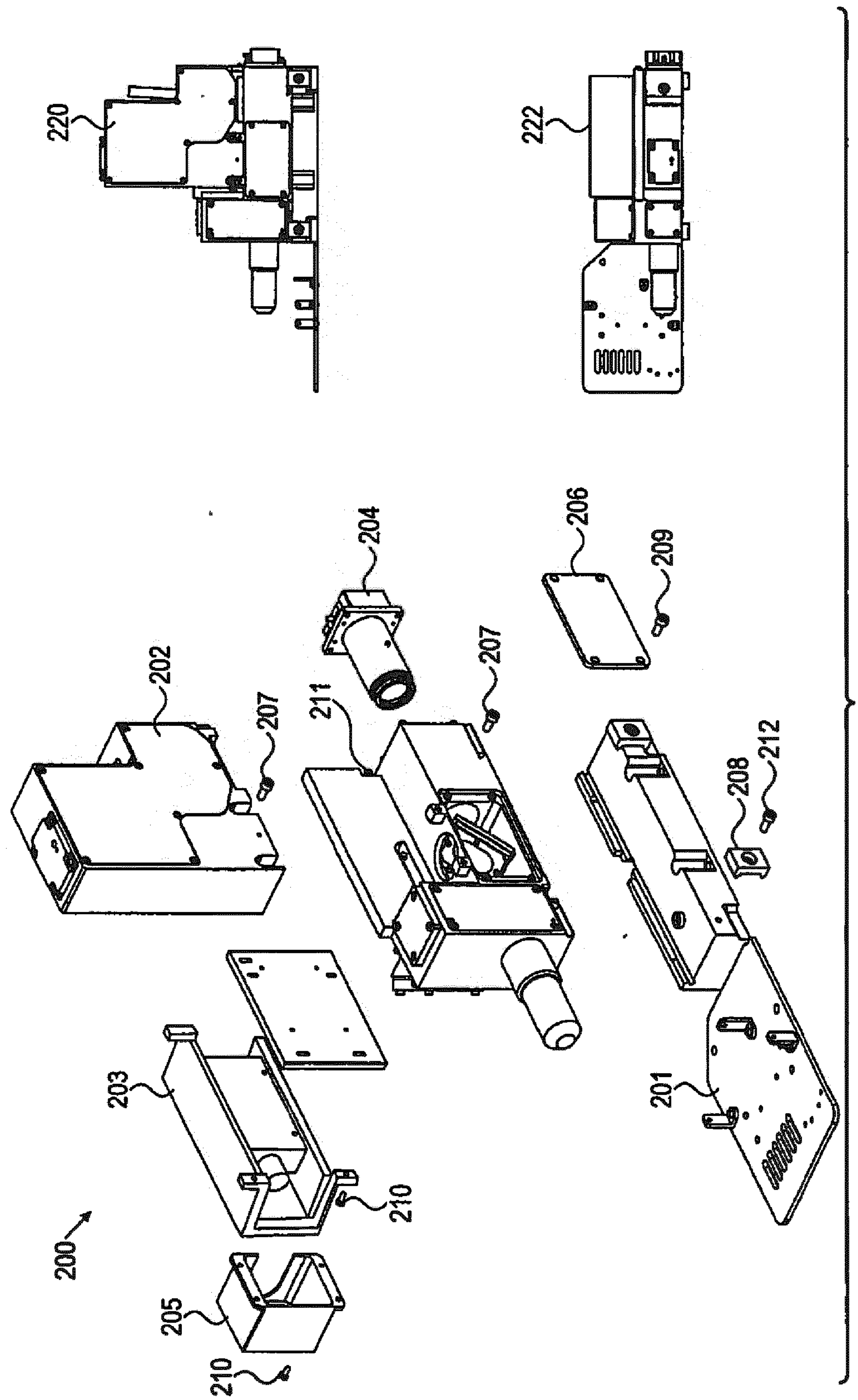
220
222
202
207
204
206
209
211
207
203
201
208
212
200
210
205
210

FIG. 2A

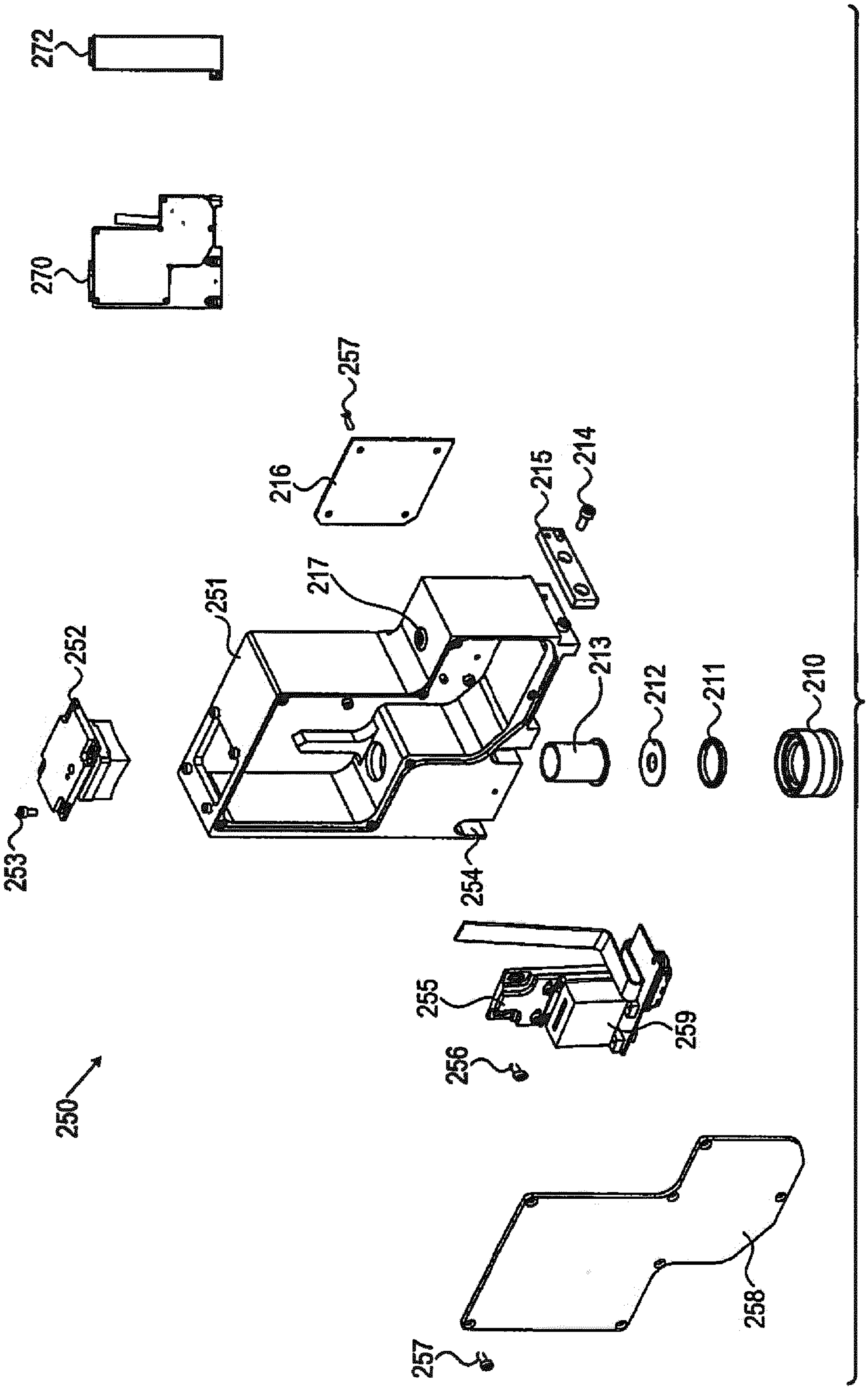
272
270
257
216
215
214
217
251
252
253
213
212
211
210
254
255
259
256
250
258
257

FIG. 2B

FIG. 3A

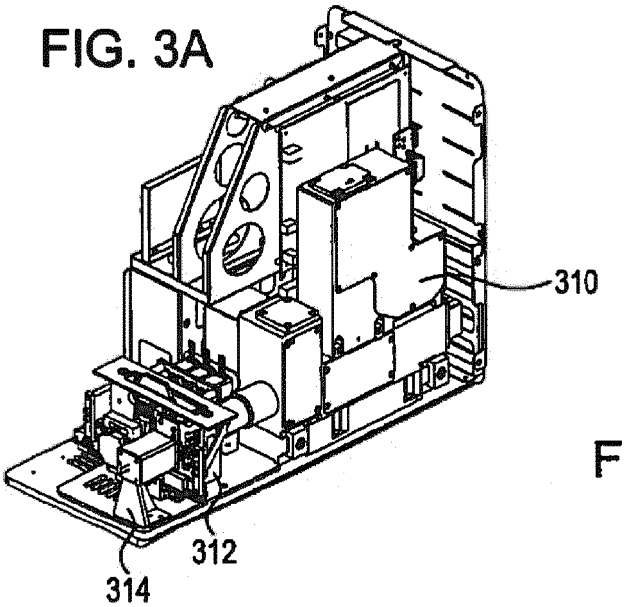


FIG. 3B

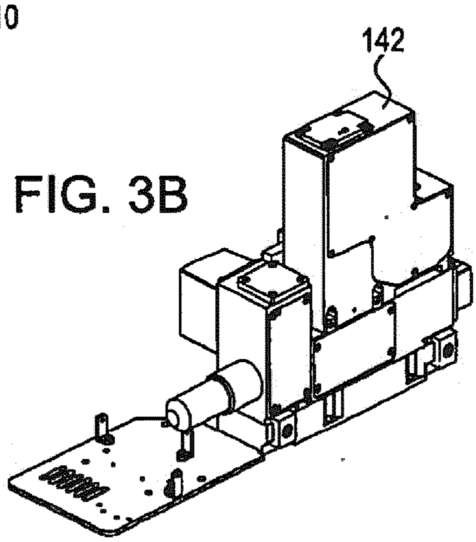


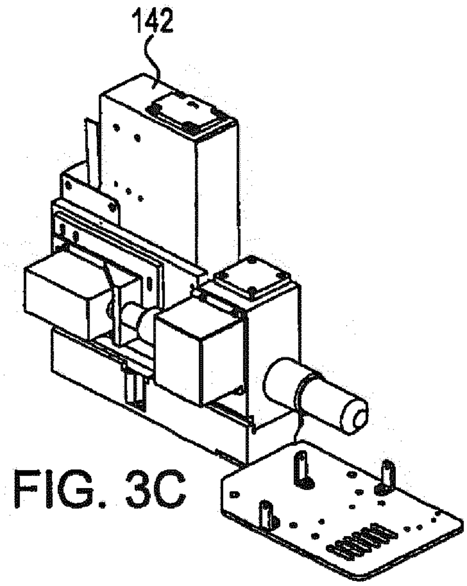


FIG. 3C

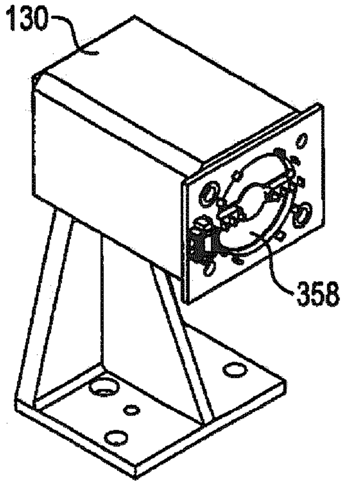


FIG. 3D

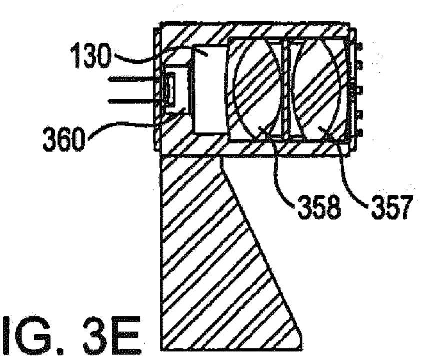


FIG. 3E

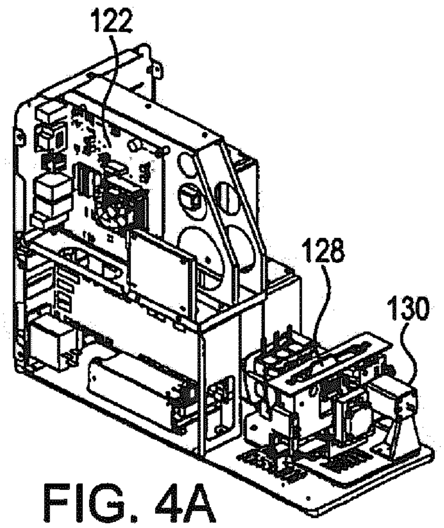


FIG. 4A

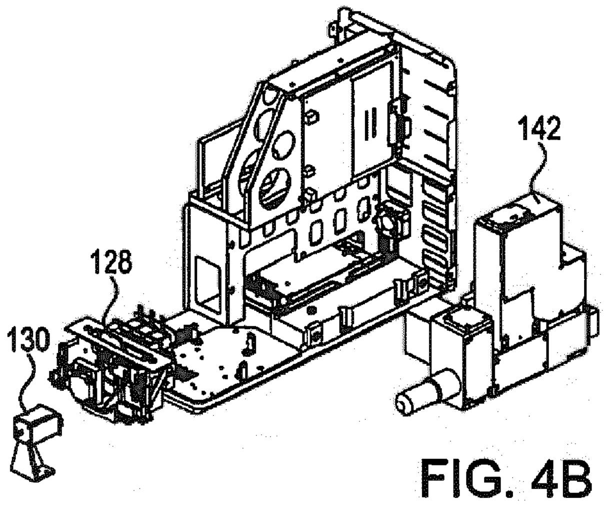


FIG. 4B

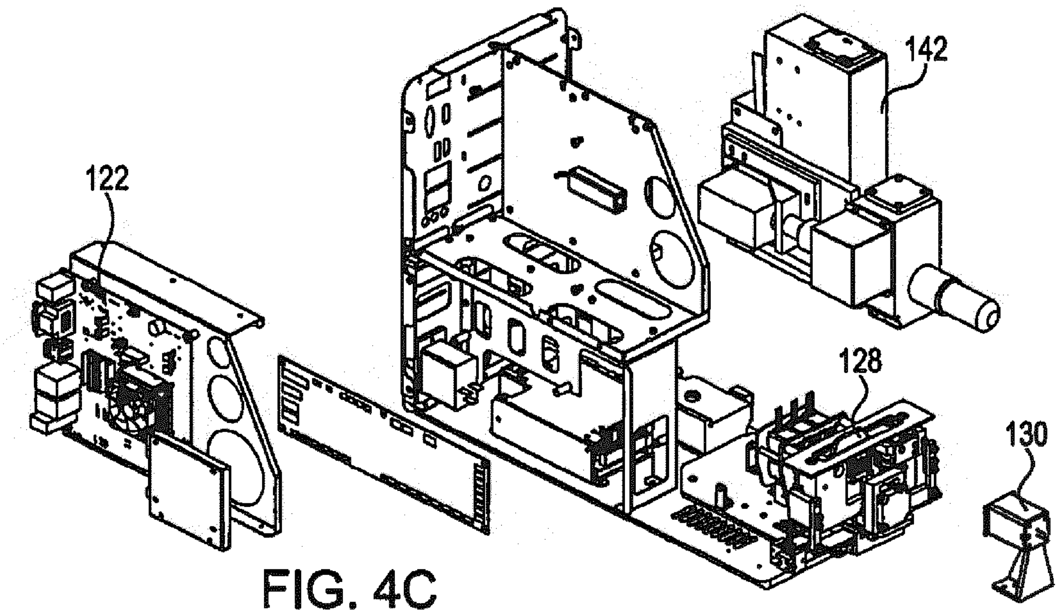


FIG. 4C

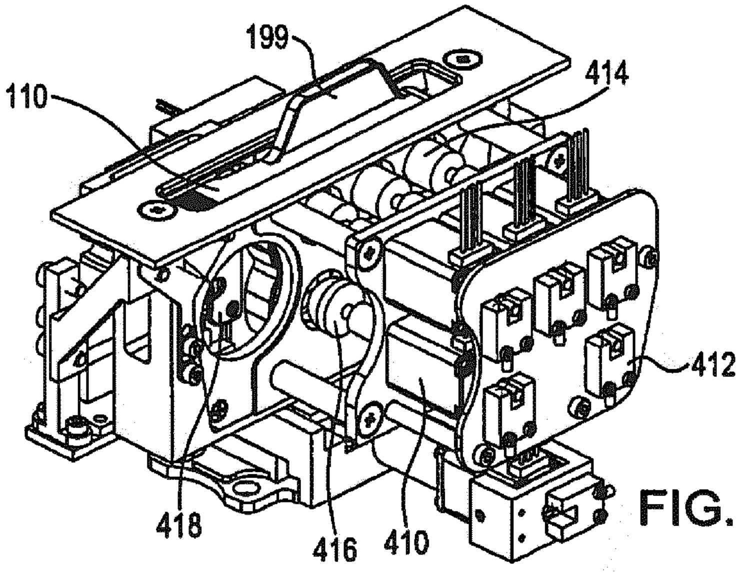


FIG. 4D

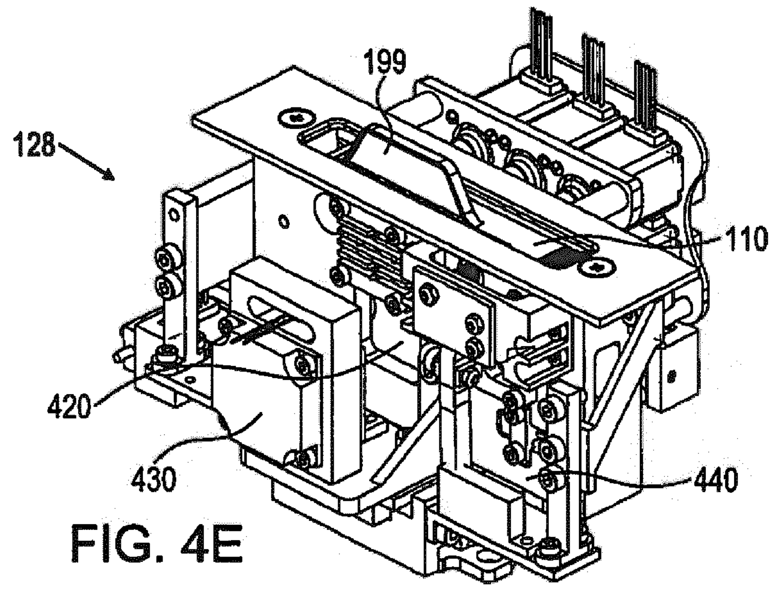


FIG. 4E

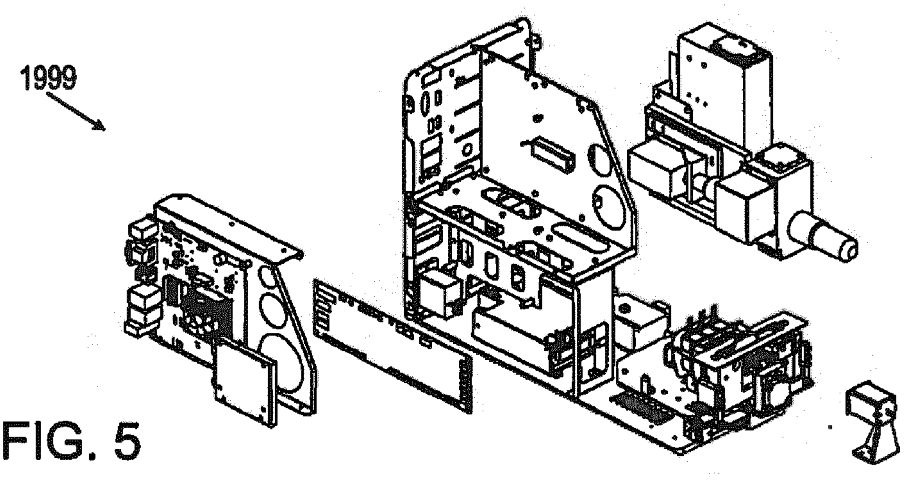


FIG. 5

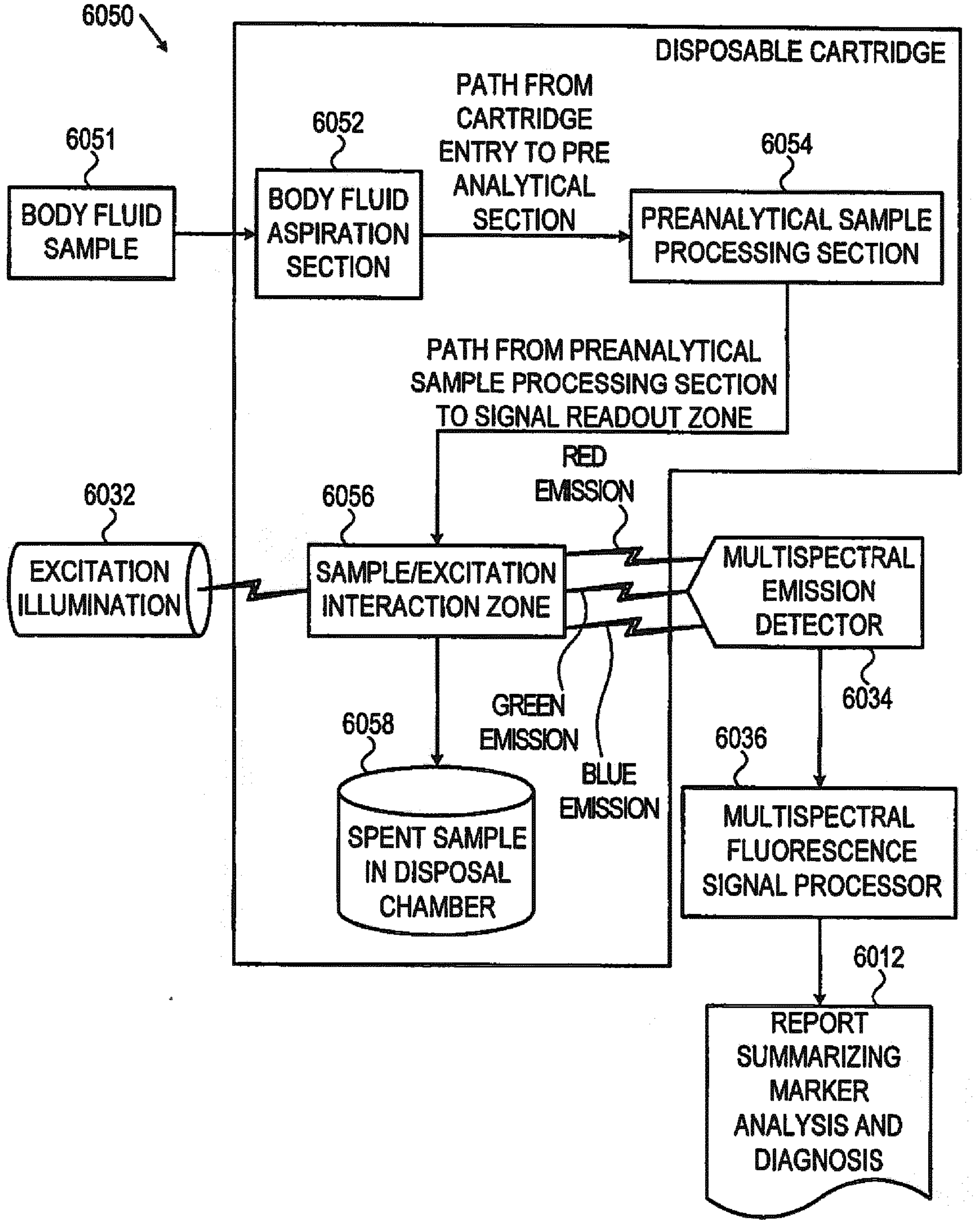
6050
DISPOSABLE CARTRIDGE
6051
BODY FLUID SAMPLE
6052
BODY FLUID ASPIRATION SECTION
PATH FROM CARTRIDGE ENTRY TO PRE ANALYTICAL SECTION
6054
PREANALYTICAL SAMPLE PROCESSING SECTION
PATH FROM PREANALYTICAL SAMPLE PROCESSING SECTION TO SIGNAL READOUT ZONE
RED EMISSION
6032
EXCITATION ILLUMINATION
6056
SAMPLE/EXCITATION INTERACTION ZONE
MULTISPECTRAL EMISSION DETECTOR
6034
GREEN EMISSION
BLUE EMISSION
6058
SPENT SAMPLE IN DISPOSAL CHAMBER
6036
MULTISPECTRAL FLUORESCENCE SIGNAL PROCESSOR
6012
REPORT SUMMARIZING MARKER ANALYSIS AND DIAGNOSIS

FIG. 6

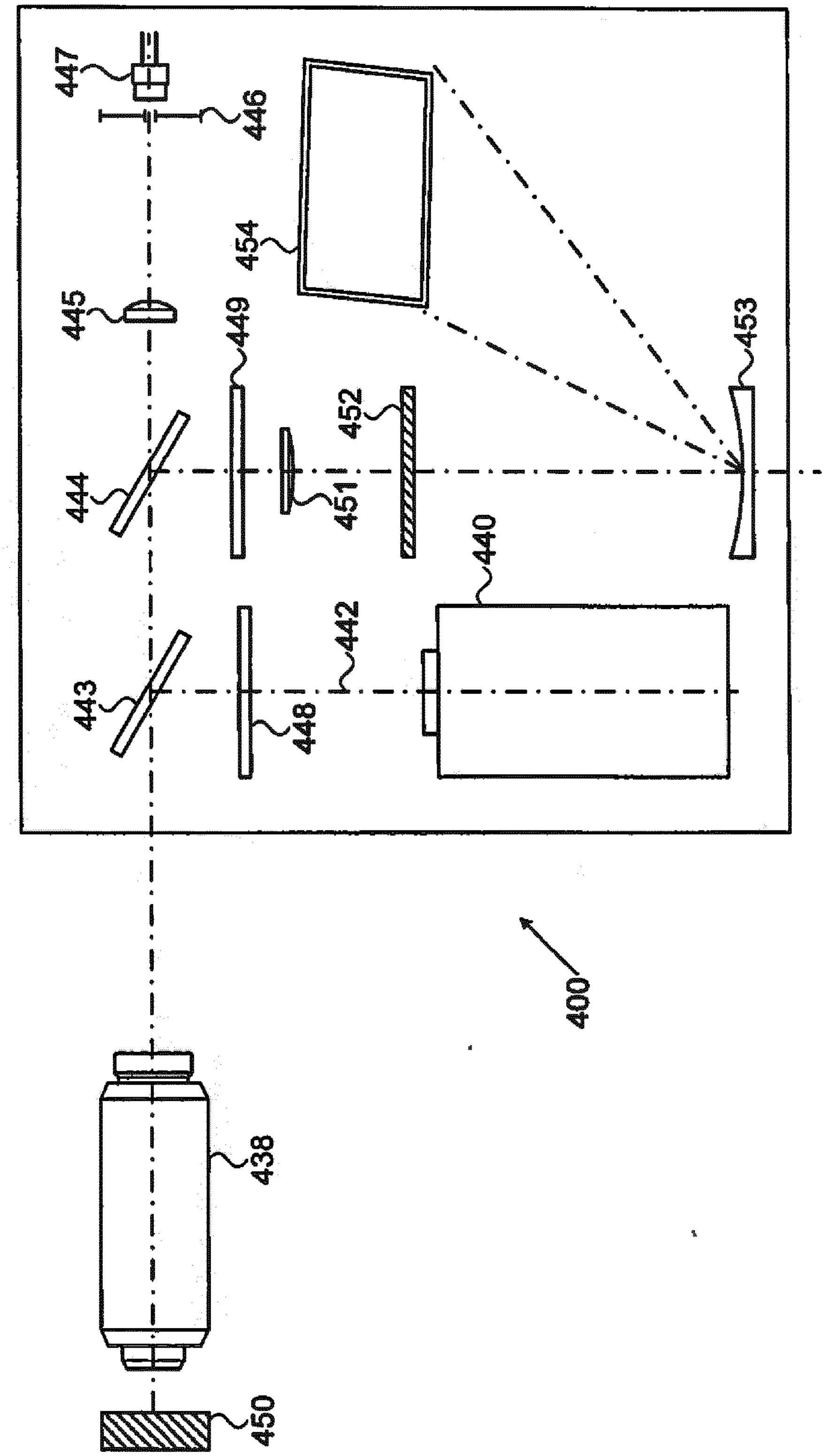


FIG. 7A

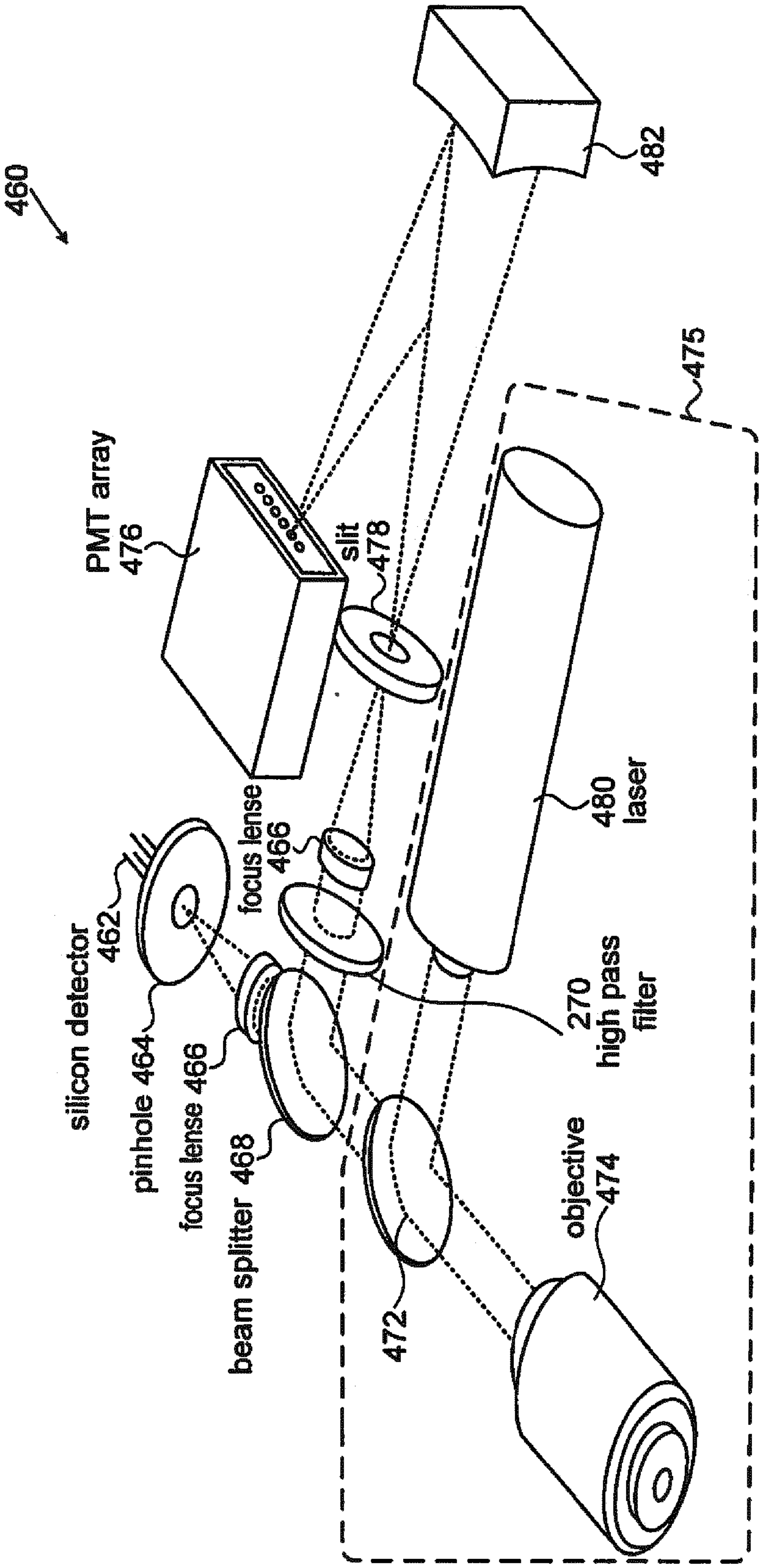
460
482
475
PMT array
476
slit
478
focus lense
466
silicon detector
462
pinhole 464
focus lense 466
beam splitter 468
480
laser
270
high pass
filter
objective
474
472

FIG. 7B

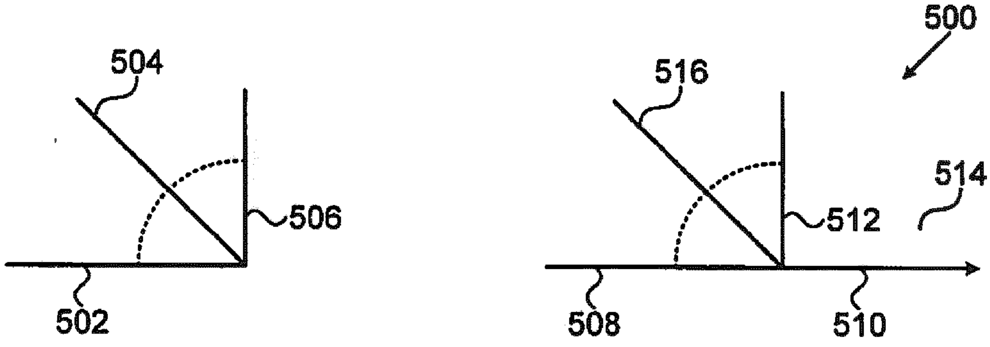


The setup for RGB lasters

FIG. 8A

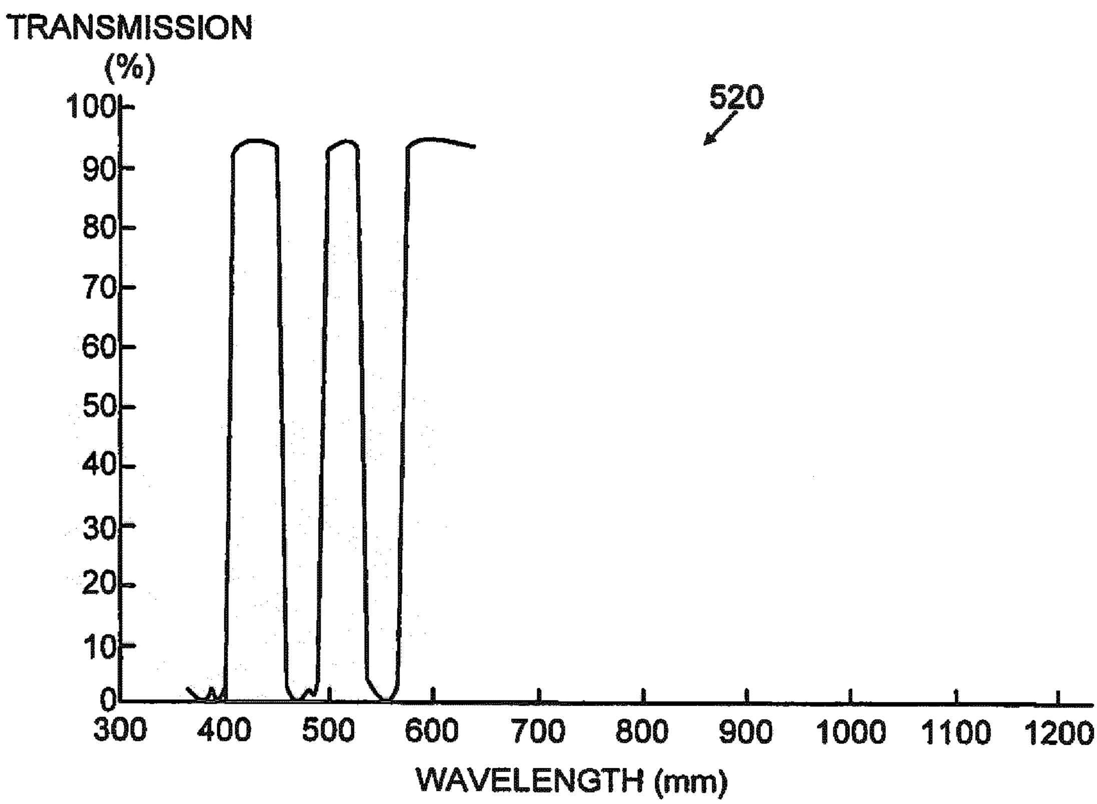


FIG. 8B

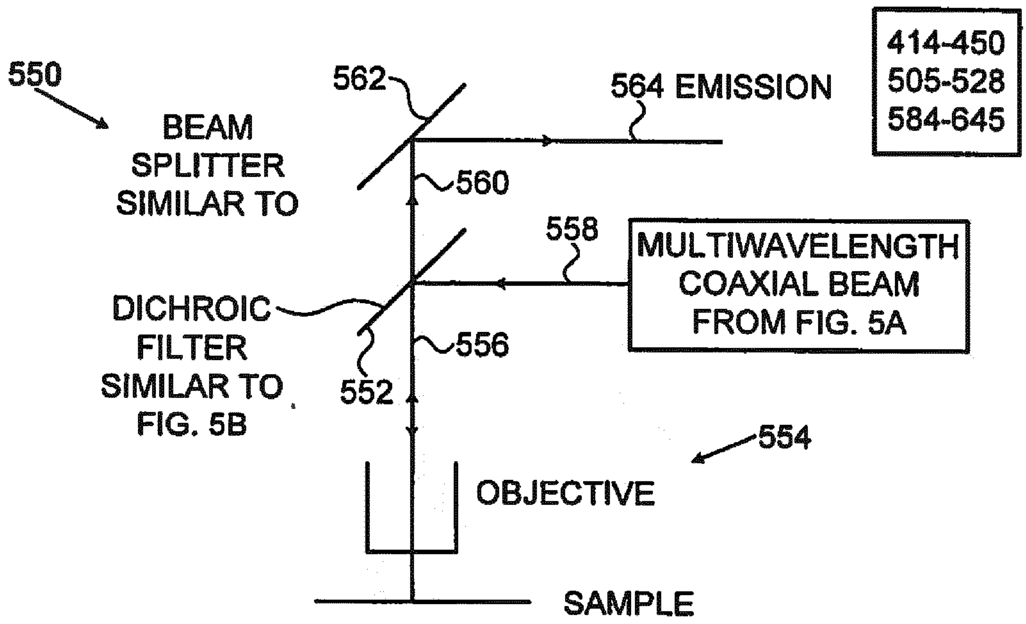
550
BEAM SPLITTER SIMILAR TO
562
564 EMISSION
414-450
505-528
584-645
560
558
MULTIWAVELENGTH COAXIAL BEAM FROM FIG. 5A
DICHROIC FILTER SIMILAR TO FIG. 5B
552
556
554
OBJECTIVE
SAMPLE

FIG. 8C

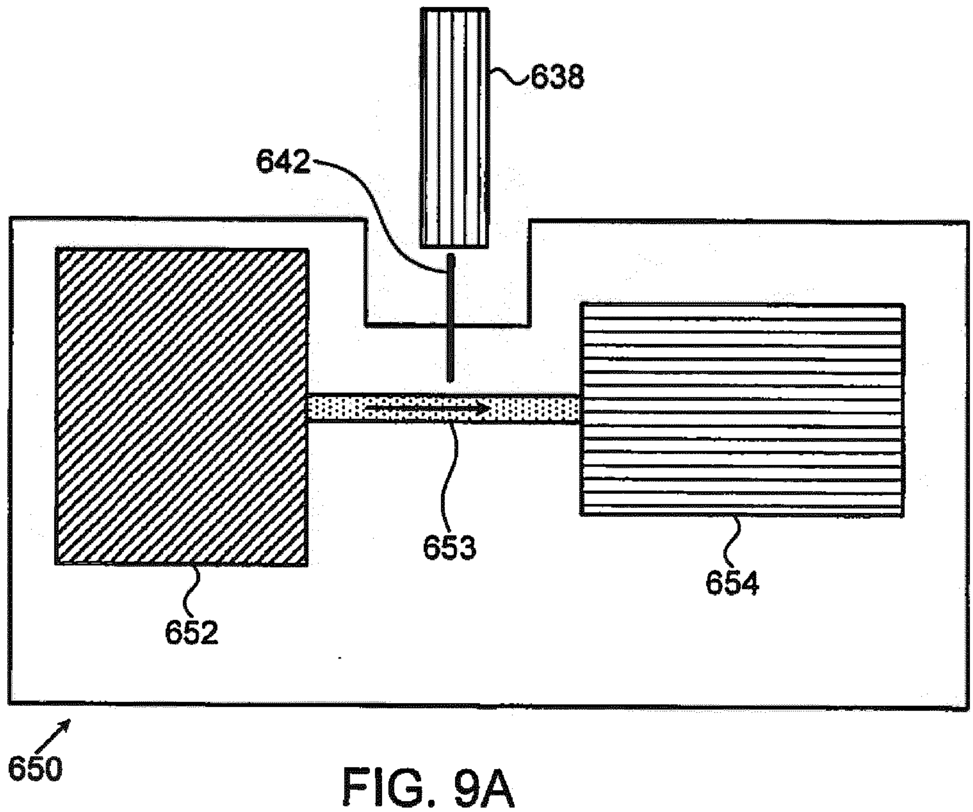
638
642
653
652
654
650

FIG. 9A

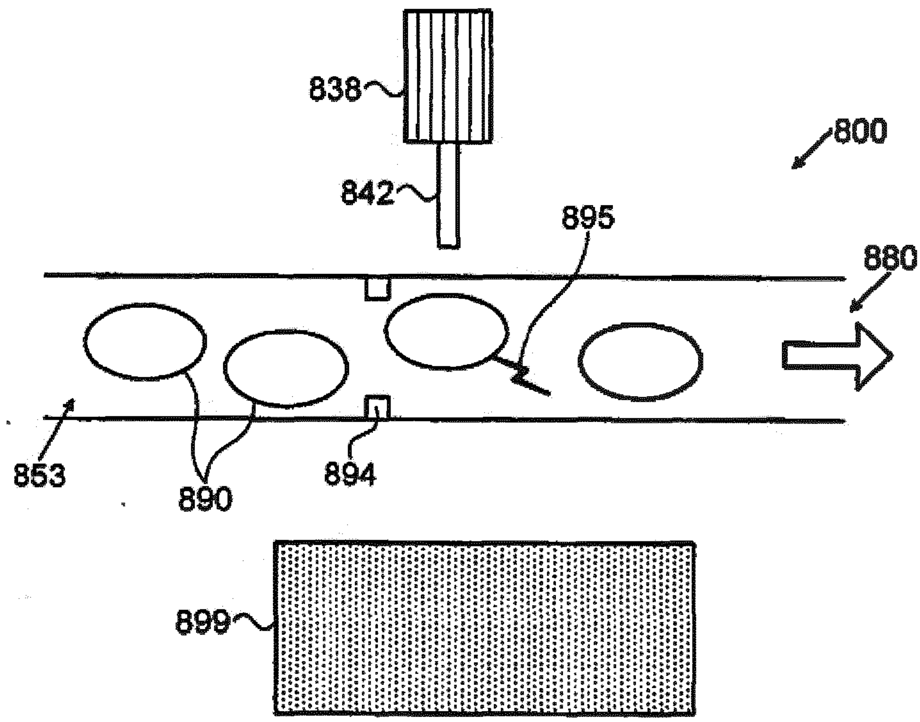


FIG. 9B

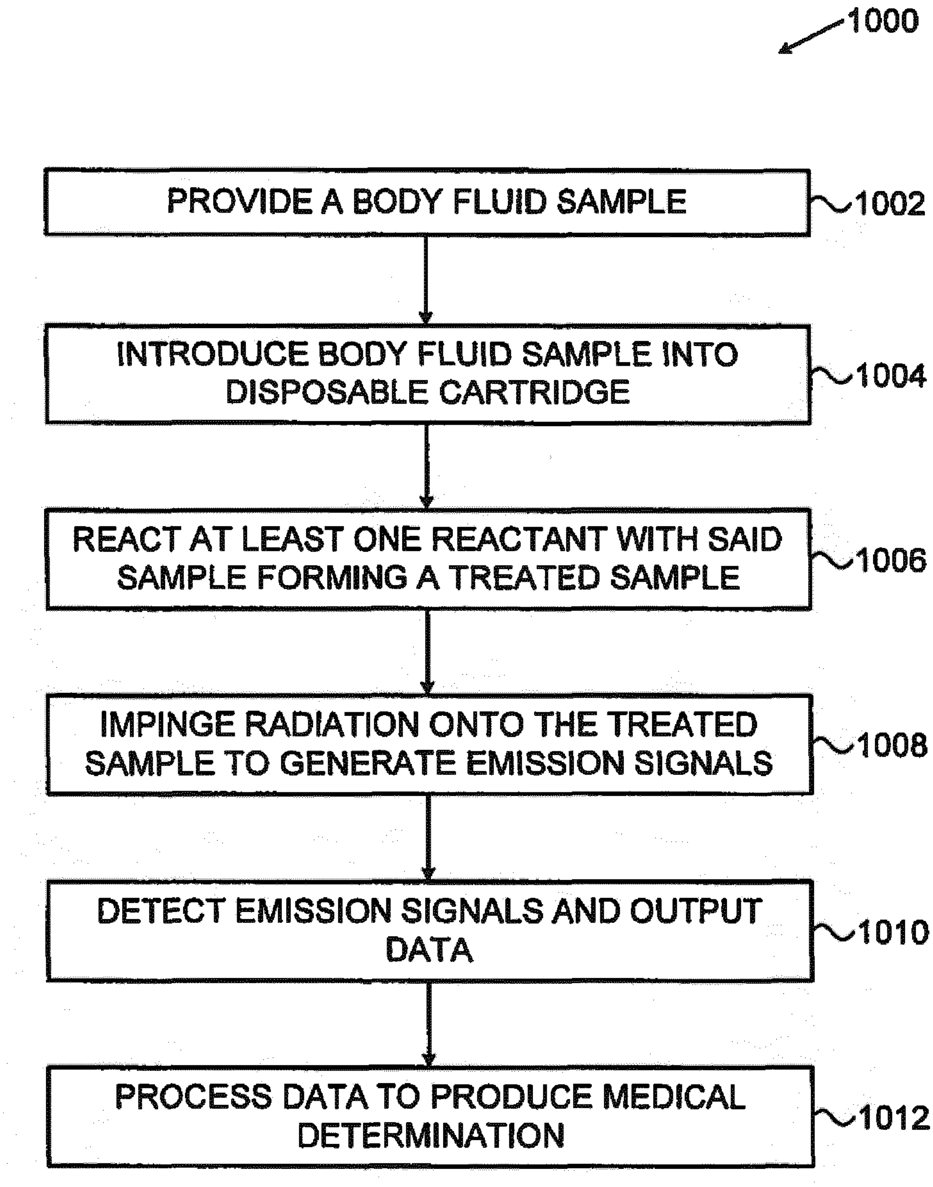
1000
PROVIDE A BODY FLUID SAMPLE
1002
INTRODUCE BODY FLUID SAMPLE INTO DISPOSABLE CARTRIDGE
1004
REACT AT LEAST ONE REACTANT WITH SAID SAMPLE FORMING A TREATED SAMPLE
1006
IMPINGE RADIATION ONTO THE TREATED SAMPLE TO GENERATE EMISSION SIGNALS
1008
DETECT EMISSION SIGNALS AND OUTPUT DATA
1010
PROCESS DATA TO PRODUCE MEDICAL DETERMINATION
1012

FIG. 10

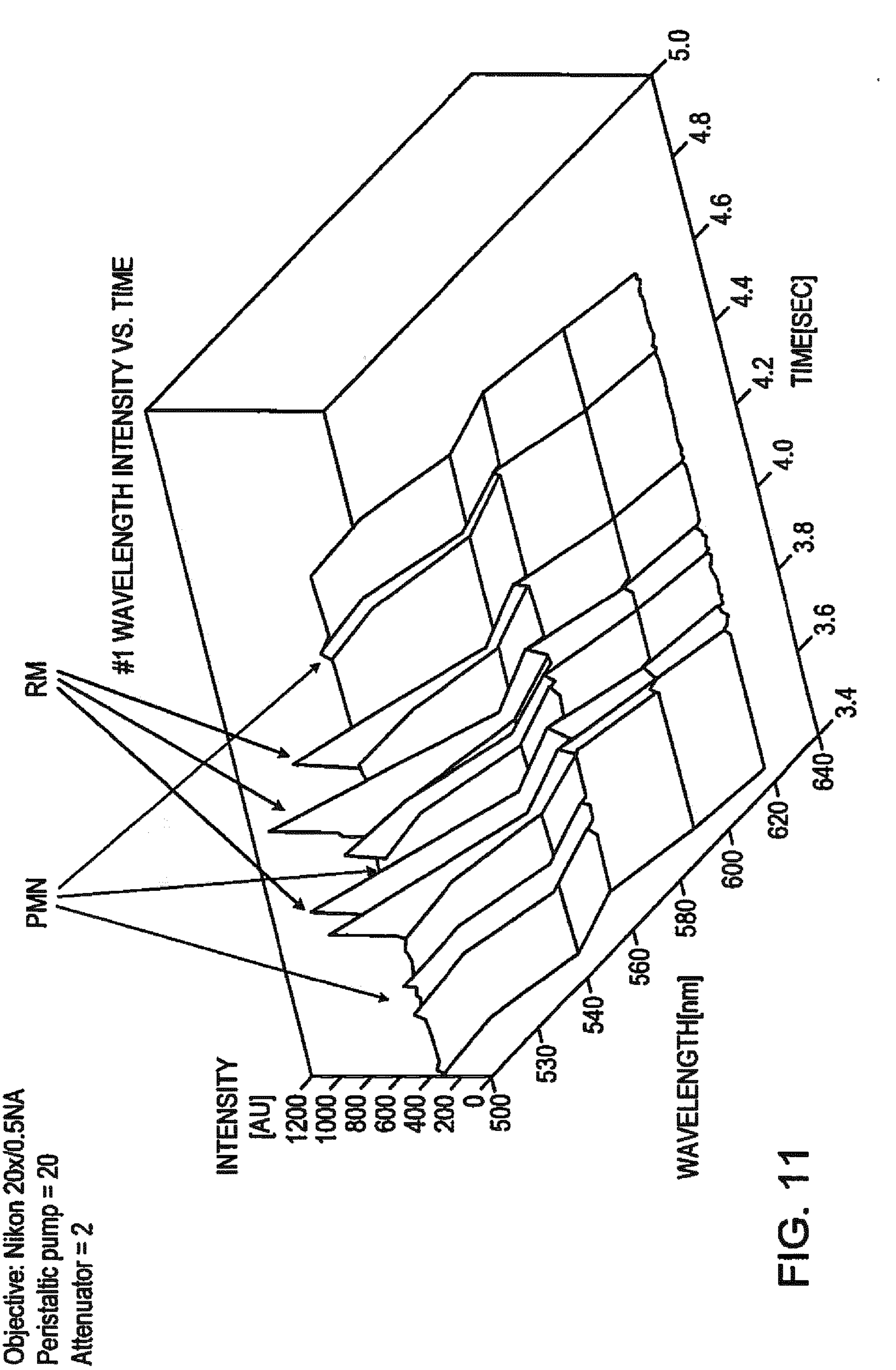


- Objective: Nikon 20x/0.5NA
- Peristaltic pump = 20
- Attenuator = 2

FIG. 11

FIG. 12A
(Grayscale)

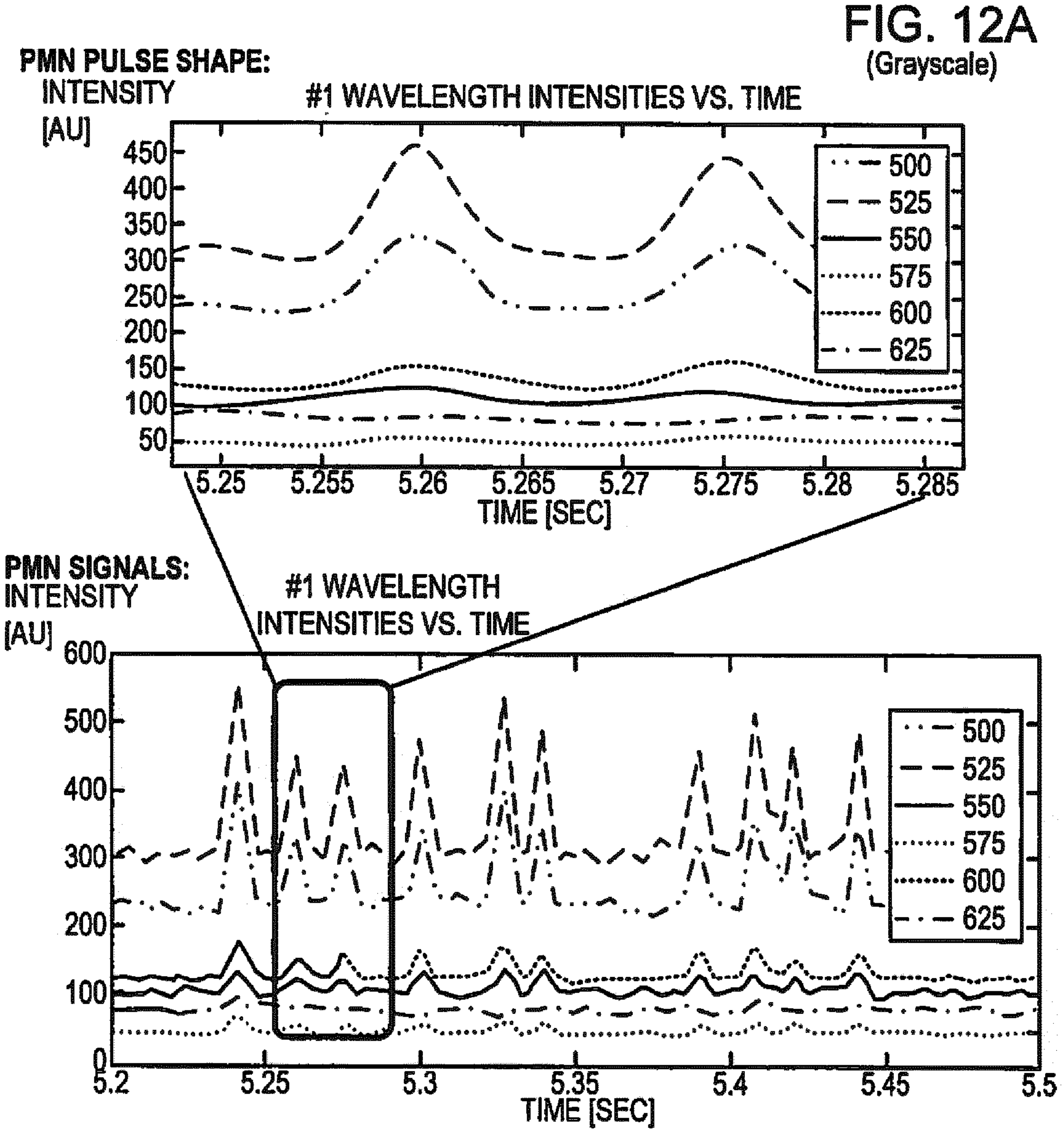


FIG. 12B

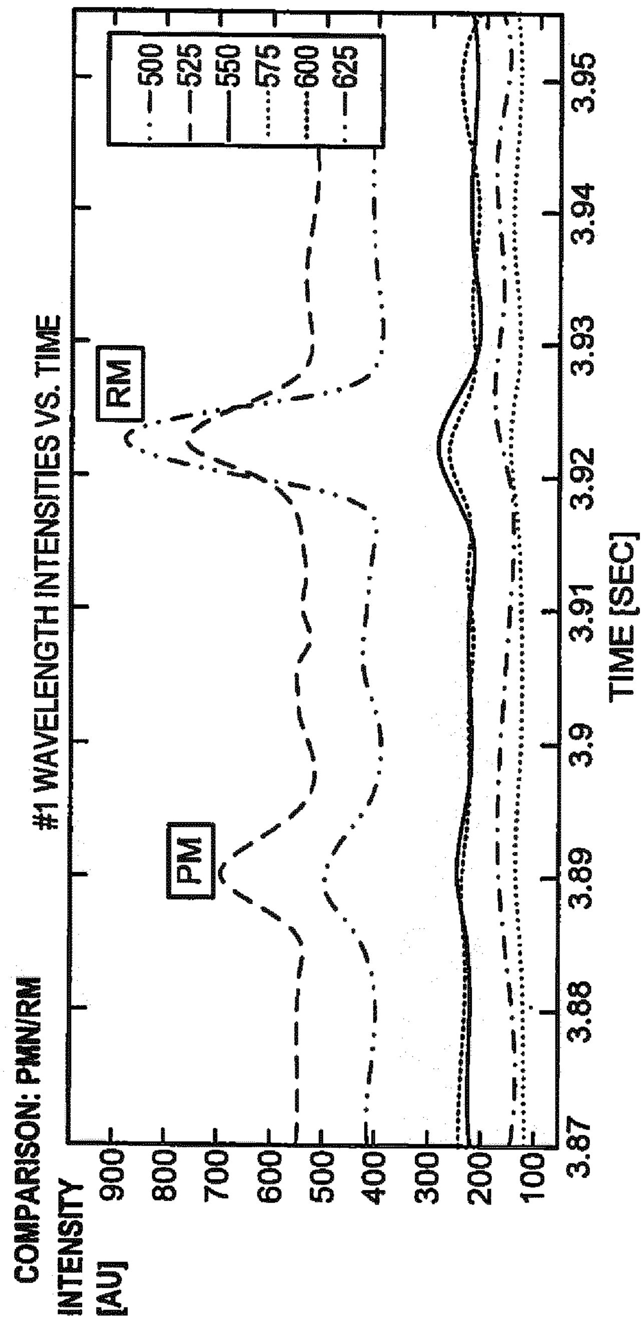
COMPARISON: PMN/RM
#1 WAVELENGTH INTENSITIES VS. TIME
INTENSITY
[AU]
900
800
700
600
500
400
300
200
100
PM
RM
500
525
550
575
600
625
3.87
3.88
3.89
3.9
3.91
3.92
3.93
3.94
3.95
TIME [SEC]

FIG. 12C

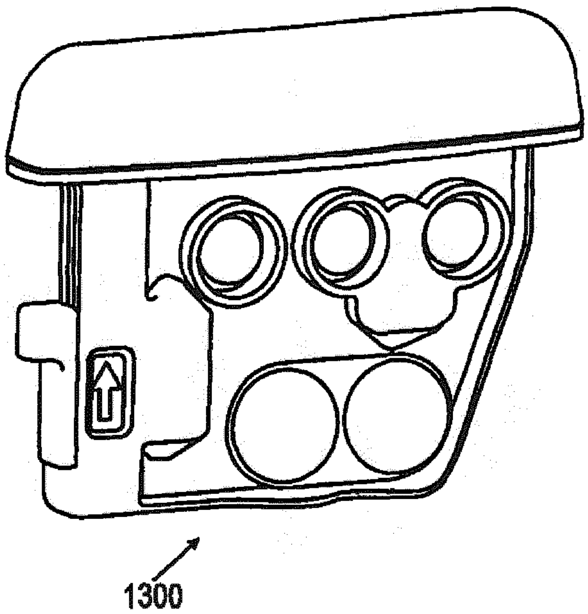


FIG. 13A

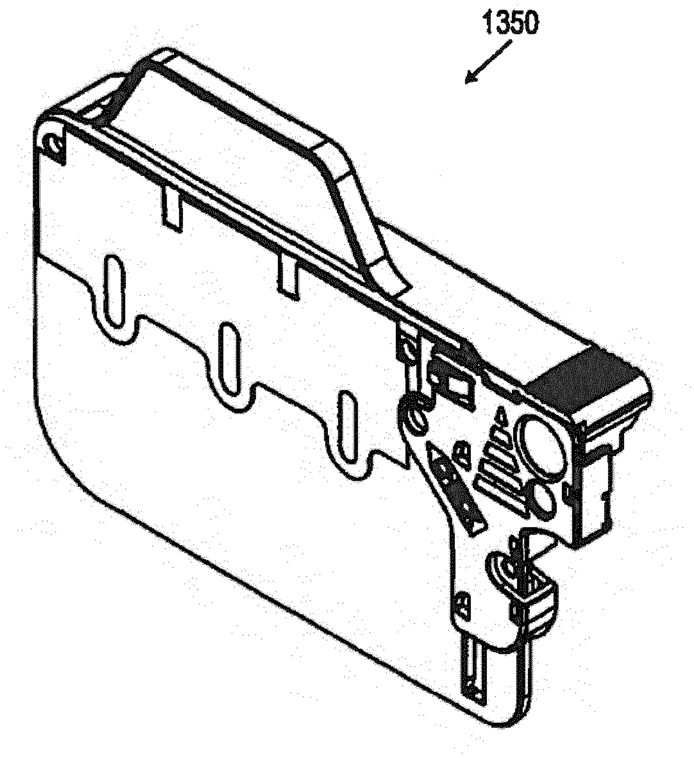


FIG. 13B

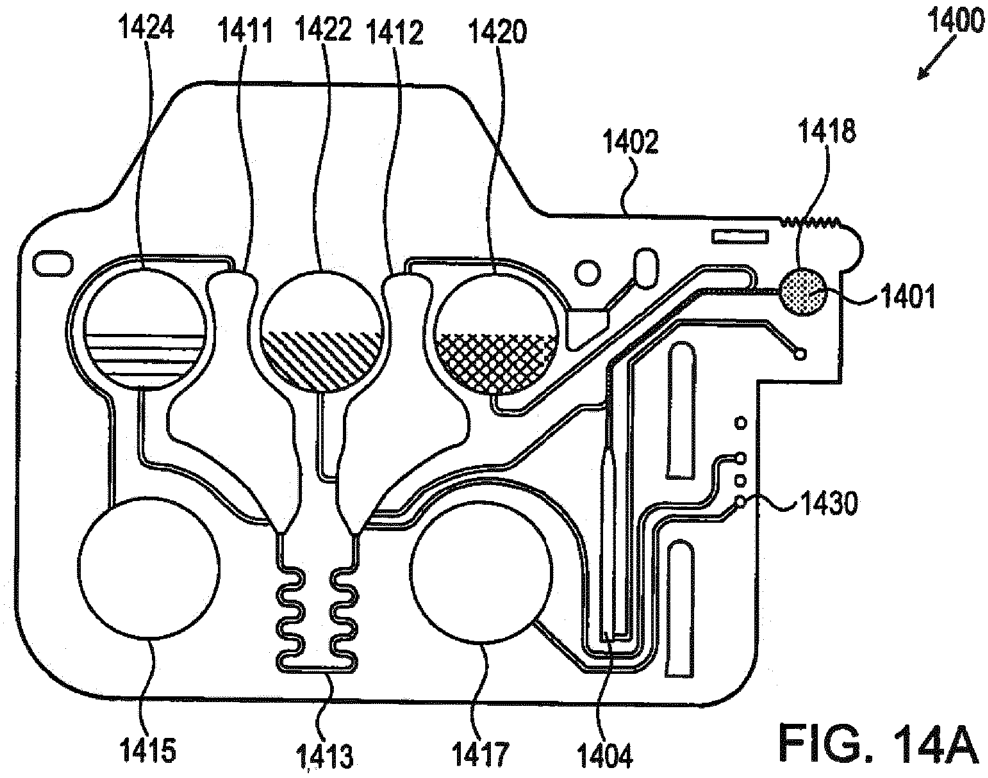


FIG. 14A

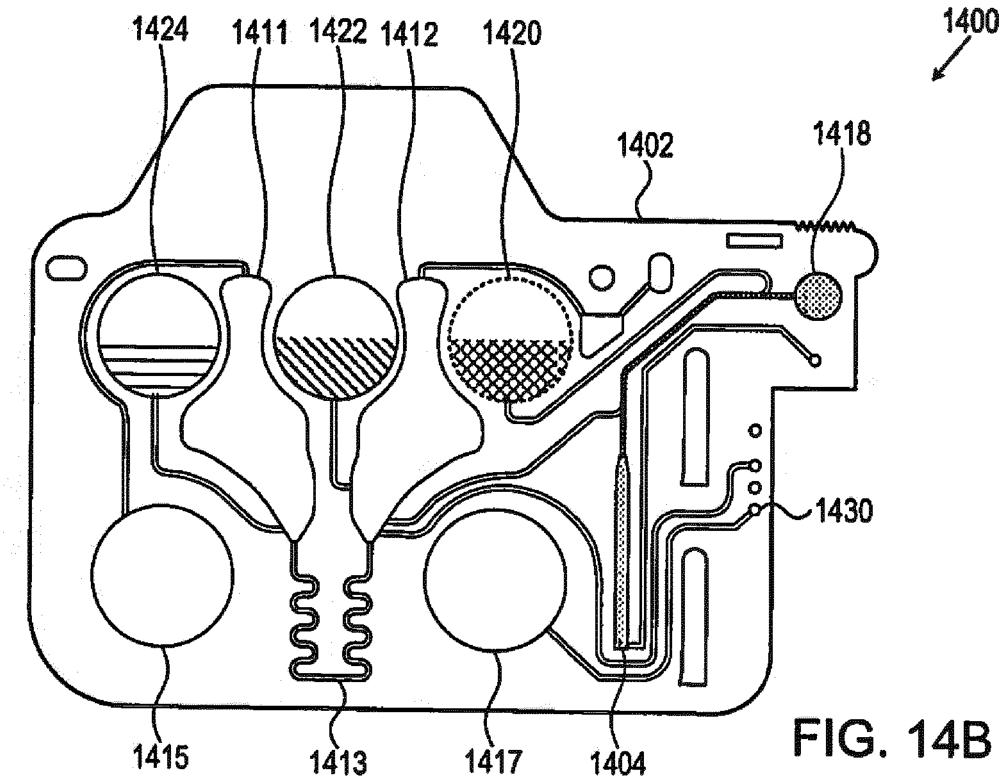


FIG. 14B

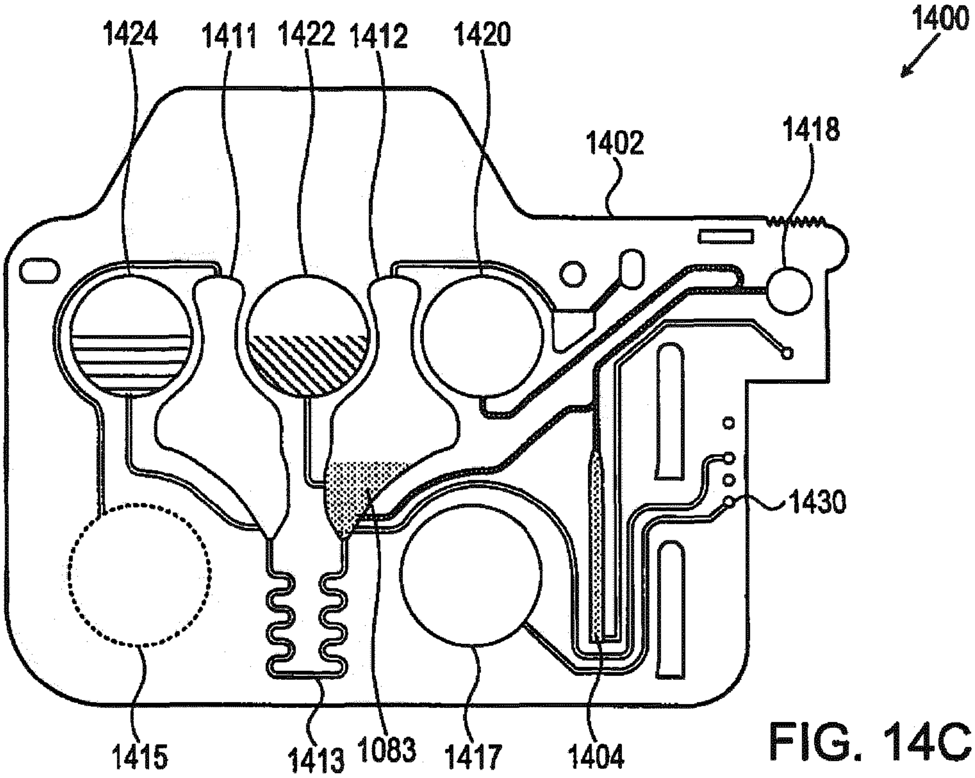


FIG. 14C

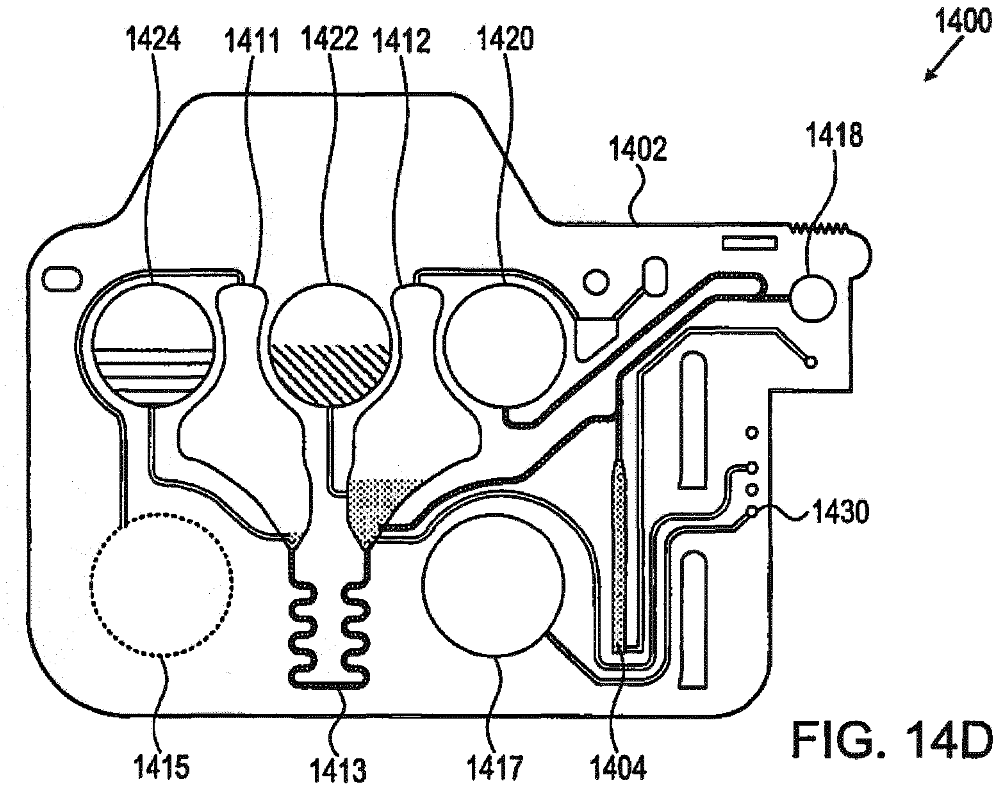


FIG. 14D

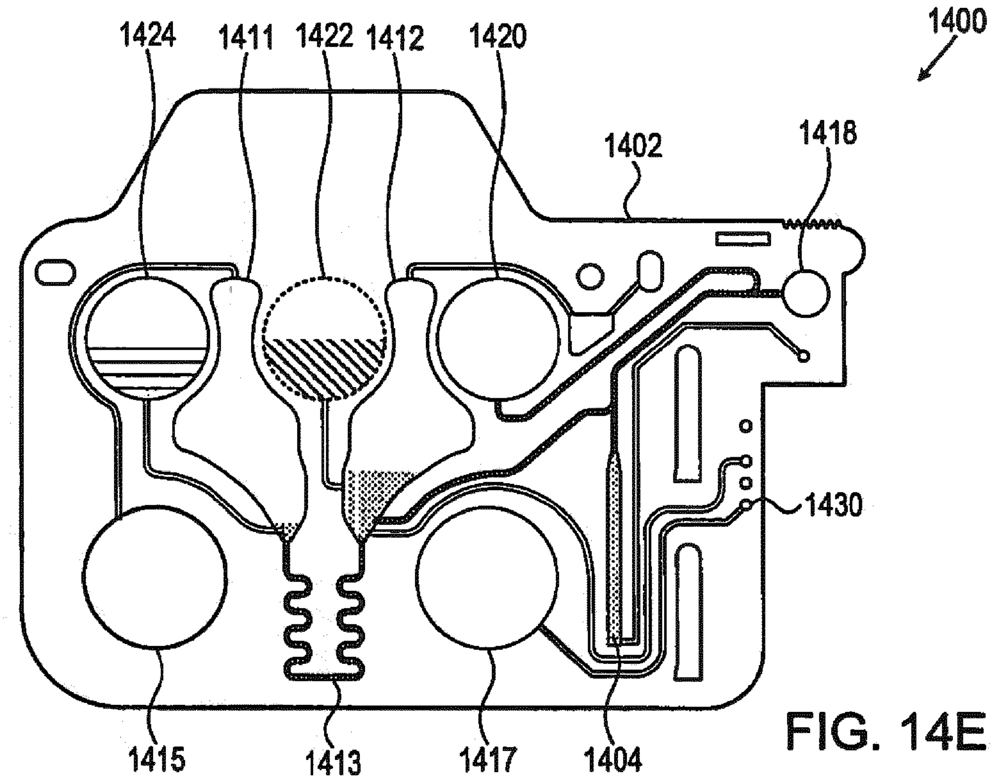


FIG. 14E

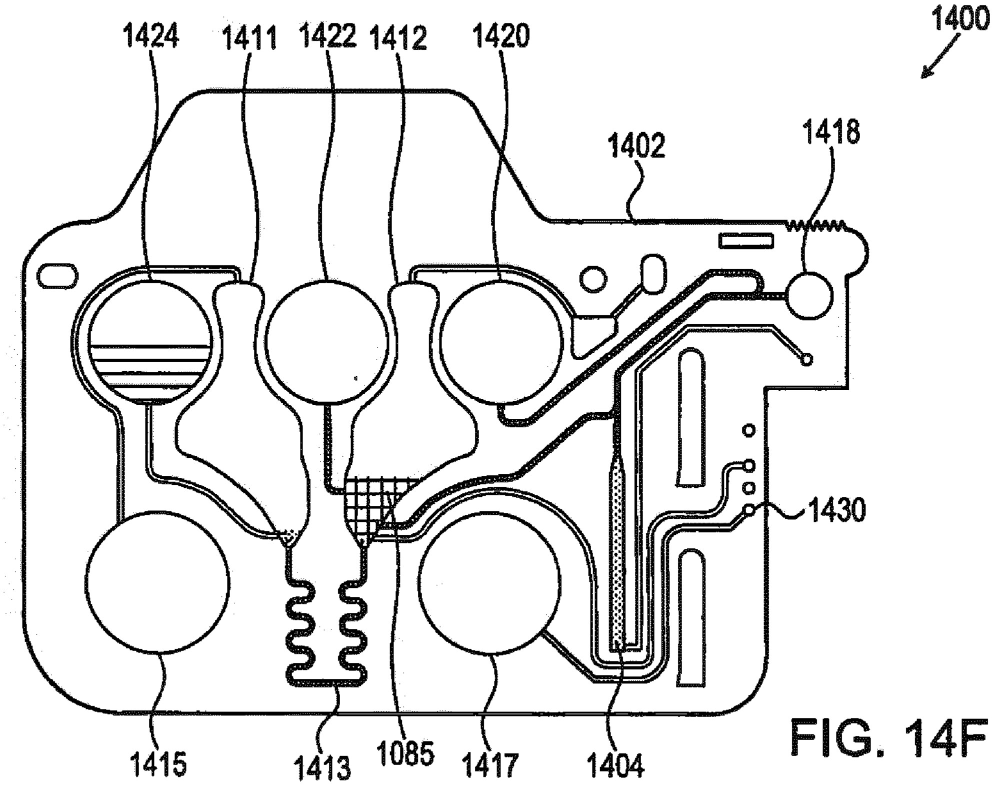


FIG. 14F

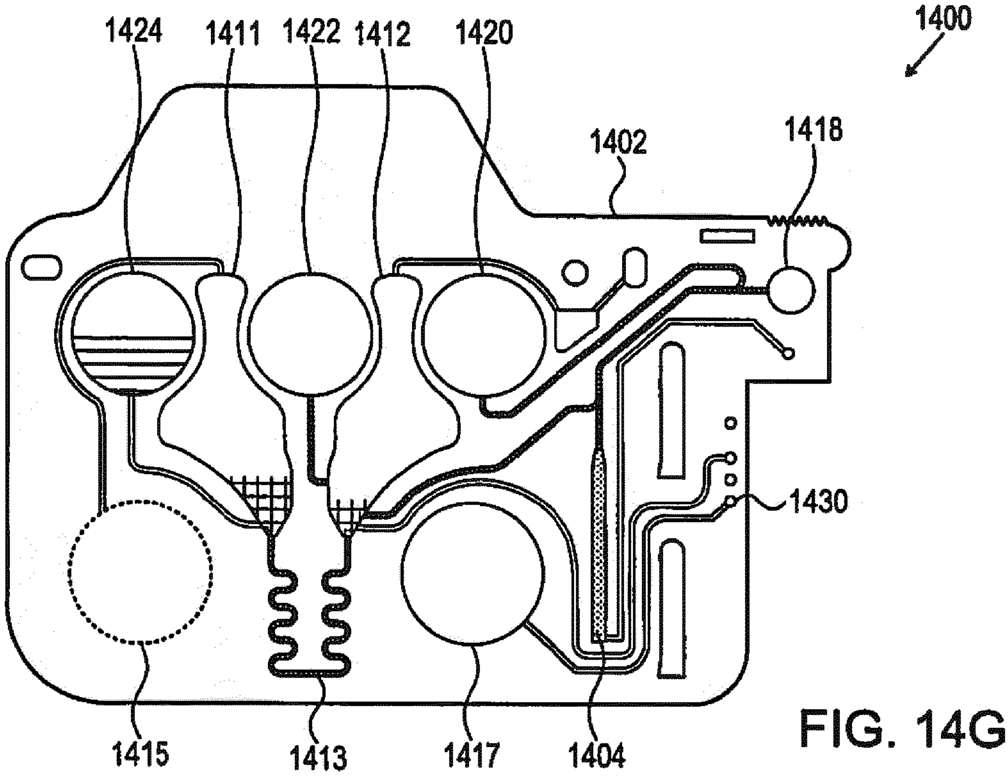


FIG. 14G

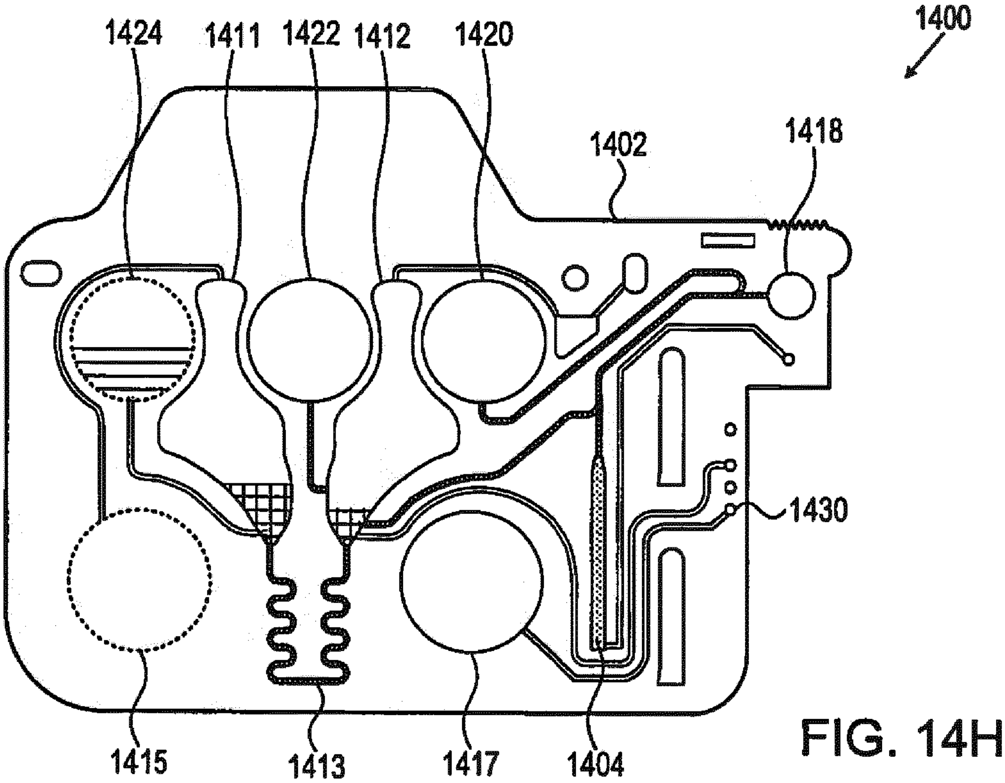


FIG. 14H

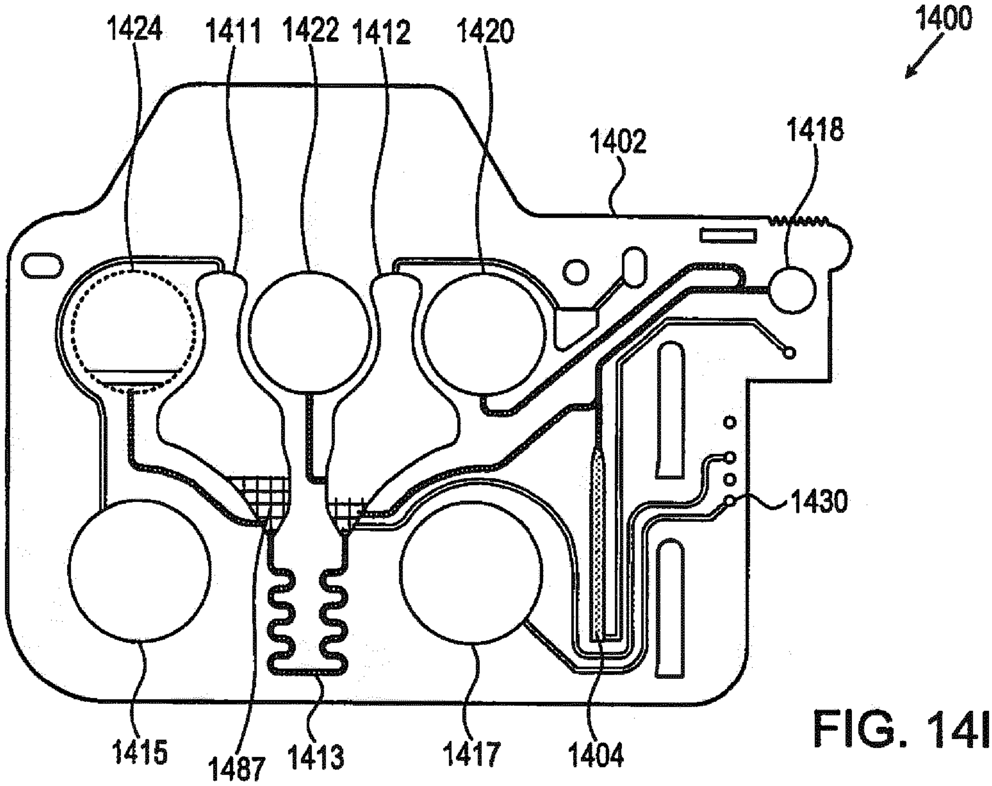


FIG. 14I

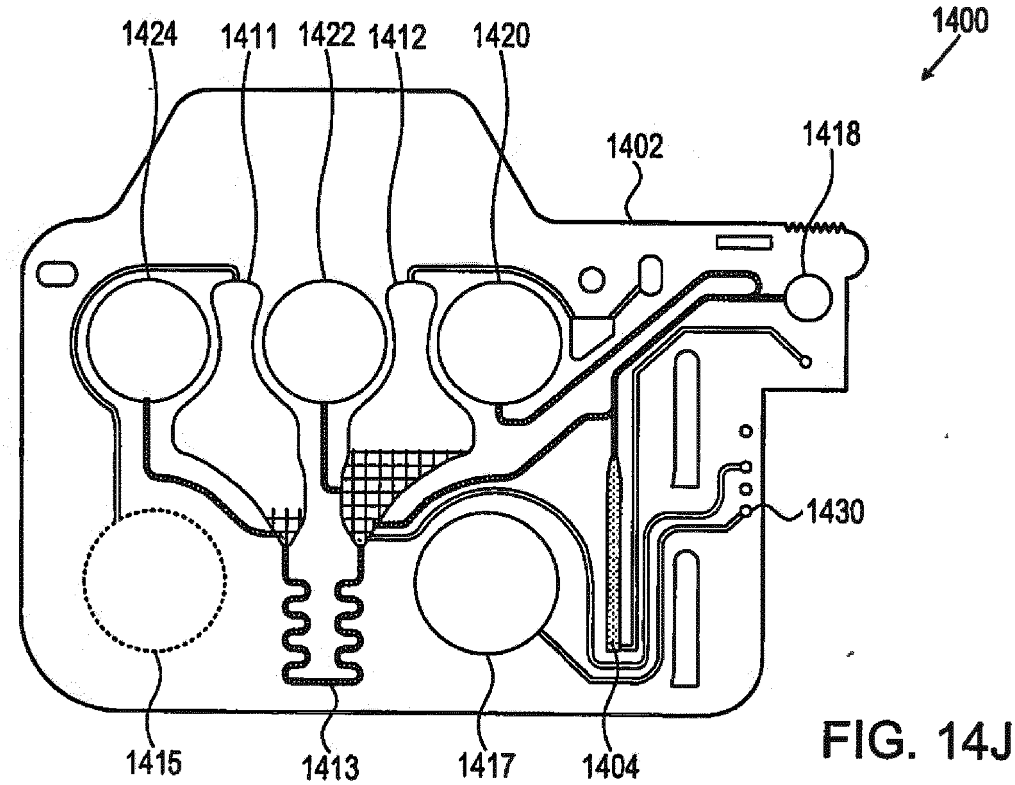


FIG. 14J

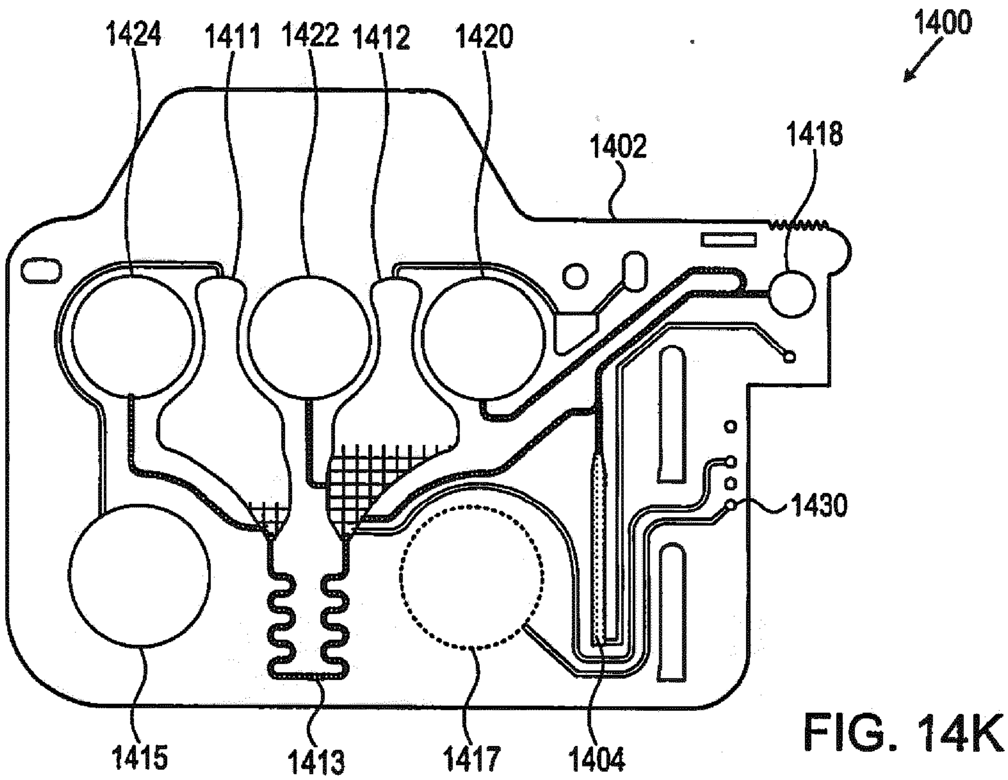


FIG. 14K

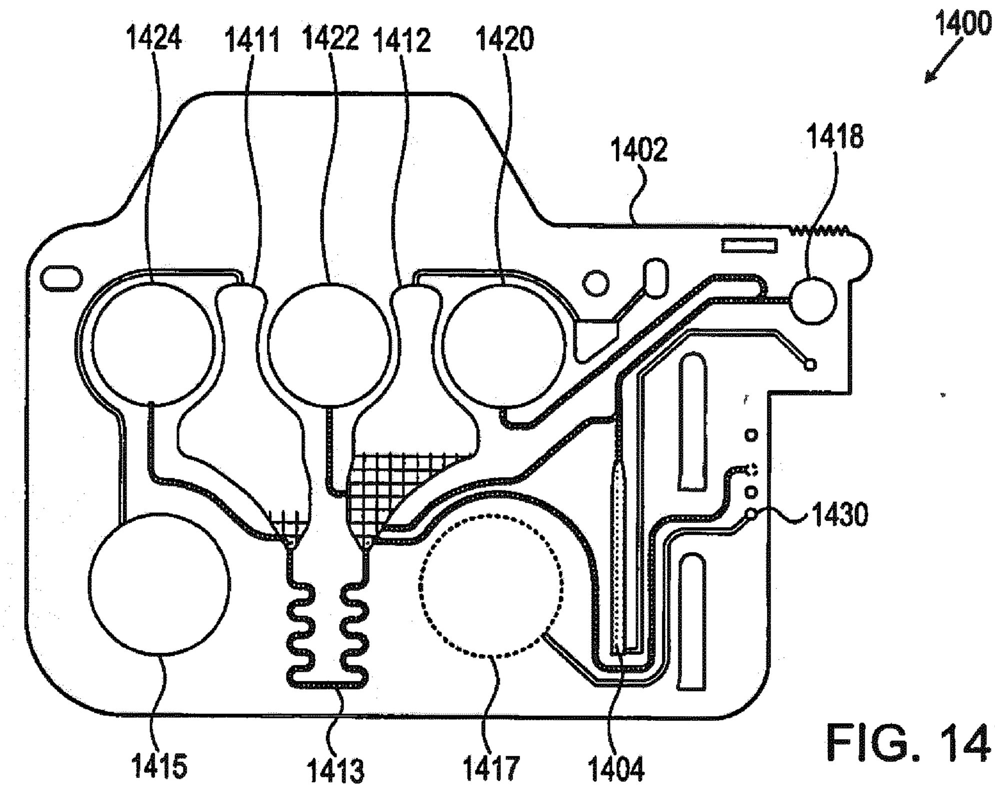


FIG. 14L

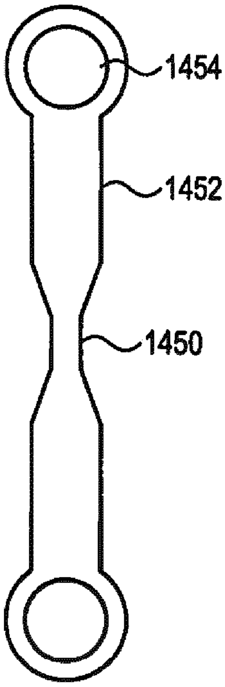


FIG. 14M

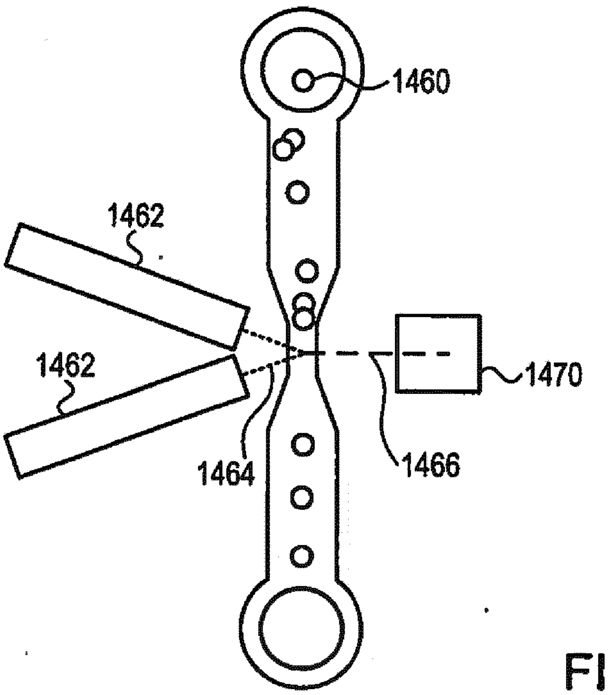


FIG. 14N

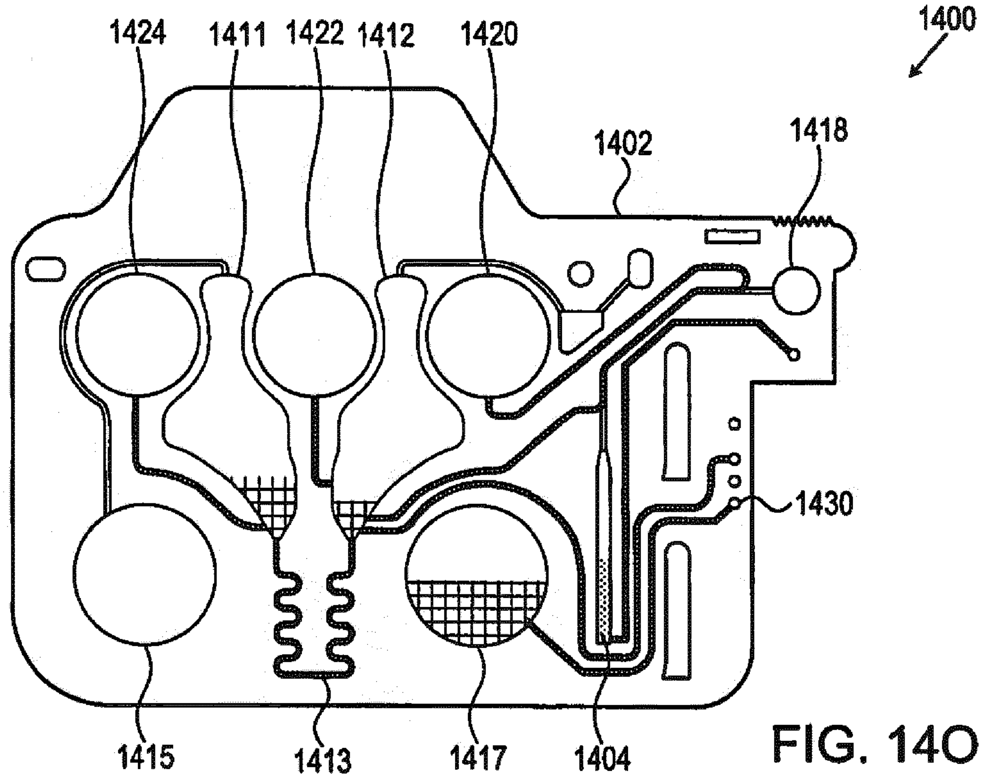
1400
1424
1411
1422
1412
1420
1402
1418
1430
1415
1413
1417
1404

FIG. 14O

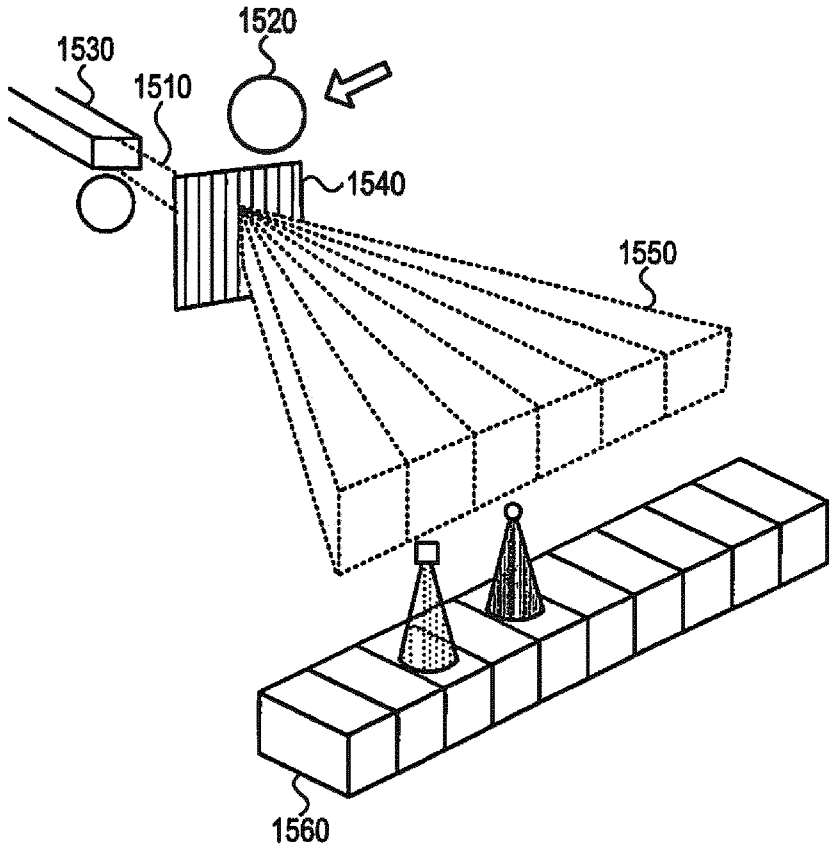


FIG. 15

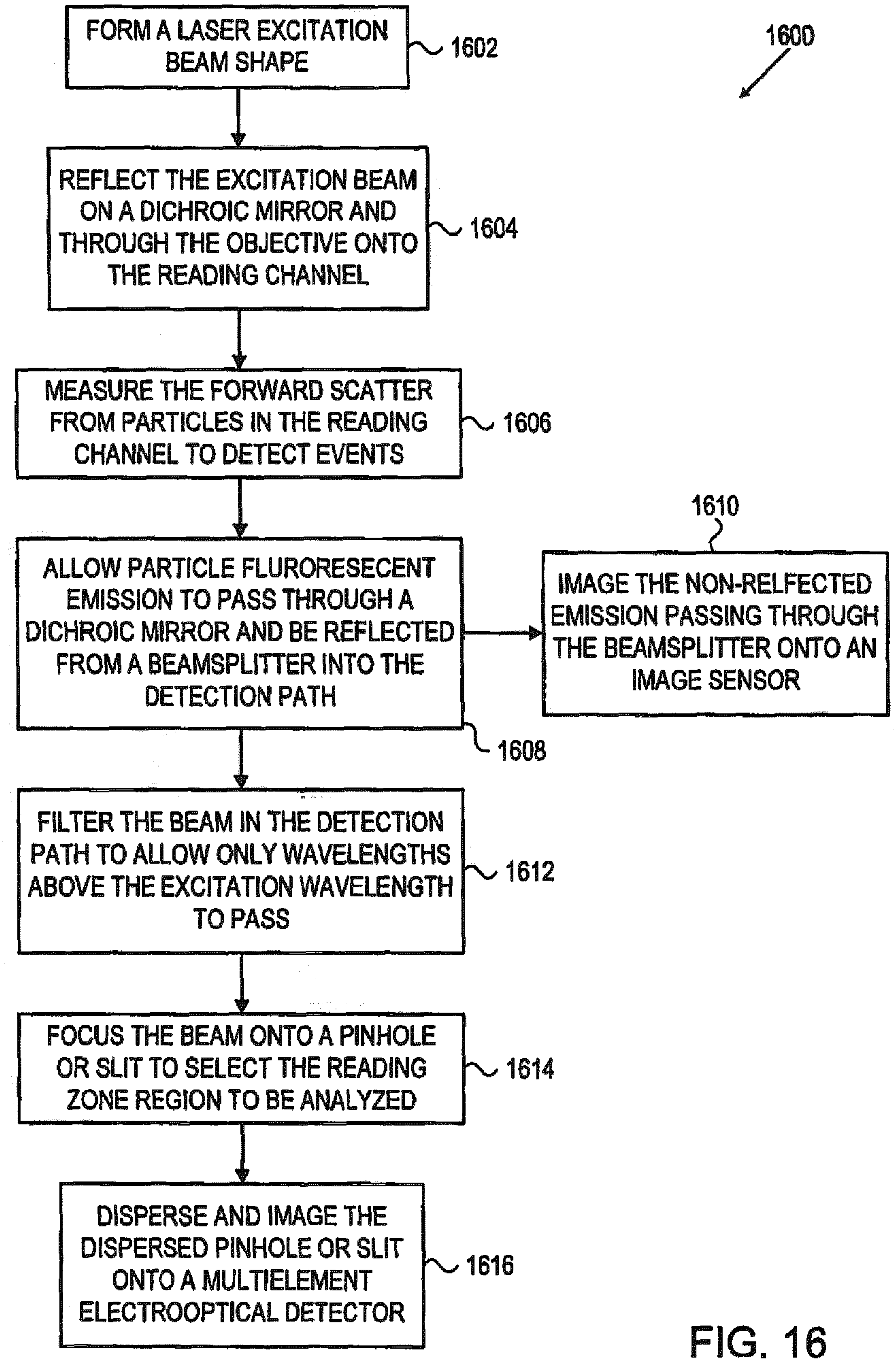
FORM A LASER EXCITATION BEAM SHAPE
1602
1600
REFLECT THE EXCITATION BEAM ON A DICHROIC MIRROR AND THROUGH THE OBJECTIVE ONTO THE READING CHANNEL
1604
MEASURE THE FORWARD SCATTER FROM PARTICLES IN THE READING CHANNEL TO DETECT EVENTS
1606
1610
ALLOW PARTICLE FLURORESECENT EMISSION TO PASS THROUGH A DICHROIC MIRROR AND BE REFLECTED FROM A BEAMSPLITTER INTO THE DETECTION PATH
IMAGE THE NON-RELFECTED EMISSION PASSING THROUGH THE BEAMSPLITTER ONTO AN IMAGE SENSOR
1608
FILTER THE BEAM IN THE DETECTION PATH TO ALLOW ONLY WAVELENGTHS ABOVE THE EXCITATION WAVELENGTH TO PASS
1612
FOCUS THE BEAM ONTO A PINHOLE OR SLIT TO SELECT THE READING ZONE REGION TO BE ANALYZED
1614
DISPERSE AND IMAGE THE DISPERSED PINHOLE OR SLIT ONTO A MULTIELEMENT ELECTROOPTICAL DETECTOR
1616

FIG. 16

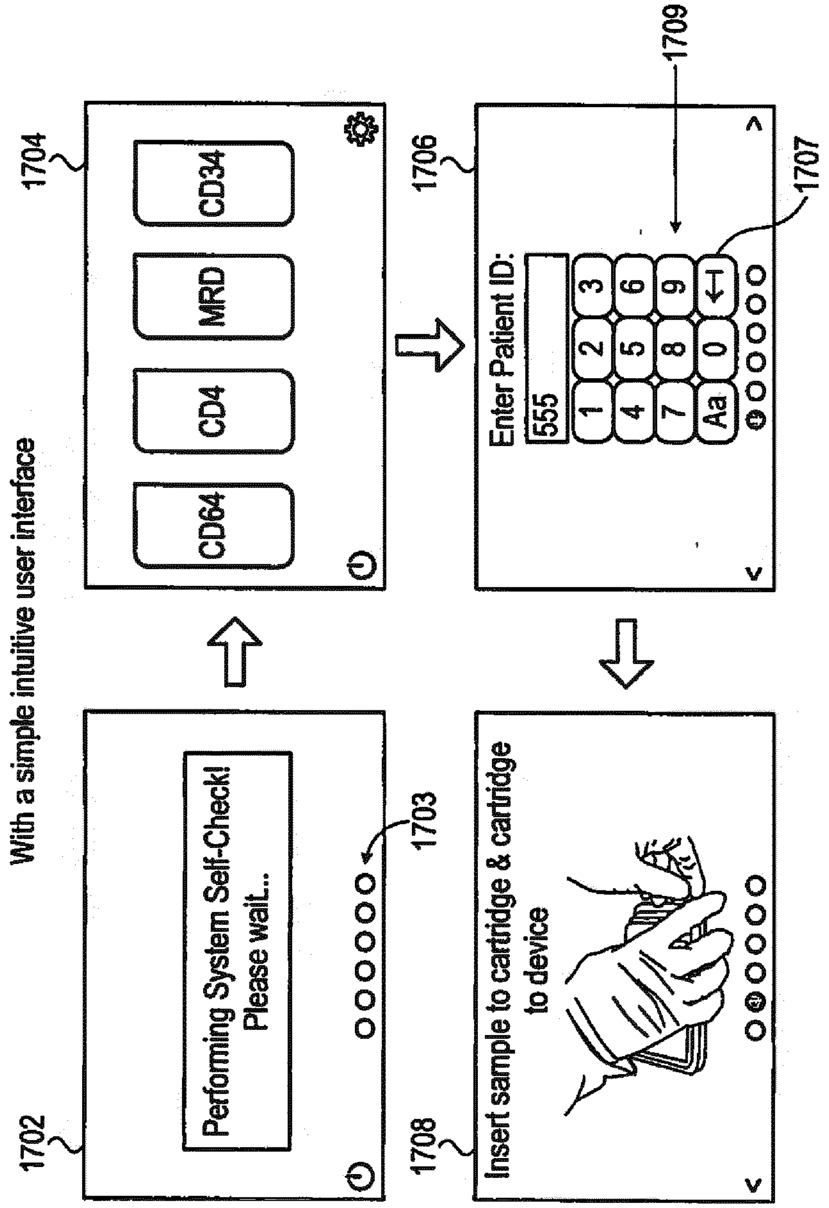
With a simple intuitive user interface
1702
Performing System Self-Check!
Please wait...
1703
1704
CD64
CD4
MRD
CD34
1706
Enter Patient ID:
555
1
2
3
4
5
6
7
8
9
Aa
0
1709
1707
1708
Insert sample to cartridge & cartridge
to device

FIG. 17A

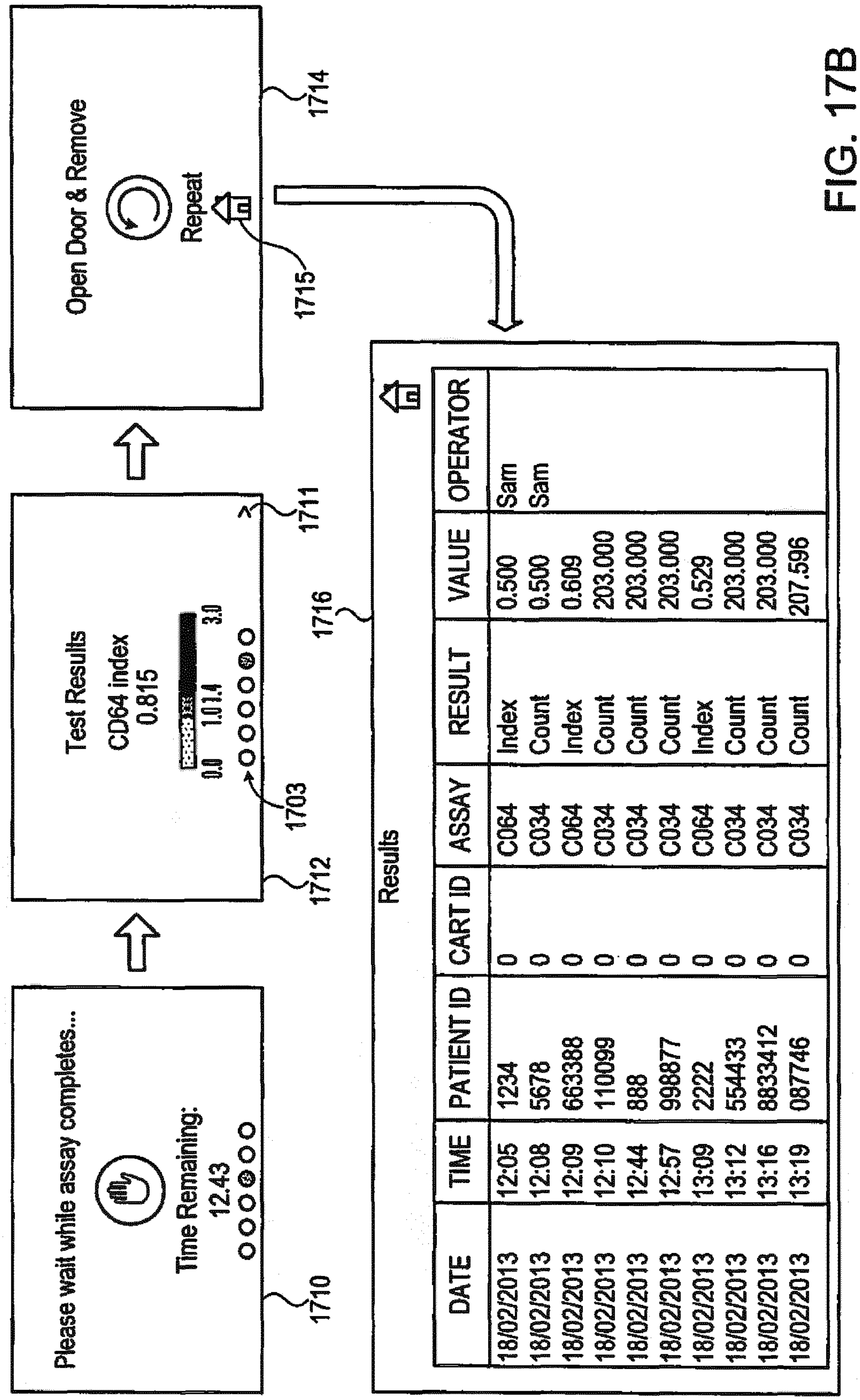


| DATE | TIME | PATIENT ID | CART ID | ASSAY | RESULT | VALUE | OPERATOR |
|---|---|---|---|---|---|---|---|
| 18/02/2013 | 12:05 | 1234 | 0 | C064 | Index | 0.500 | Sam |
| 18/02/2013 | 12:08 | 5678 | 0 | C034 | Count | 0.500 | Sam |
| 18/02/2013 | 12:09 | 663388 | 0 | C064 | Index | 0.609 | |
| 18/02/2013 | 12:10 | 110099 | 0 | C034 | Count | 203.000 | |
| 18/02/2013 | 12:44 | 888 | 0 | C034 | Count | 203.000 | |
| 18/02/2013 | 12:57 | 998877 | 0 | C034 | Count | 203.000 | |
| 18/02/2013 | 13:09 | 2222 | 0 | C064 | Index | 0.529 | |
| 18/02/2013 | 13:12 | 554433 | 0 | C034 | Count | 203.000 | |
| 18/02/2013 | 13:16 | 8833412 | 0 | C034 | Count | 203.000 | |
| 18/02/2013 | 13:19 | 087746 | 0 | C034 | Count | 207.596 | |

FIG. 17B

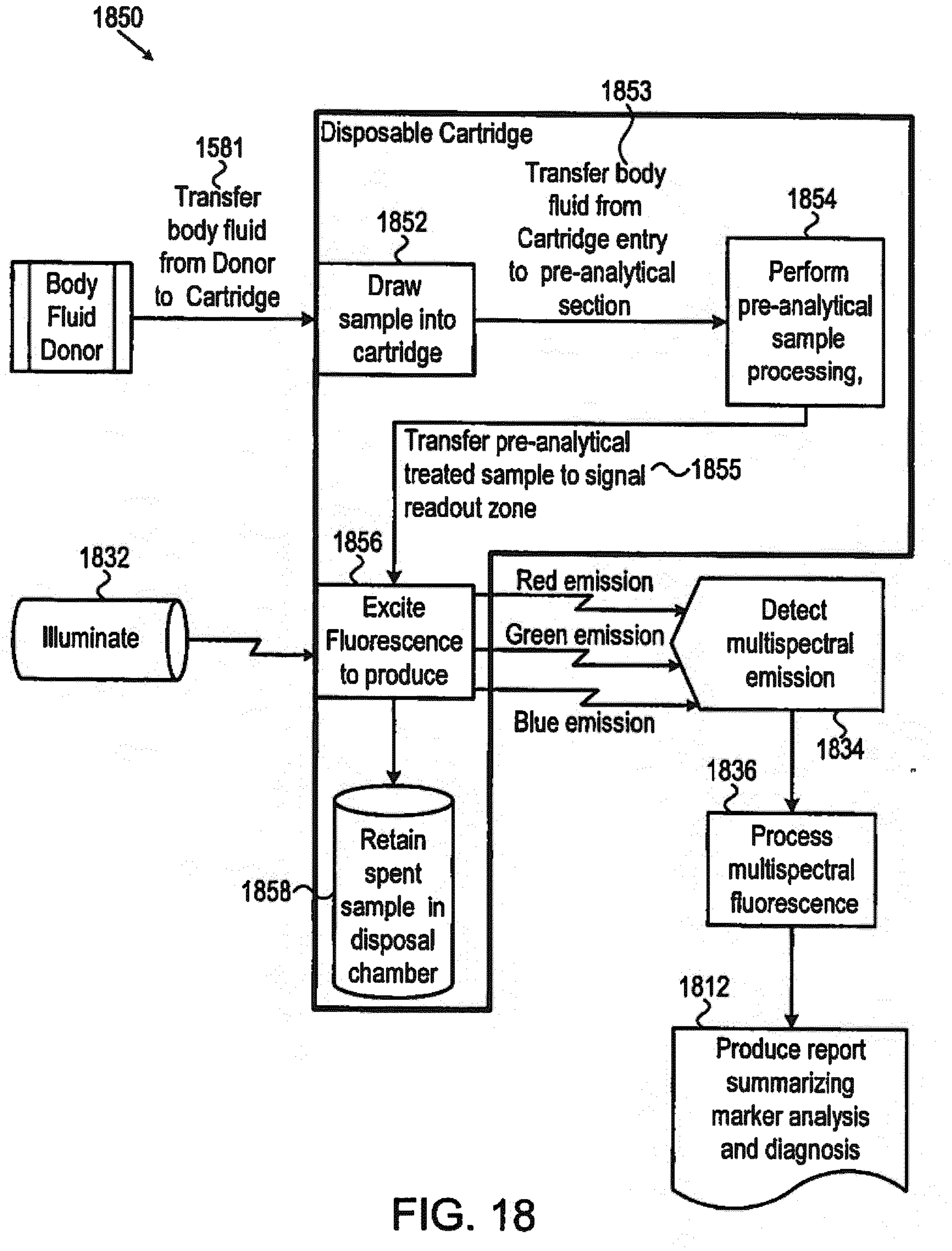
1850
1853
Disposable Cartridge
1581
Transfer body fluid from Donor to Cartridge
Transfer body fluid from Cartridge entry to pre-analytical section
1854
1852
Body Fluid Donor
Draw sample into cartridge
Perform pre-analytical sample processing,
Transfer pre-analytical treated sample to signal readout zone
1855
1856
1832
Illuminate
Excite Fluorescence to produce
Red emission
Green emission
Blue emission
Detect multispectral emission
1834
1836
Process multispectral fluorescence
1858
Retain spent sample in disposal chamber
1812
Produce report summarizing marker analysis and diagnosis

FIG. 18

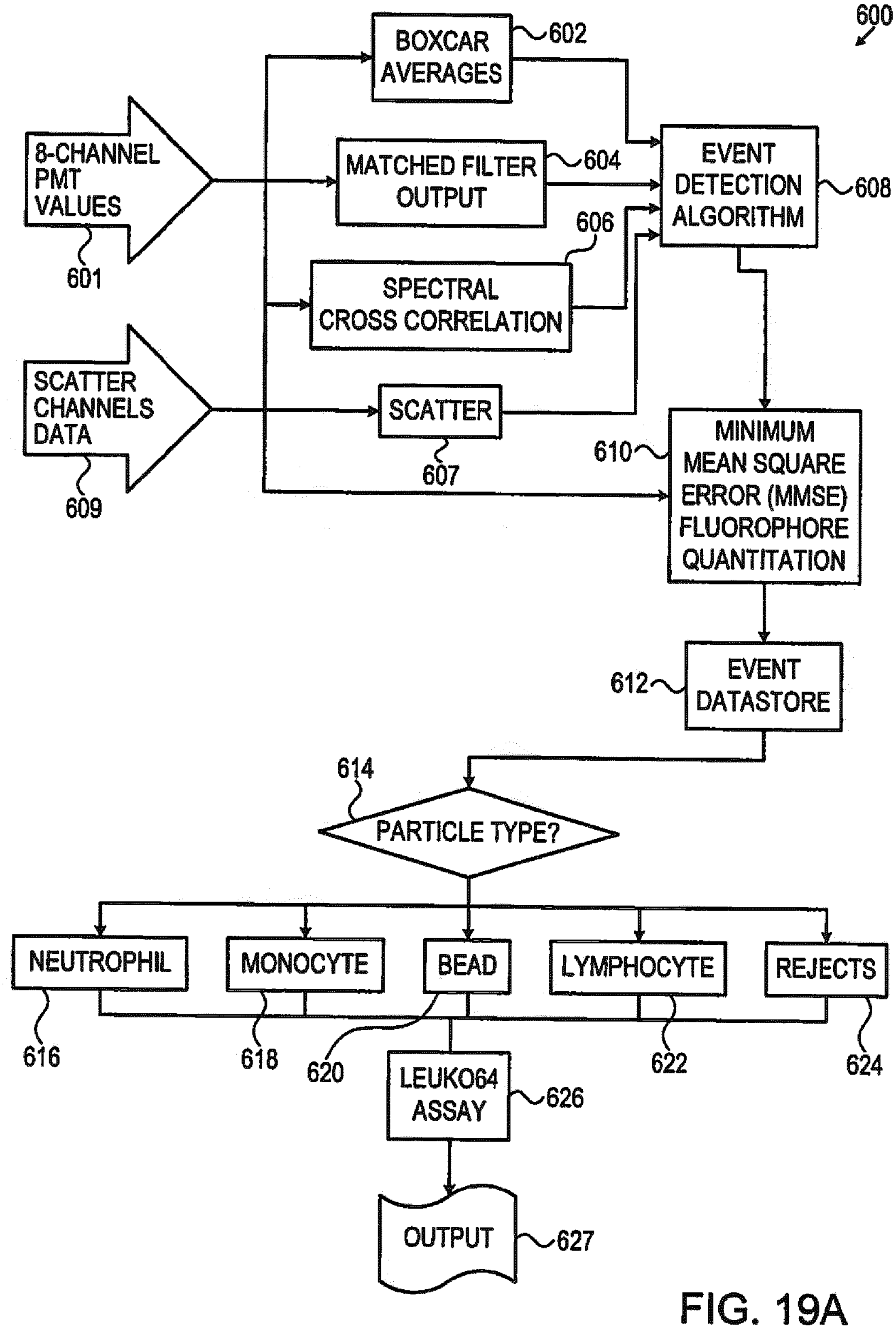
600
BOXCAR AVERAGES
602
8-CHANNEL PMT VALUES
601
MATCHED FILTER OUTPUT
604
EVENT DETECTION ALGORITHM
608
606
SPECTRAL CROSS CORRELATION
SCATTER CHANNELS DATA
609
SCATTER
607
610
MINIMUM MEAN SQUARE ERROR (MMSE) FLUOROPHORE QUANTITATION
612
EVENT DATASTORE
614
PARTICLE TYPE?
NEUTROPHIL
MONOCYTE
BEAD
LYMPHOCYTE
REJECTS
616
618
620
622
624
LEUKO64 ASSAY
626
OUTPUT
627

FIG. 19A

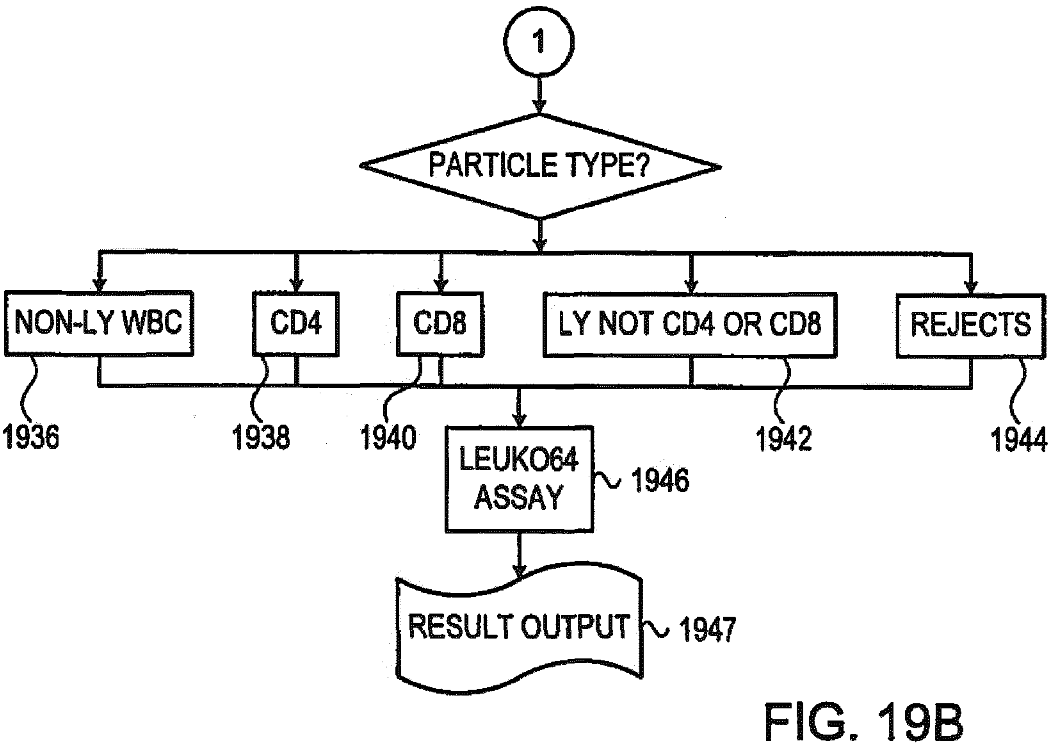
1
PARTICLE TYPE?
NON-LY WBC
CD4
CD8
LY NOT CD4 OR CD8
REJECTS
1936
1938
1940
1942
1944
LEUKO64 ASSAY
1946
RESULT OUTPUT
1947

FIG. 19B

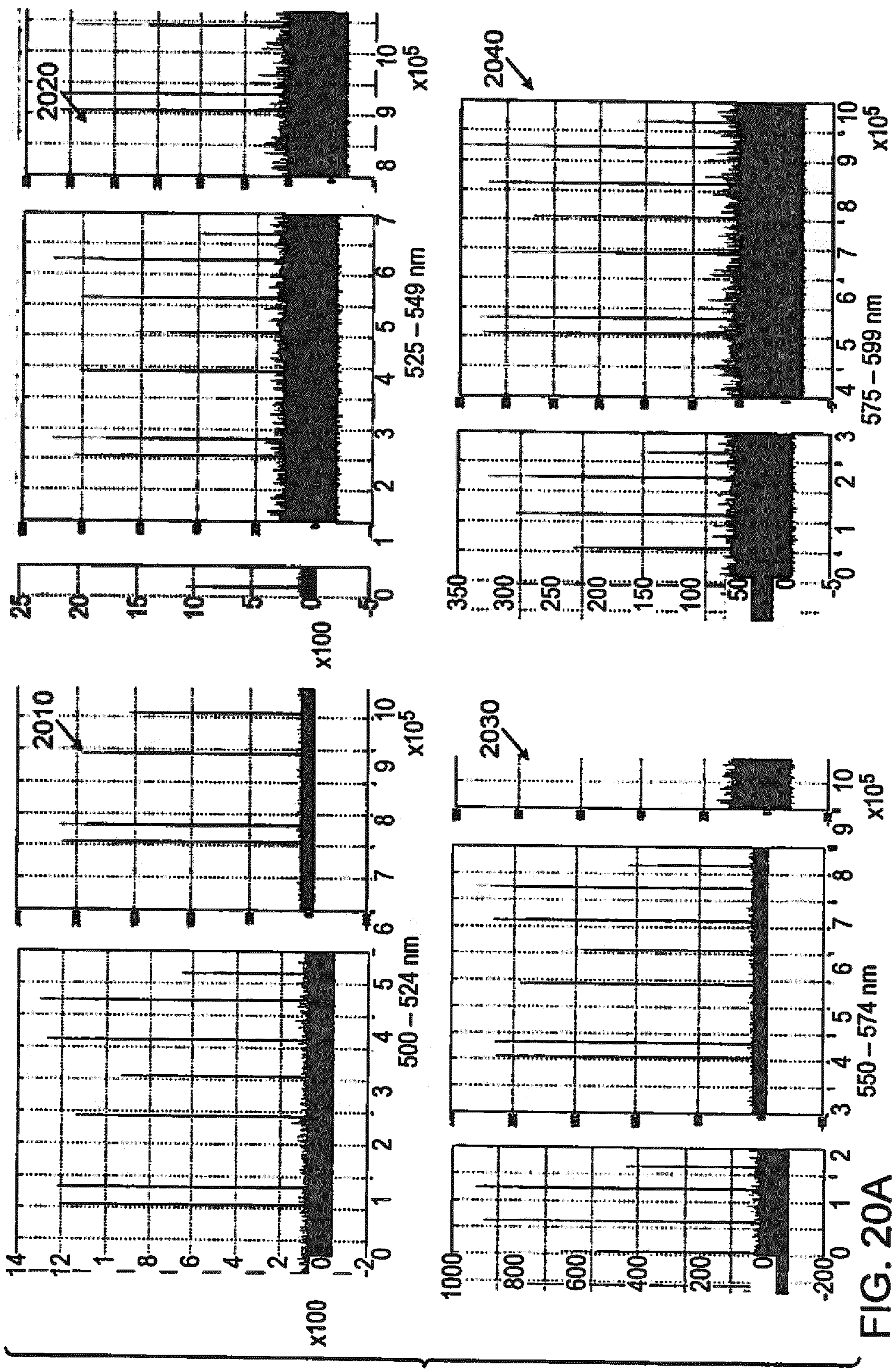
2010
2020
2030
2040
500 – 524 nm
525 – 549 nm
550 – 574 nm
575 – 599 nm
x100
x100
x10^5

FIG. 20A

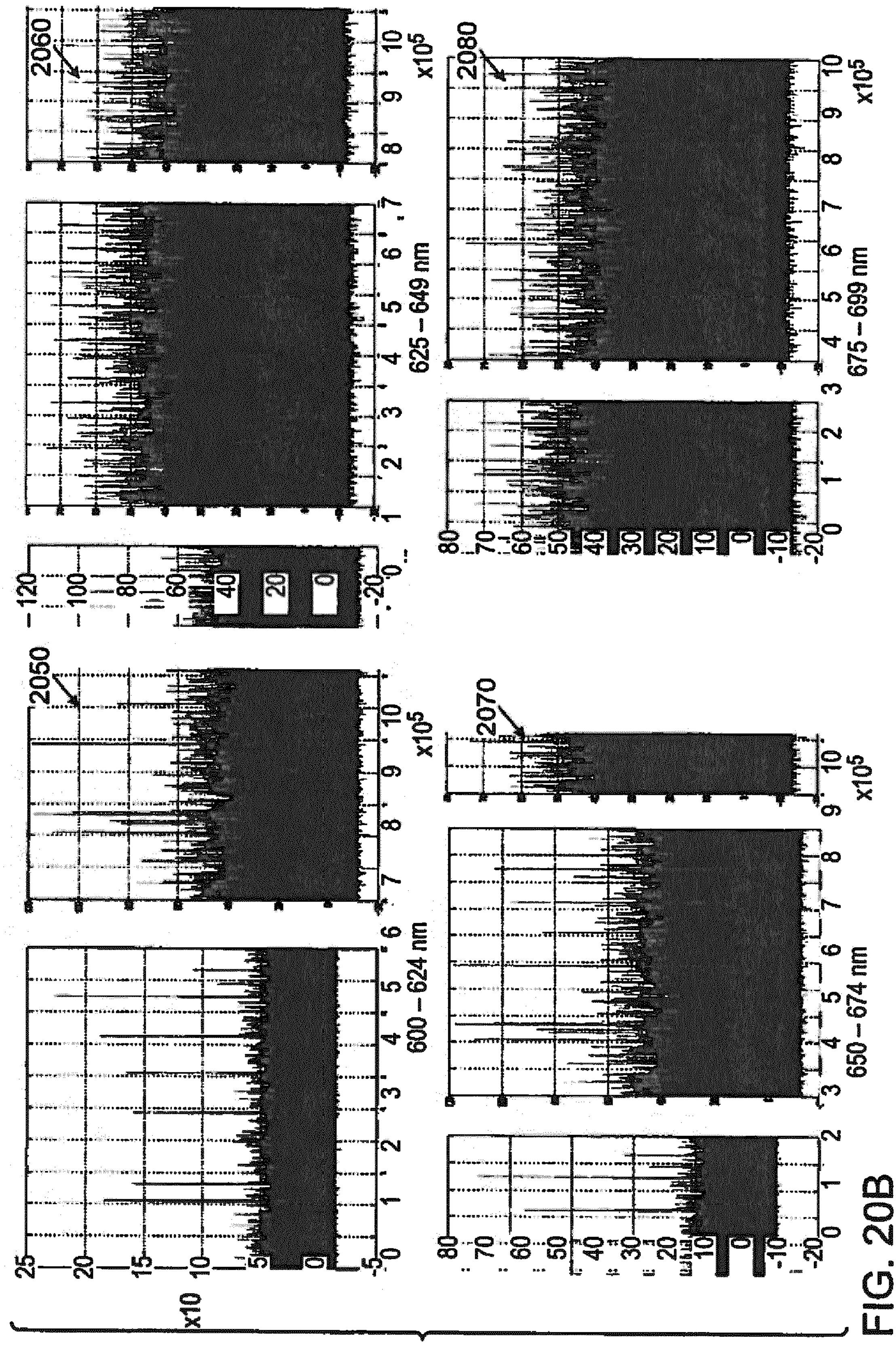
2050
2060
2070
2080
600 – 624 nm
625 – 649 nm
650 – 674 nm
675 – 699 nm
x10
x10^5

FIG. 20B

Solving for the fluor decomposition of the observed signal (pulse)

```
function [ x ] = FluorSolver( A, b )

%FluorSolver Solve for fluorophore composition of an observed 8-channel
%response
% Detailed explanation:
% This routine finds x, the solution of the equation Ax = b
% A is a matrix with 8 rows whose columns are normalized fluor
%        responses, i.e. the sum of the 8-elements in each column is 1.
% b is a column vector with 8-rows or a matrix of such column vectors
%        that is the observed response
% x is a column vector with the number of rows equal to the number of
%        columns A (number of possible fluors) if b is a column vector. If
%        b is a matrix, x is a matrix with with the number of rows equal to
%        the number of columns A and the number of columns equal to the
%        number of columns of b. Each column of b is the weighting of the
%        fluors in the columns of A that best match the observed fluor response.
% For example using a 6-element fluor representation
%     A' = [1 10 5 0 0 0;
%           1 5 10 4 0 0;
%           0 0 3 10 5 0;
%           0 0 10 7 3 2]
%A' =
% 1 10  5  0 0 0
% 1  5 10  4 0 0
% 0  0  3 10 5 0
% 0  0 10  7 3 2
%     b' = [1 11 5 0 0 0;
%           2 15 15 4 0 0;
%           2 20 13 10 5 0]
%b' =
% 1 11  5  0 0 0
% 2 15 15  4 0 0
% 2 20 13 10 5 0
%[ x ] = FluorSolver( A, b )
%x =
%  1.1191  1.0000  2.0000
% -0.0416  1.0000  0.0000
%  0.0301  0.0000  1.0000
% -0.0253 -0.0000 -0.0000
%
x = A\b;
end
```

FIG. 21A

Current fluor signature matrix A

| F488 | Noise | PE488 | SFR488 |
|---|---|---|---|
| 0.269784 | 0.125 | 0.005805 | 0 |
| 0.471087 | 0.125 | 0.015633 | 0 |
| 0.158544 | 0.125 | 0.318742 | 0 |
| 0.062158 | 0.125 | 0.396312 | 0 |
| 0.021845 | 0.125 | 0.156353 | 0 |
| 0.006157 | 0.125 | 0.072972 | 0 |
| 0.006048 | 0.125 | 0.024215 | 0.426647 |
| 0.004377 | 0.125 | 0.009968 | 0.573353 |

FIG. 21B

| 2211 MESF Value | 2212 FITC_ MFI | 2211 MESF Value | 2213 F488 Values | 2214 F488 # |
|---|---|---|---|---|
| 5268 | 25 | 5268 | 30.5 | 13 |
| 20165 | 50 | 20165 | 99.6 | 54 |
| 83051 | 250 | 83051 | 354 | 82 |
| 358662 | 1000 | 358662 | 1240 | 456 |
| 1127616 | 3000 | 1127616 | 3519.8 | 1640 |

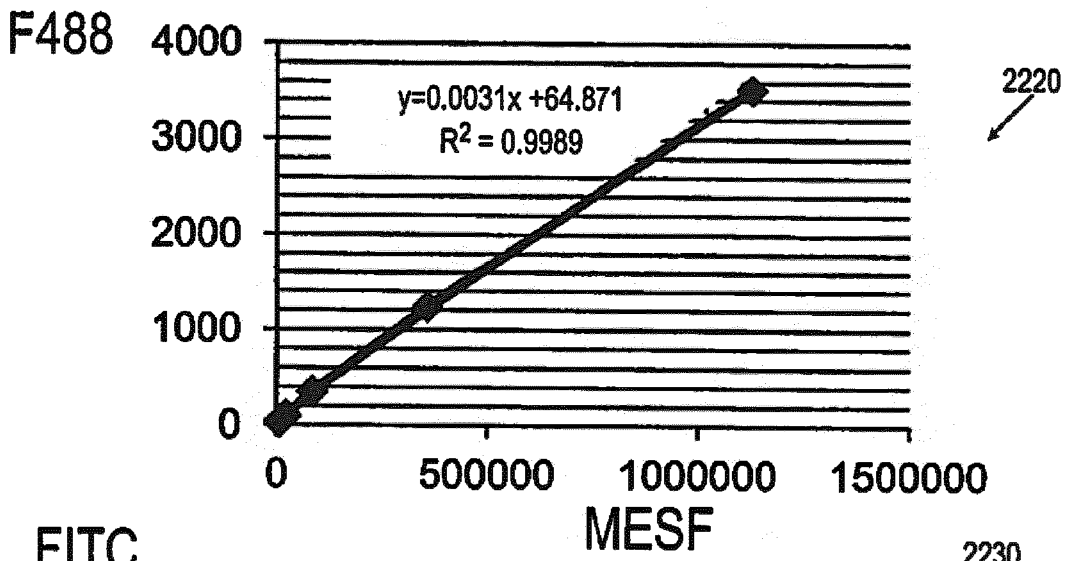


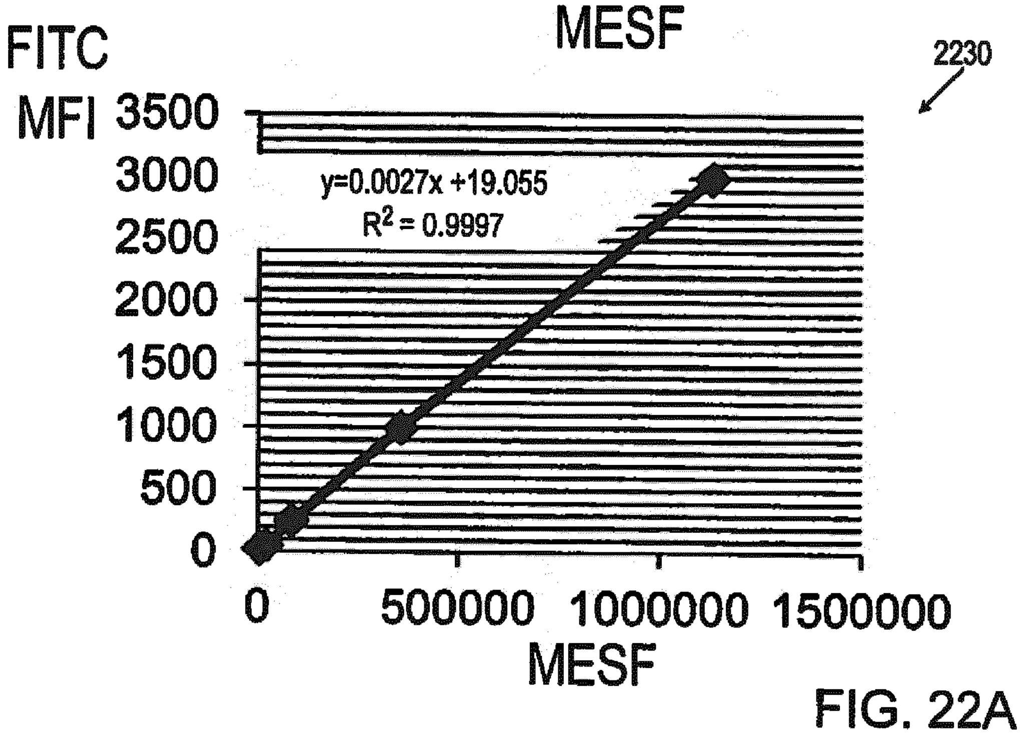


FIG. 22A

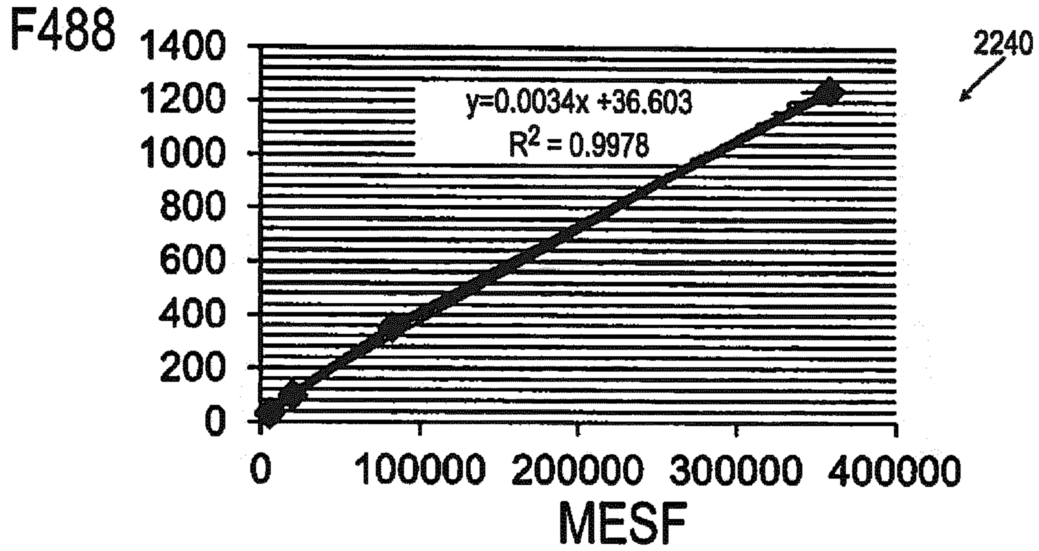
F488
1400
1200
1000
800
600
400
200
0
y=0.0034x +36.603
R2 = 0.9978
0
100000
200000
300000
400000
MESF
2240

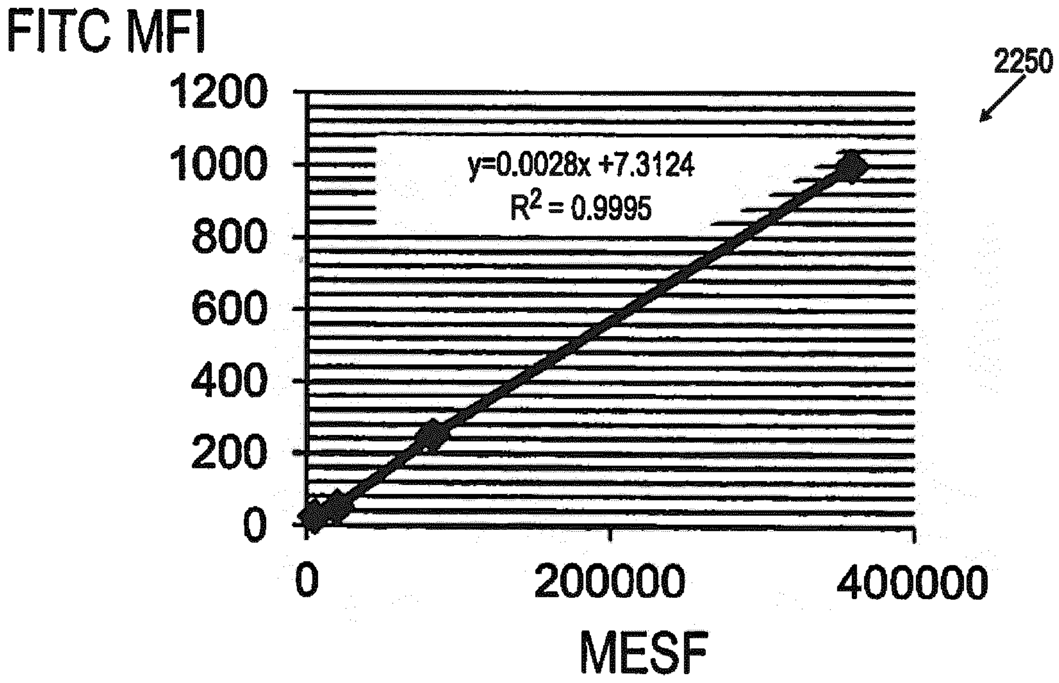
FITC MFI
1200
1000
800
600
400
200
0
y=0.0028x +7.3124
R2 = 0.9995
0
200000
400000
MESF
2250

FIG. 22B

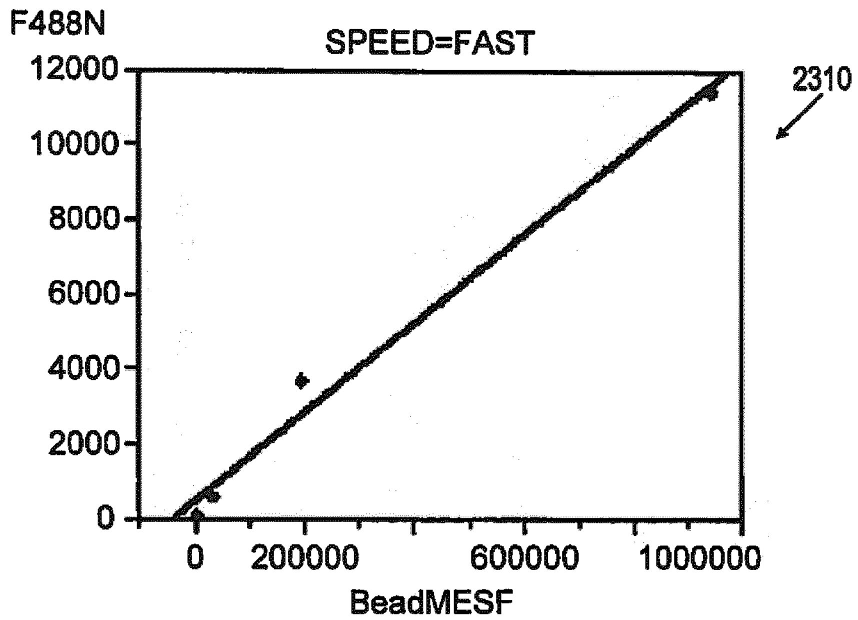


LINEAR FIT

F488N=463.95252+0.0118577 BeadMESF

2320

SUMMARY OF FIT

| | |
|---|---|
| RSquare | 0.986887 |
| RSquare Adj | 0.980331 |
| Root Mean Square Error | 736.7946 |
| Mean of Response | 3944.267 |
| Observation (or Sum Wgts) | 4 |

ANALYSIS OF VARIANCE

| SOURCE | DF | SUM OF SQRS | MEAN SQR | F RATIO |
|---|---|---|---|---|
| Model | 1 | 81714622 | 81714622 | 150.5244 |
| Error | 2 | 1085733 | 542866.31 | Prob>F |
| C.Total | 3 | 82800355 | | 0.0066 |

PARAMETER ESTIMATES

| TERM | ESTIMATE | STD ERROR | t RATIO | PROB>[t] |
|---|---|---|---|---|
| Intercept | 463.95252 | 464.9579 | 1.00 | 0.4235 |
| BeadMESF | 0.0118577 | 0.00966 | 12.27 | 0.0066 |

FIG. 23A

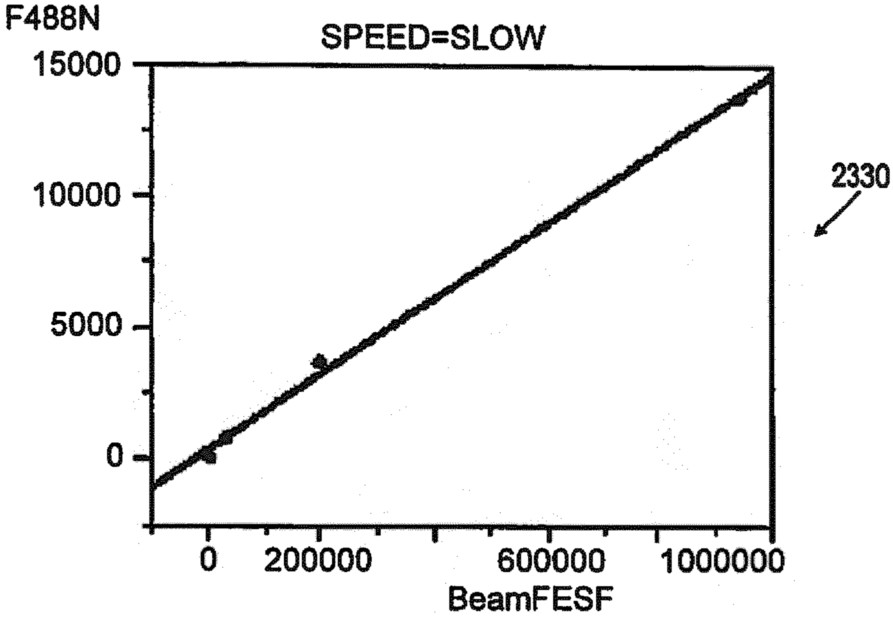


LINEAR FIT

F488N=372.07609+0.0143173 BeadMESF

SUMMARY OF FIT

| | |
|---|---|
| RSquare | 0.986889 |
| RSquare Adj | 0.995334 |
| Root Mean Square Error | 431.117 |
| Mean of Response | 4574.303 |
| Observation (or Sum Wgts) | 4 |

2340

ANALYSIS OF VARIANCE

| SOURCE | DF | SUM OF SQRS | MEAN SQR | F RATIO |
|---|---|---|---|---|
| Model | 1 | 119130179 | 119130179 | 640.9608 |
| Error | 2 | 371724 | 195861.87 | Prob>F |
| C.Total | 3 | 119501903 | | 0.0016 |

PARAMETER ESTIMATES

| TERM | ESTIMATE | STD ERROR | t RATIO | PROB>[t] |
|---|---|---|---|---|
| Intercept | 372.07609 | 272.0585 | 1.37 | 0.3048 |
| BeadMESF | 0.0143173 | 0.000566 | 25.32 | 0.0016 |

FIG. 23B

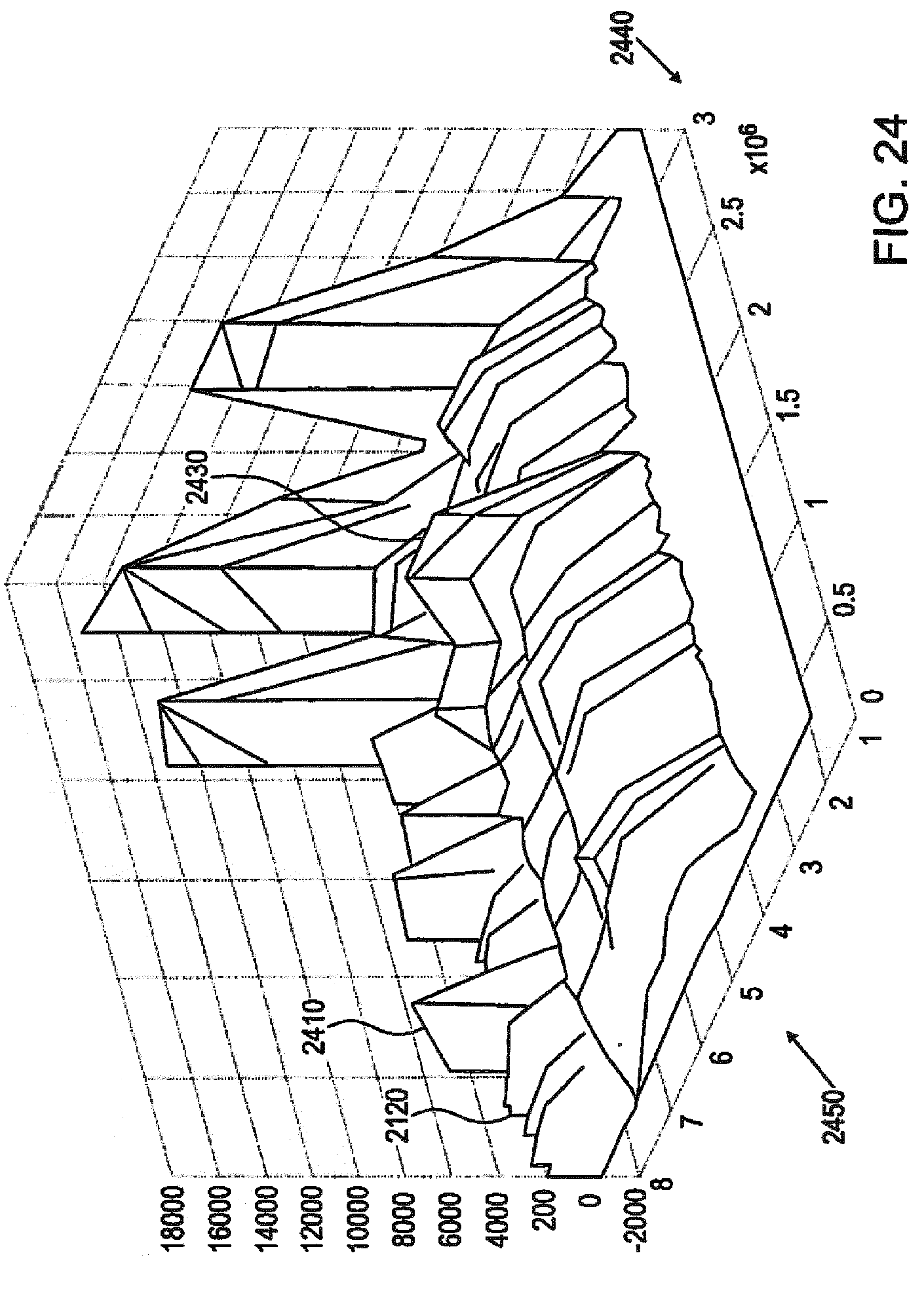
2440
2430
2410
2120
2450
x10^6
18000
16000
14000
12000
10000
8000
6000
4000
200
0
-2000
0 0.5 1 1.5 2 2.5 3
1 2 3 4 5 6 7 8

FIG. 24

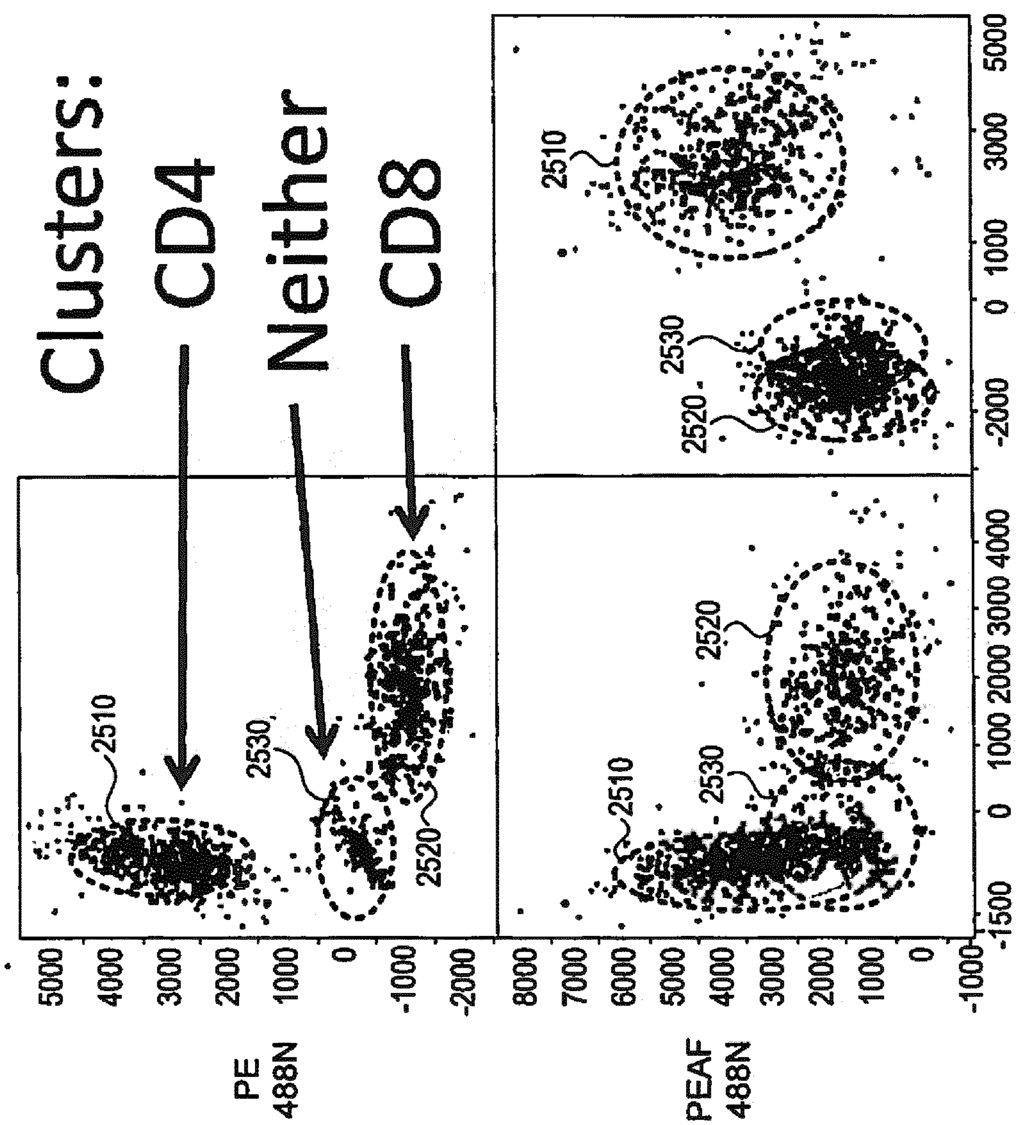
Clusters:
CD4
Neither
CD8
PE 488N
PEAF 488N
ALEXA 488N
PE488N
2510
2520
2530
5000
4000
3000
2000
1000
0
-1000
-2000
8000
7000
6000
-1500


FIG. 25

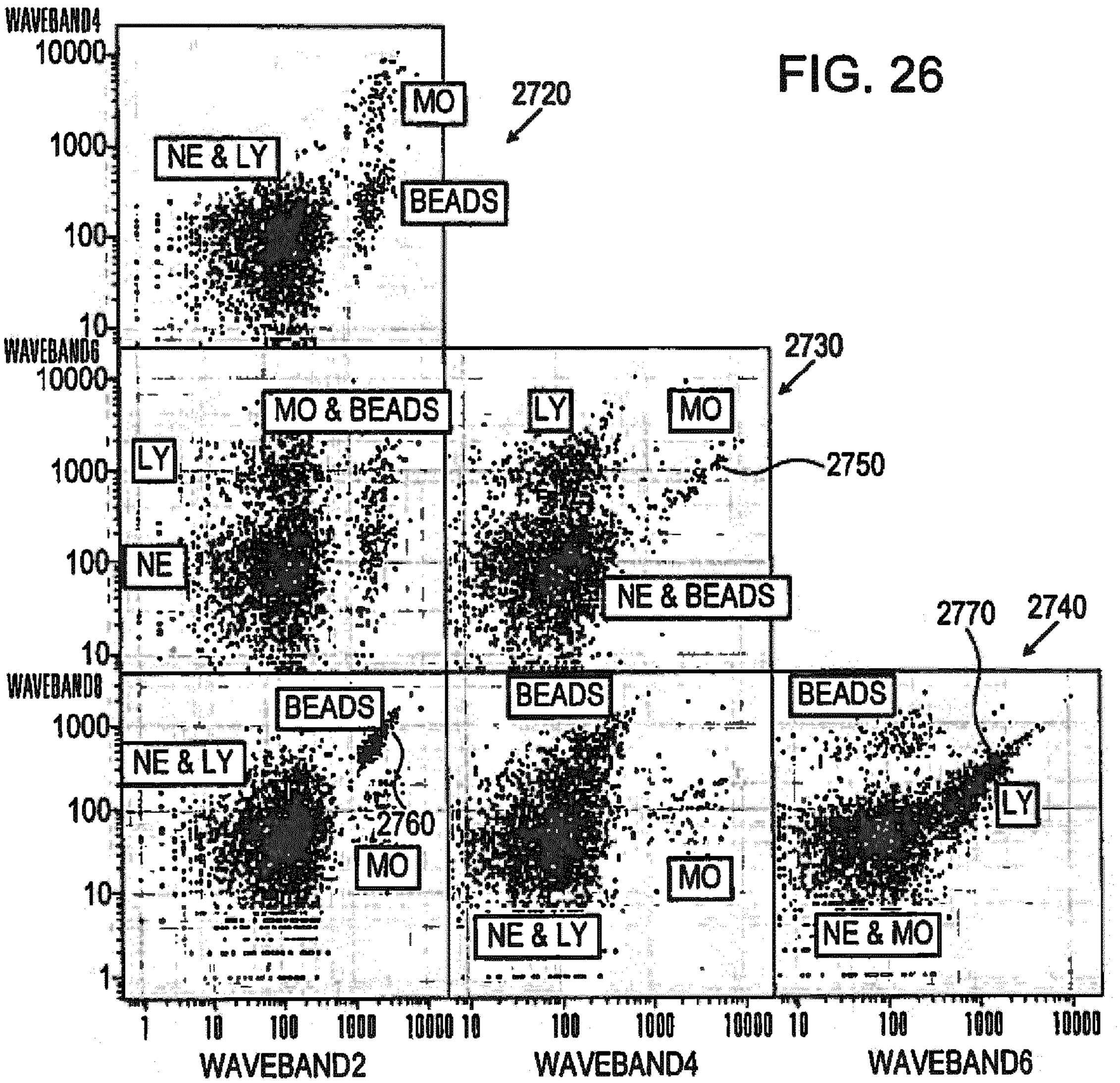


FIG. 26

| Waveband | F488 | Noise | PE488 | SFR488 | PEAF488 |
|---|---|---|---|---|---|
| 1: 500-524 | 0.269784 | 0.125 | 0.005805 | 0 | 0.006927 |
| <u>2: 525-549</u> | <u>0.471087</u> | 0.125 | 0.015633 | 0 | 0.016996 |
| 3: 550-574 | 0.158544 | 0.125 | 0.318742 | 0 | 0.038335 |
| <u>4: 575-599</u> | 0.062158 | 0.125 | <u>0.396312</u> | 0 | 0.040674 |
| 5: 600-624 | 0.021845 | 0.125 | 0.156353 | 0 | 0.364123 |
| <u>6: 625-649</u> | 0.006157 | 0.125 | 0.072972 | 0 | <u>0.340389</u> |
| 7: 650-674 | 0.006048 | 0.125 | 0.024215 | 0.426647 | 0.127784 |
| <u>8: 675-700</u> | 0.004377 | 0.125 | 0.009968 | <u>0.573353</u> | 0.064773 |

FIG. 27

| Type No. | Anode Type | | Effective Area per Channel (mm) | Channel Pitch (mm) | Dynode Structure / No. of Stages | Weight (g) | Insulation Cover Material |
|---|---|---|---|---|---|---|---|
| H9530 SERIES | 8-Channel IUi near Array | 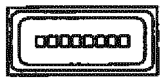 | 2.0x2.5 | 2.8 | MC/12 | 25.5 | P.O.M |
| H1 05158 SERIES | 16·Ghannel Linear Anay | 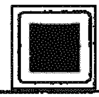 | 0.8 x 16 | 1 | MC110 | 49.0 | P.O.M |
| H7260 SERIES | 32·Ghannel Linear Anay | 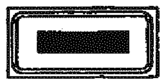 | 0.8 x7 | 1 | MC/12 | 62.0 | P.O.M |

FIG. 28

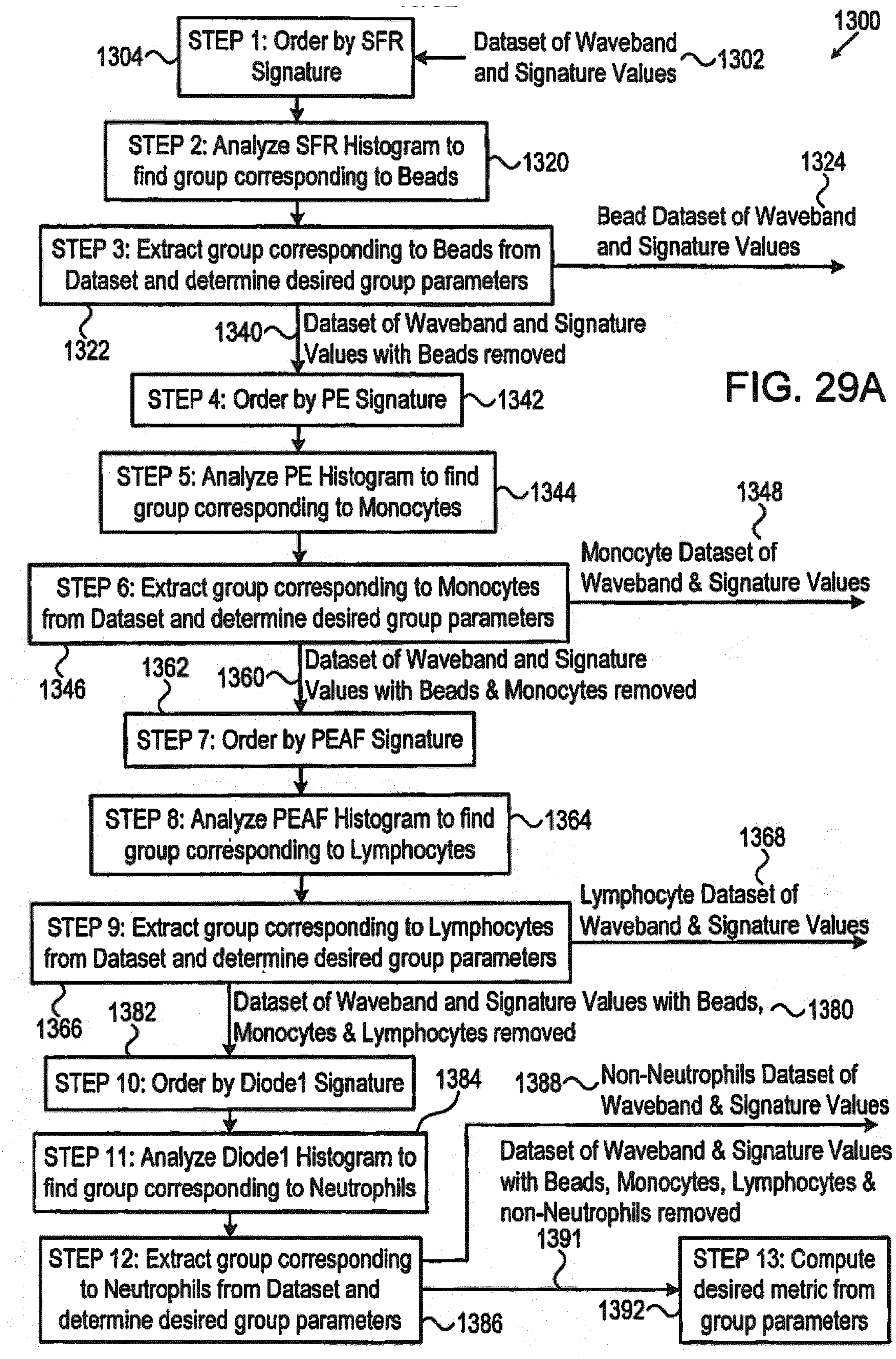
1300
STEP 1: Order by SFR Signature
1304
Dataset of Waveband and Signature Values
1302
STEP 2: Analyze SFR Histogram to find group corresponding to Beads
1320
STEP 3: Extract group corresponding to Beads from Dataset and determine desired group parameters
1322
1324
Bead Dataset of Waveband and Signature Values
1340
Dataset of Waveband and Signature Values with Beads removed
STEP 4: Order by PE Signature
1342
STEP 5: Analyze PE Histogram to find group corresponding to Monocytes
1344
1348
Monocyte Dataset of Waveband & Signature Values
STEP 6: Extract group corresponding to Monocytes from Dataset and determine desired group parameters
1346
1362
1360
Dataset of Waveband and Signature Values with Beads & Monocytes removed
STEP 7: Order by PEAF Signature
STEP 8: Analyze PEAF Histogram to find group corresponding to Lymphocytes
1364
1368
Lymphocyte Dataset of Waveband & Signature Values
STEP 9: Extract group corresponding to Lymphocytes from Dataset and determine desired group parameters
1366
1382
Dataset of Waveband and Signature Values with Beads, Monocytes & Lymphocytes removed
1380
STEP 10: Order by Diode1 Signature
1384
1388
Non-Neutrophils Dataset of Waveband & Signature Values
STEP 11: Analyze Diode1 Histogram to find group corresponding to Neutrophils
Dataset of Waveband & Signature Values with Beads, Monocytes, Lymphocytes & non-Neutrophils removed
1391
STEP 12: Extract group corresponding to Neutrophils from Dataset and determine desired group parameters
1386
1392
STEP 13: Compute desired metric from group parameters

FIG. 29A

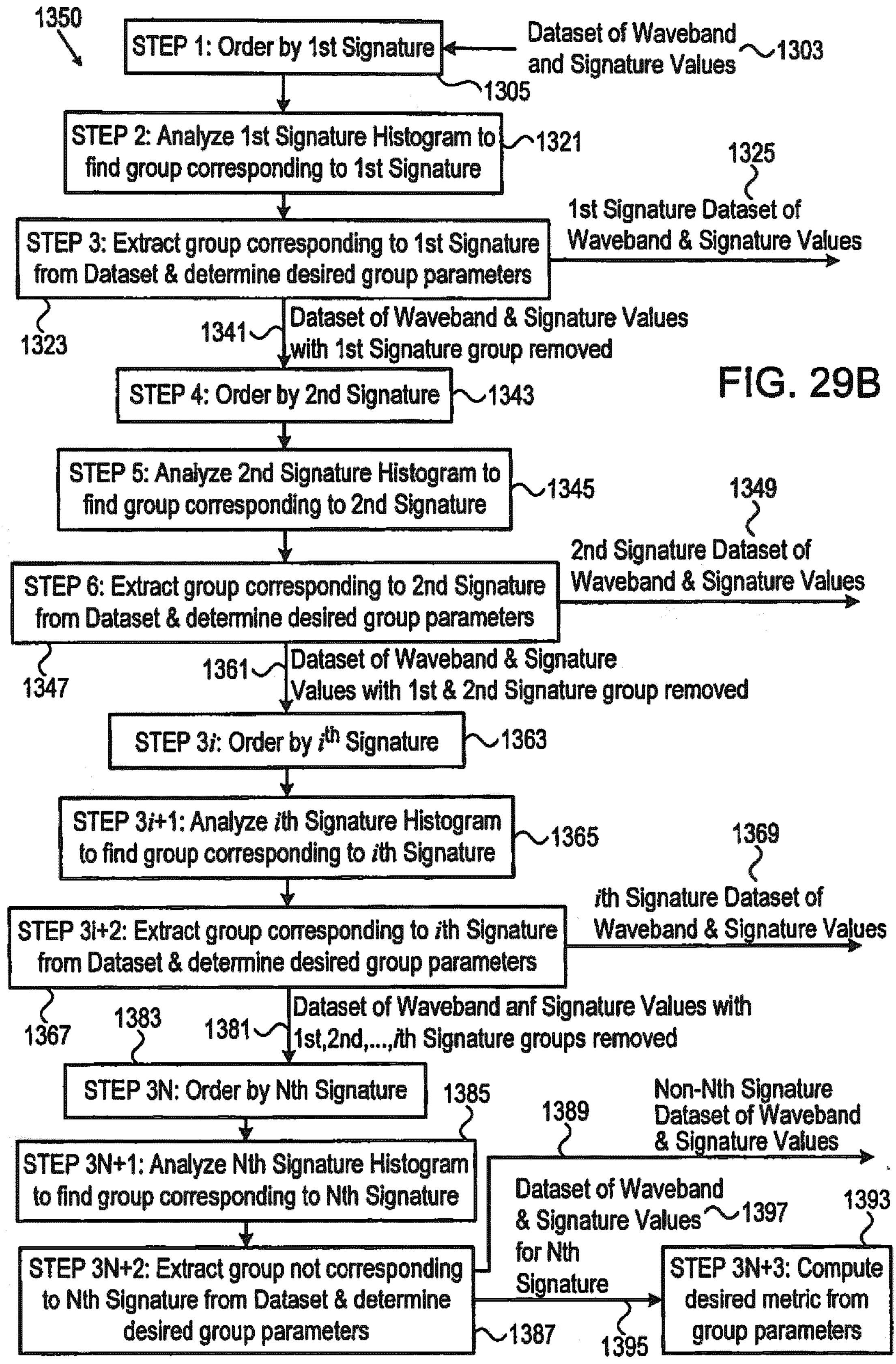
1350
STEP 1: Order by 1st Signature
Dataset of Waveband and Signature Values
1303
1305
STEP 2: Analyze 1st Signature Histogram to find group corresponding to 1st Signature
1321
1325
1st Signature Dataset of Waveband & Signature Values
STEP 3: Extract group corresponding to 1st Signature from Dataset & determine desired group parameters
1323
1341
Dataset of Waveband & Signature Values with 1st Signature group removed
STEP 4: Order by 2nd Signature
1343
STEP 5: Analyze 2nd Signature Histogram to find group corresponding to 2nd Signature
1345
1349
2nd Signature Dataset of Waveband & Signature Values
STEP 6: Extract group corresponding to 2nd Signature from Dataset & determine desired group parameters
1347
1361
Dataset of Waveband & Signature Values with 1st & 2nd Signature group removed
STEP 3i: Order by ith Signature
1363
STEP 3i+1: Analyze ith Signature Histogram to find group corresponding to ith Signature
1365
1369
ith Signature Dataset of Waveband & Signature Values
STEP 3i+2: Extract group corresponding to ith Signature from Dataset & determine desired group parameters
1367
1383
1381
Dataset of Waveband anf Signature Values with 1st,2nd,...,ith Signature groups removed
STEP 3N: Order by Nth Signature
1385
1389
Non-Nth Signature Dataset of Waveband & Signature Values
STEP 3N+1: Analyze Nth Signature Histogram to find group corresponding to Nth Signature
Dataset of Waveband & Signature Values for Nth Signature
1397
1393
STEP 3N+2: Extract group not corresponding to Nth Signature from Dataset & determine desired group parameters
1387
1395
STEP 3N+3: Compute desired metric from group parameters

FIG. 29B

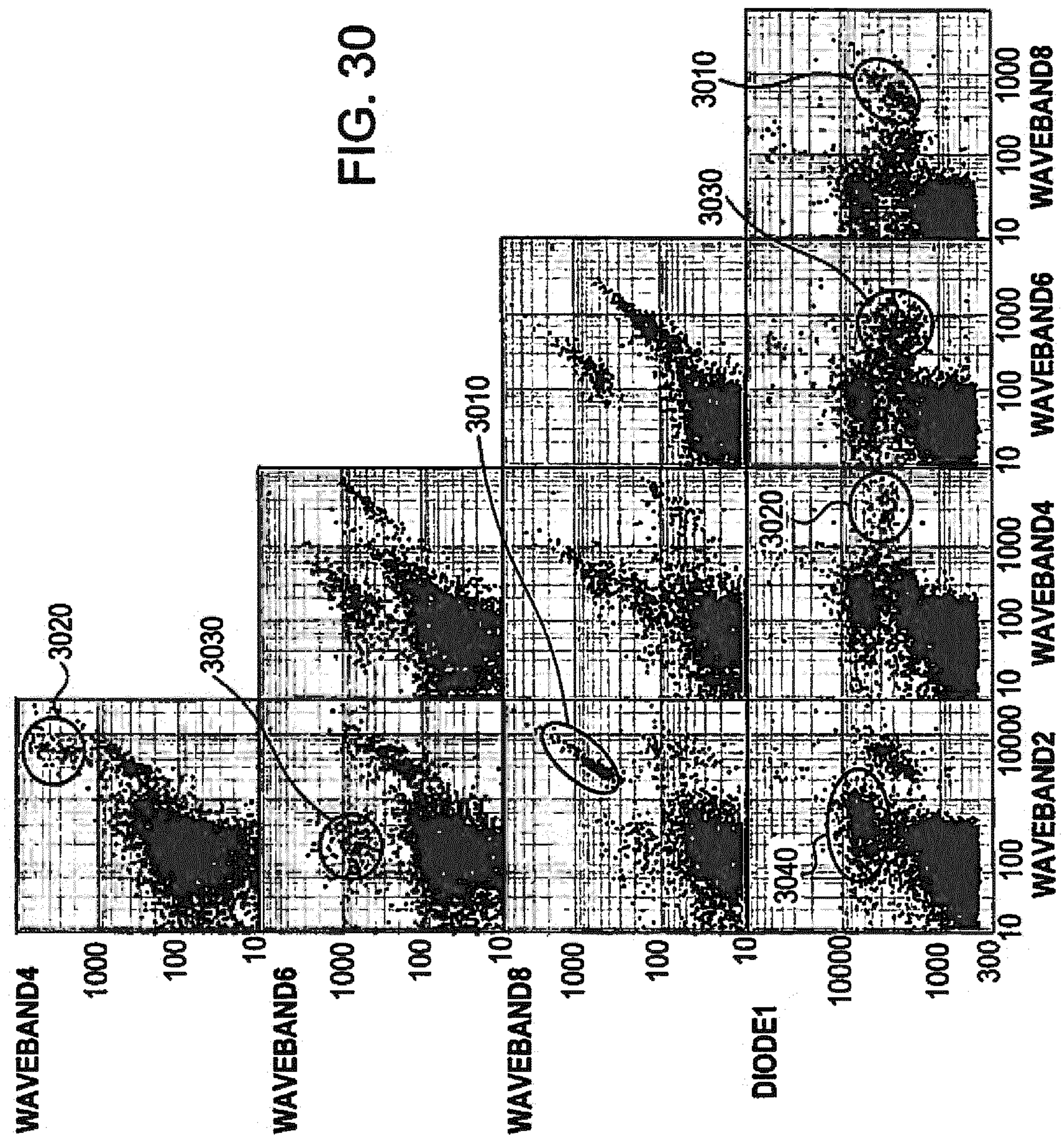
WAVEBAND4
WAVEBAND6
WAVEBAND8
DIODE1
WAVEBAND2
WAVEBAND4
WAVEBAND6
WAVEBAND8
3010
3020
3030
3040

FIG. 30

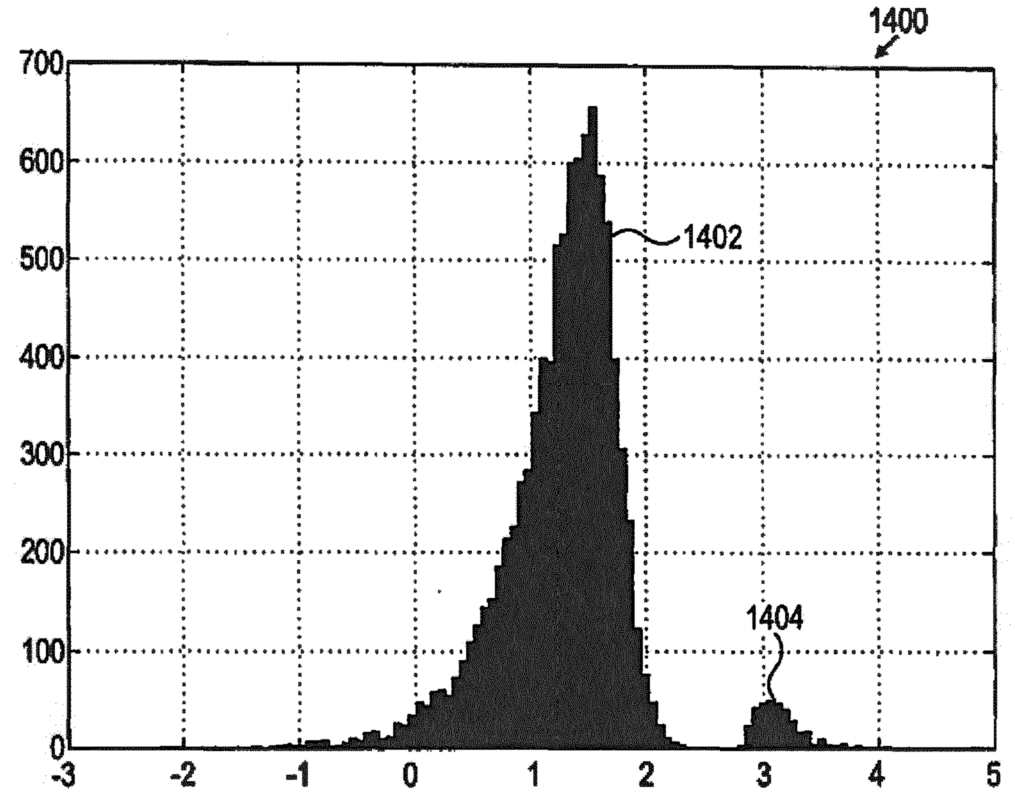


FIG. 31A

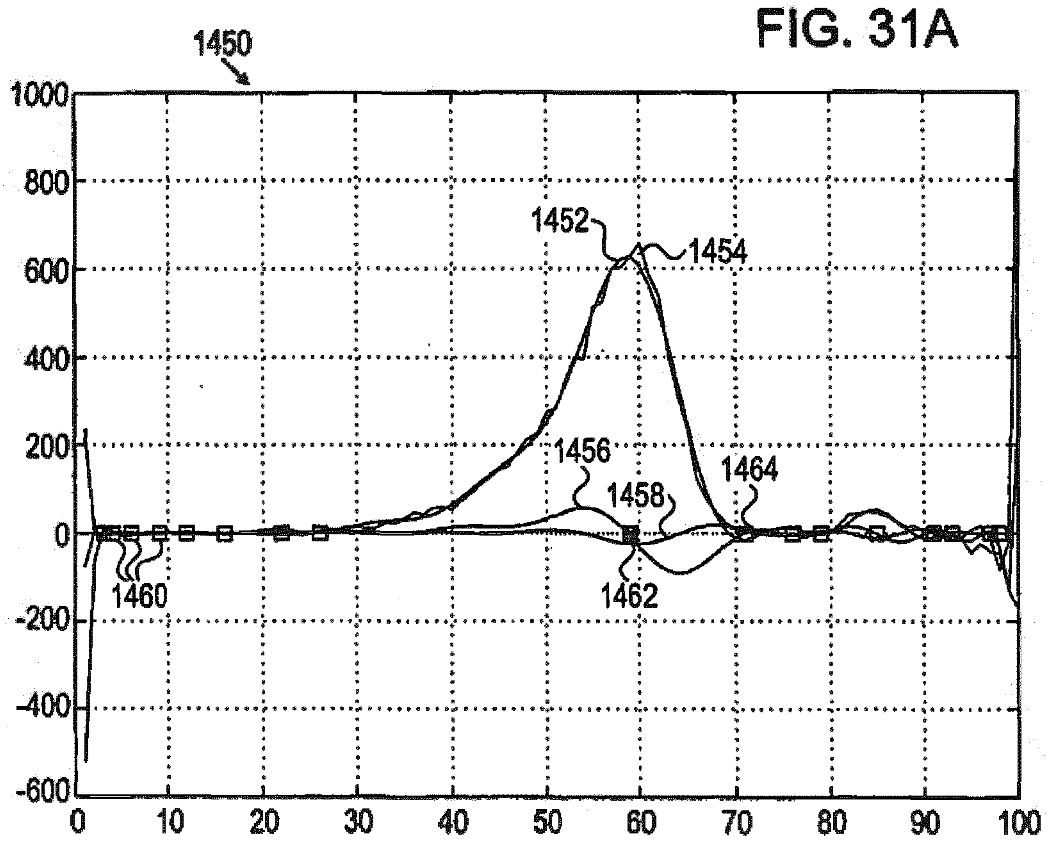


FIG. 31B

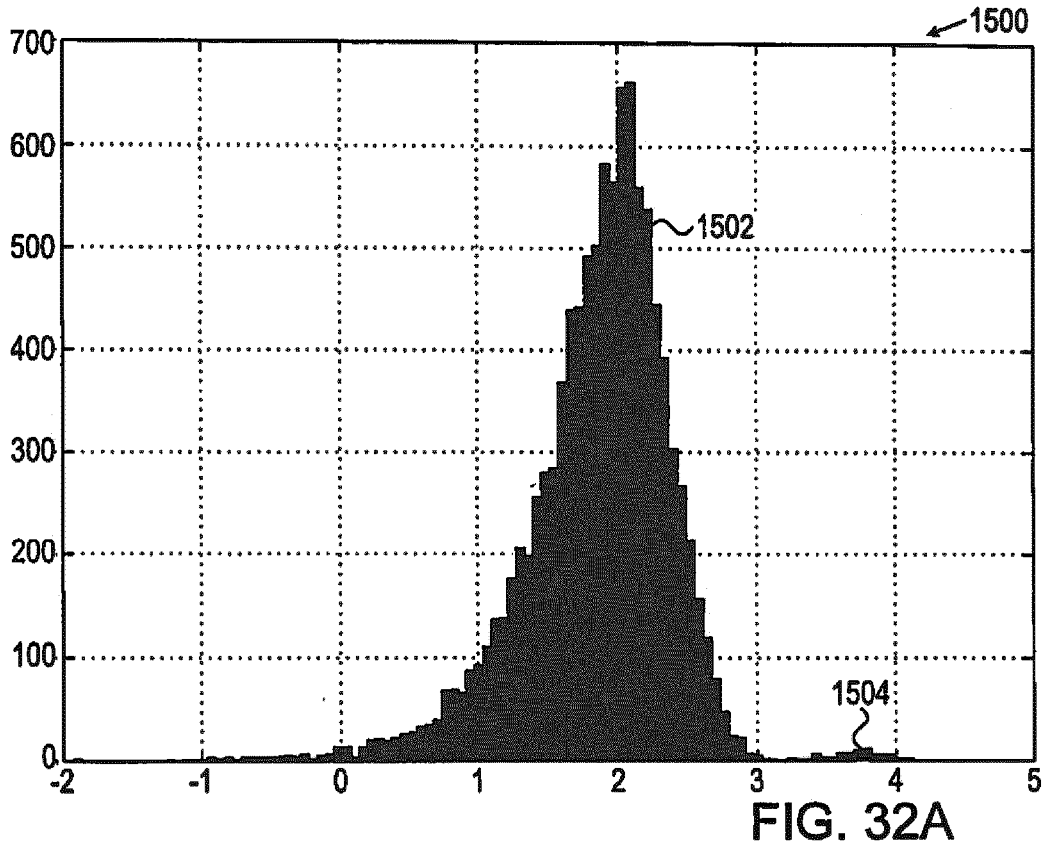


FIG. 32A

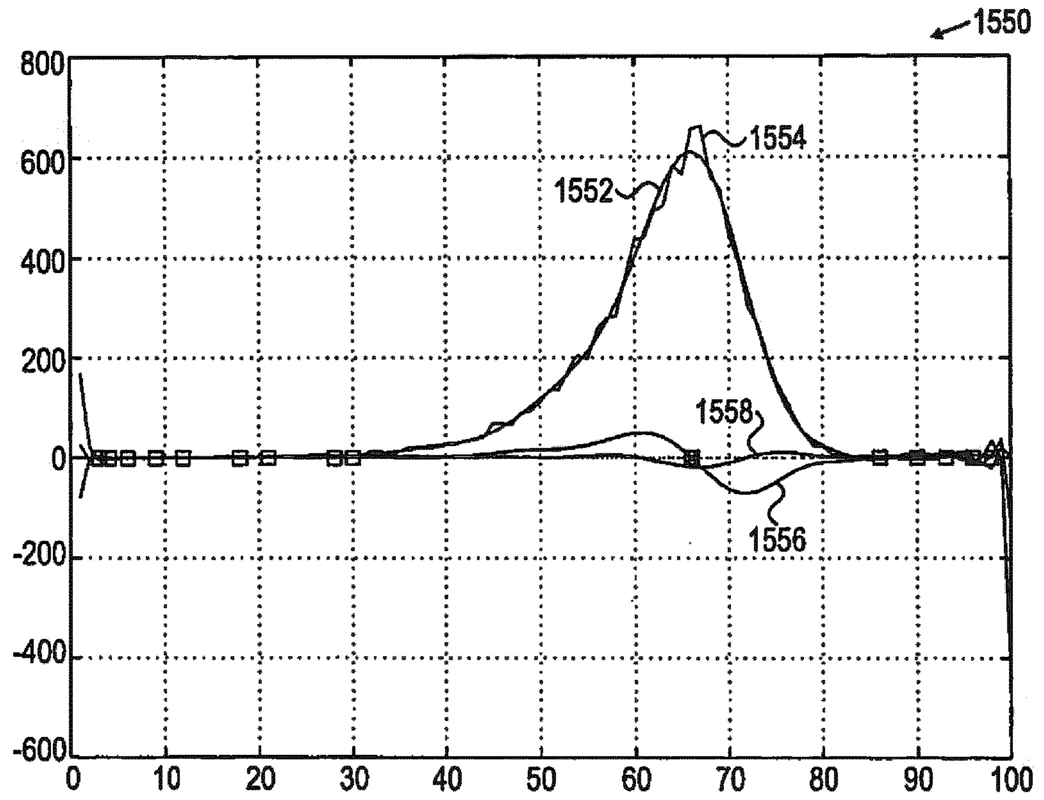


FIG. 32B

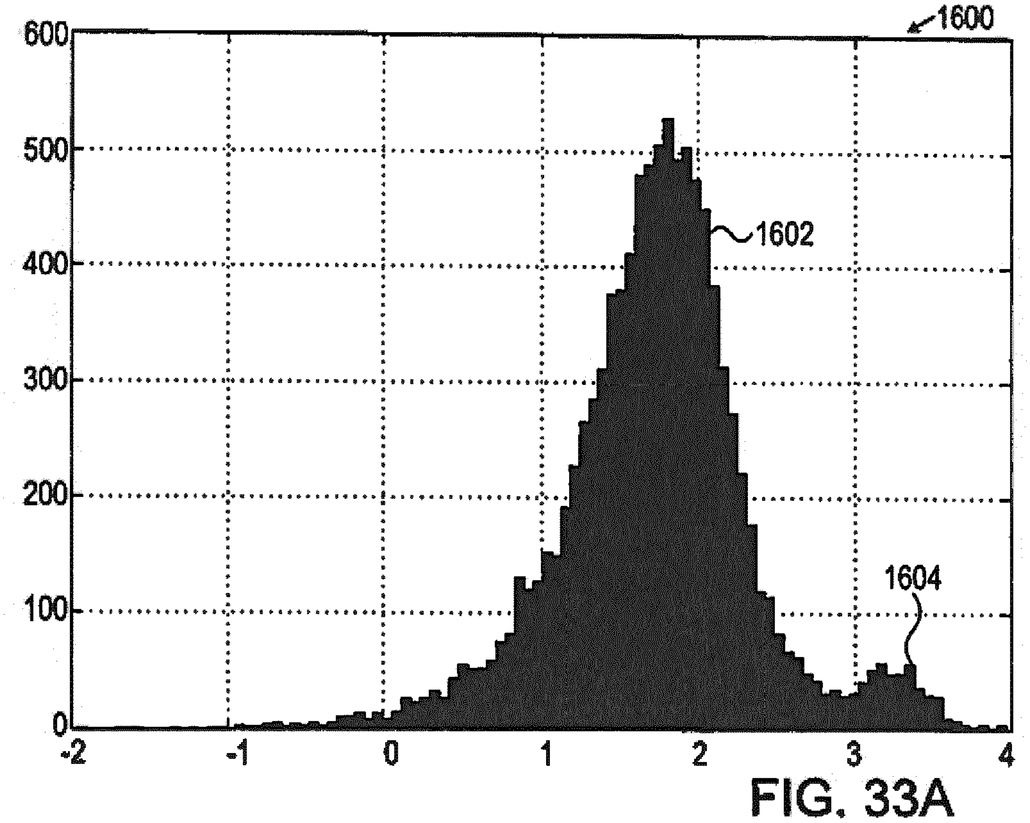


FIG. 33A

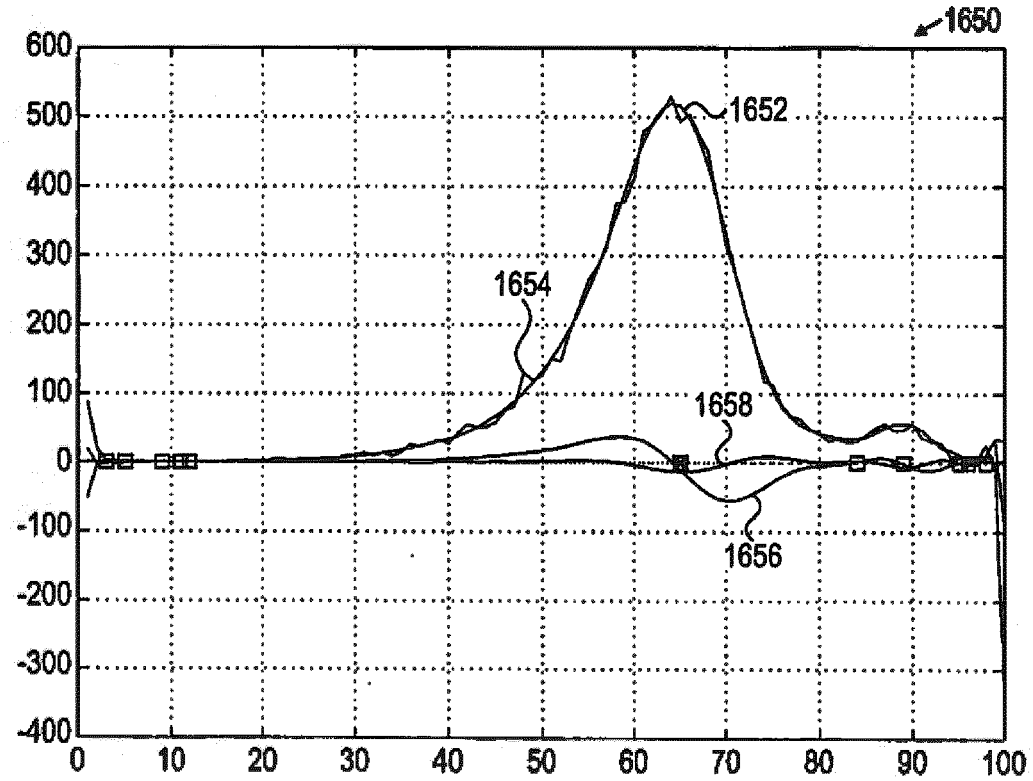


FIG. 33B

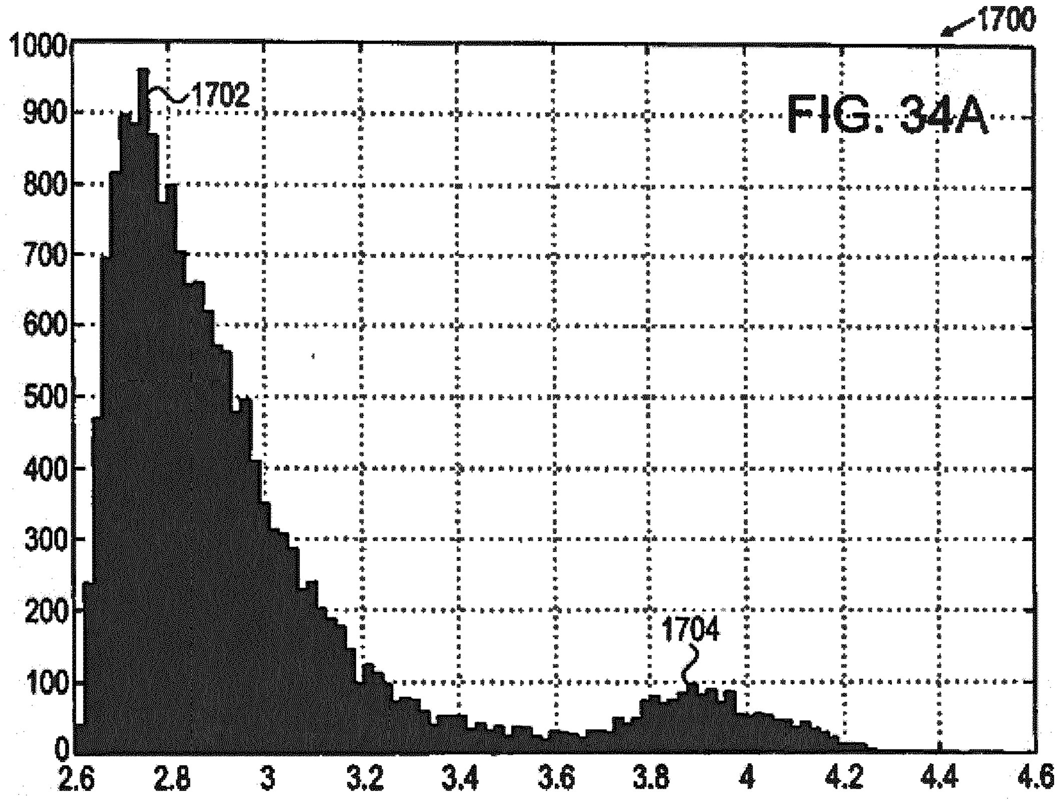


FIG. 34A

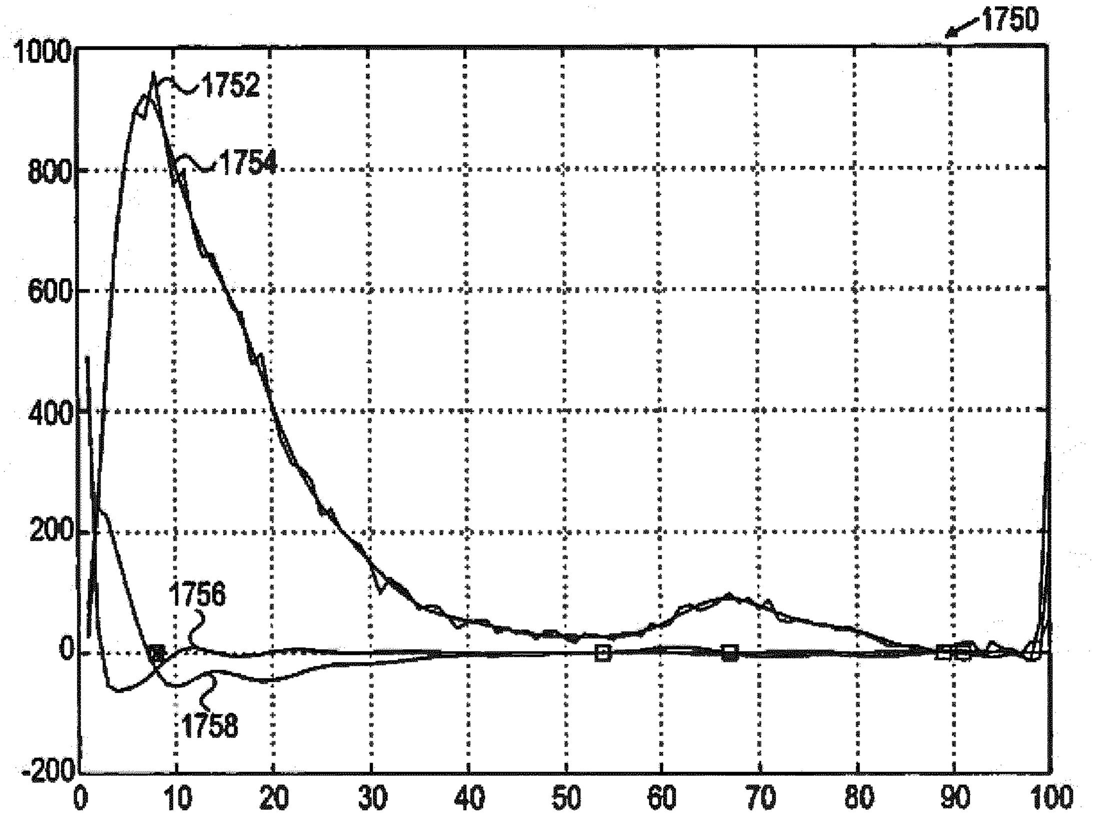


FIG. 34B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8116984 B
- US 2006215155 A
- US 2012187117 A
- WO 2007076549 A
- WO 2011128893 A, Kasdan


### Non-patent literature cited in the description


- **ASSICOT, MARCEL et al.** High serum procalcitonin concentrations in patients with sepsis and infection. *The Lancet,* 1993, vol. 341 (8844), 515-518
- **AULESA, C. et al.** Validation of the Coulter LH 750 in a hospital reference laboratory. *Laboratory Hematology,* 2003, vol. 9 (1), 15-28
- **HAWKINS, ROBERT C.** Laboratory turnaround time. *The Clinical Biochemist Reviews,* 2007, vol. 28 (4), 179
- **AULT, KENNETH A.** Flow cytometric measurement of platelet function and reticulated platelets. *Annals of the New York Academy of Sciences,* 1993, vol. 677 (1), 293-308
- **BLAJCHMAN, MORRIS A. et al.** Bacterial detection of platelets: current problems and possible resolutions. *Transfusion medicine reviews,* 2005, vol. 19 (4), 259-272
- **BODENSTEINER, DAVID C.** A flow cytometric technique to accurately measure post-filtration white blood cell counts. *Transfusion,* 1989, vol. 29 (7), 651-653
- **CHESON, BRUCE D. et al.** National Cancer Institute-sponsored Working Group guidelines for chronic lymphocytic leukemia: revised guidelines for diagnosis and treatment. *Blood,* 1996, vol. 87 (12), 4990-4997
- **CHRIST-CRAIN, MIRJAM et al.** Effect of procalcitonin-guided treatment on antibiotic use and outcome in lower respiratory tract infections: cluster-randomised, single-blinded intervention trial. *Lancet,* 2004, vol. 363 (9409), 600-607
- **CRISTOFANILLI, MASSIMO et al.** Circulating tumor cells, disease progression, and survival in metastatic breast cancer. *New England Journal of Medicine,* 2004, vol. 351 (8), 781-791
- **DAVIS, BRUCE H. et al.** Neutrophil CD64 is an improved indicator of infection or sepsis in emergency department patients. *Archives of pathology & laboratory medicine,* 2006, vol. 130 (5), 654-661
- **DIEYE, TANDAKHA NDIAYE et al.** Absolute CD4 T-cell counting in resource-poor settings: direct volumetric measurements versus bead-based clinical flow cytometry instruments. *JAIDS Journal of Acquired Immune Deficiency Syndromes,* 2005, vol. 39 (1), 32-37
- **DIVERS, S. G. et al.** Quantitation of CD62, soluble CD62, and lysosomeassociated membrane proteins 1 and 2 for evaluation of the quality of stored platelet concentrates. *Transfusion,* 2003, vol. 35 (4), 292-297
- **DREXLER, HANS G. et al.** Diagnostic value of immunological leukemia phenotyping. *Acta haematologica,* 1986, vol. 76 (1), 1-8
- **DZIEGIEL, MORTEN HANEFELD ; LEIF KOFOED NIELSEN ; ADELA BERKOWICZ.** Detecting fetomatemal hemorrhage by flow cytometry. *Current opinion in hematology,* 2006, vol. 13 (6), 490
- **FISCHER, JOHANNES C. et al.** Reducing costs in flow cytometric counting of residual white blood cells in blood products: utilization of a single platform bead free flowrate calibration method. *Transfusion,* 2011, vol. 51 (7), 1431-1438
- **GRAFF, JOCHEN et al.** Close relationship between the platelet activation marker CD62 and the granular release of platelet-derived growth factor. *Journal of Pharmacology and Experimental Therapeutics,* 2002, vol. 300 (3), 952-957
- **GUERTI, K. et al.** Performance evaluation of the PENTRA 60C+ automated hematology analyzer and comparison with the AD VIA 2120. *International journal of laboratory hematology,* 2009, vol. 31 (2), 132-141
- **HERSHMAN, M. J. et al.** Monocyte HLA-DR antigen expression characterizes clinical outcome in the trauma patient. *British Journal of Surgery,* 2005, vol. 77 (2), 204-207

- **HILFRICH, RALF ; JALIL HARIRI.** Prognostic relevance of human papillomavirus L1 capsid protein detection within mild and moderate dysplastic lesions of the cervix uteri in combination with pl6 biomarker. *Analytical and Quantitative Cytology and Histology,* 2008, vol. 30 (2), 78-82
- **HILLIER, SHARON L. et al.** A case-control study of chorioamnionic infection and histologic chorioamnionitis in prematurity. *New England Journal of Medicine,* 1988, vol. 319 (15), 972-978
- **HOFFMANN, JOHANNES JML.** Neutrophil CD64 as a sepsis biomarker. *Biochemia Medica,* 2011, vol. 21 (3), 282-290
- **KIBE, SAVITRI ; KATE ADAMS ; GAVIN BARLOW.** Diagnostic and prognostic biomarkers of sepsis in critical care. *Journal of Antimicrobial Chemotherapy,* 2011, vol. 66 (2), ii33-ii40
- **LAROSA, STEVEN P. ; STEVEN M. OPAL.** Biomarkers: the future. *Critical care clinics,* 2011, vol. 27 (2), 407
- **LIU, N. I. N. G. ; A. H. WU ; SHAN S. WONG.** Improved quantitative Apt test for detecting fetal hemoglobin in bloody stools of newborns. *Clinical chemistry,* 1993, vol. 39 (11), 2326-2329
- **LOTAN, YAIR et al.** Bladder cancer screening in a high risk asymptomatic population using a point of care urine based protein tumor marker. *The Journal of urology,* 2009, vol. 182 (1), 52-58
- **MASSE, M. et al.** Validation of a simple method to count very low white cell concentrations in filtered red cells or platelets. *Transfusion,* 2003, vol. 32 (6), 565-571
- **MATIC, GORAN B. et al.** Whole blood analysis of reticulated platelets: improvements of detection and assay stability. *Cytometry,* 1998, vol. 34 (5), 229-234
- **MCDONALD, C. P. et al.** Use of a solid-phase fluorescent cytometric technique for the detection of bacteria in platelet concentrates. *Transfusion Medicine,* 2005, vol. 15 (3), 175-183
- **MICHELSON, ALAN D.** Flow cytometry: a clinical test of platelet function. *Open Access Articles,* 1996, 290
- Proteomics in Microfluidic Devices. **MILLER, E.M. ; FREIRE, S.L.S. ; WHEELER, A.R.** Encyclopedia of Micro- and Nanofluidics. Springer: Heidelberg, 2008, vol. 3, 1749-1758
- **MORO, RICARDO et al.** A new broad-spectrum cancer marker. *Vitro Diagnostic Technology,* 2005
- **PERRY, SARA E. et al.** Is low monocyte HLA-DR expression helpful to predict outcome in severe sepsis?. *Intensive care medicine,* 2003, vol. 29 (8), 1245-1252
- **RAMAKUMAR, SANJAY et al.** Comparison of screening methods in the detection of bladder cancer. *The Journal of urology,* 1999, vol. 161 (2), 388-394
- **RAWSTRON, ANDY C. et al.** Quantitation of minimal disease levels in chronic lymphocytic leukemia using a sensitive flow cytometric assay improves the prediction of outcome and can be used to optimize therapy. *Blood,* 2001, vol. 98 (1), 29-35
- **RODRIGUEZ, WILLIAM R. et al.** A microchip CD4 counting method for HIV monitoring in resource-poor settings. *PLoS medicine,* 2005, vol. 2 (7), el82
- **RYLATT, D. B. et al.** An immunoassay for human D dimer using monoclonal antibodies. *Thrombosis research,* 1983, vol. 31 (6), 767-778
- **SACKS, DAVID B. et al.** Guidelines and recommendations for laboratory analysis in the diagnosis and management of diabetes mellitus. *Clinical Chemistry,* 2002, vol. 48 (3), 436-472
- **SEGAL, H. C. et al.** Accuracy of platelet counting haematology analysers in severe thrombocytopenia and potential impact on platelet transfusion. *British Journal of Haematology,* 2005, vol. 128 (4), 520-525
- **STEIN, PAUL D. et al.** D-dimer for the exclusion of acute venous thrombosis and pulmonary embolism: a systematic review. *Annals of internal medicine,* 2004, vol. 140 (8), 589
- **SUTHERLAND, D. ROBERT et al.** The ISHAGE guidelines for CD34+ cell determination by flow cytometry. *Journal ofhematotherapy,* 1996, vol. 5 (3), 213-226
- **WANG, CHAO et al.** Reticulated platelets predict platelet count recovery following chemotherapy. *Transfusion,* 2002, vol. 42 (3), 368-374